# EUROPEAN PATENT APPLICATION

(11) **EP 2 441 846 A2**
(43) Date of publication of application: **18.04.2012**
(21) Application number: 11175735.7
(22) Date of filing: 09.01.2007
(51) Int. Cl.: C12P 21/02, C07H 21/04, C07K 14/25, C12N 1/21

(54) **Immunostimulatory combinations of TNFRSF, TLR, NLR, RHR, purinergic receptor, and cytokine receptor agoinsts for vaccines and tumor immunotherapy**

(30) Priority: 09.01.2006 US 757314 P
(62) Divisional of application: 07769138.4
(71) Applicant: The Regents Of the University of California, Oakland, CA 94607-5200 (US)
(72) Inventor: Kornbluth, Richard Syd, La Jolla, CA 92037-7641 (US); Stone, Geoffrey William, Toronto, Ontario M5B 2E8 (CA)
(74) Representative: Hollywood, Jane Constance

(57) **Abstract**

This invention discloses immunostimulatory combinations of Tumor Necrosis Factor Receptor Superfamily (TNFRSF) agonists, Toll-Like Receptor (TLR) agonists, "domain present in NAIP, CIITA, HET-E, TP-1(NACHT)-Leucine Rich Repeat (LRR)" or "NLR" agonists, RIG-I-Like Helicase or "RLH" agonists, purinergic receptor agonists and cytokine/chemokine receptor agonists, together with delivery methods. The combinations, when used alone at the site of pathology, provide immunostimulation that induces host humoral and cellular immunologic responses to eliminate pathogens or neoplasms. Alternatively, when the combinations are used with a defined antigens, these combinations can induce focused humoral and cellular immunologic responses useful as prophylactic and/or ameliorative therapeutic modalities for infections and the treatment of neoplastic disorders.

## Description

This invention was made in part with government support under Grant No. 1R21AI063982-01A1 awarded by the National Institutes of Health. The government has certain rights in this invention.

### BACKGROUND OF THE INVENTION

### FIELD OF THE INVENTION

The invention relates generally to immunostimulatory combinations of molecular adjuvants and genetic vaccines, to include DNA vaccines, and more specifically, to combinations of Tumor Necrosis Factor Receptor Superfamily (TNFSF) agonists, Toll-Like Receptor (TLR) agonists, "domain present in NAIP, CHTA, HET-E, TP-1(NACHT)-Leucine Rich Repeat (LRR)" or "NLR" agonists, RIG-Like Helicases (RHR), purinergic receptor, and cytokine/chemokine receptor agonists as immunotherapeutic modalities.

### BACKGROUND INFORMATION

Many viruses, bacteria, and tumors express antigens that the immune system can potential use as a way to recognize these agents, however, often times these antigens fail to elicit an effective immune response. Failure of dendritic cells (DCs) to recognize many of these live agents continues to pose problems in developing effective vaccination strategies to treat infectious and/or neoplastic disorders. Further, vaccines generated by live agents pose a threat to the host, thus in the absence of attenuation, such live vaccine modalities have fallen into disfavor as potential immunotherapeutics.

In general, vaccines comprise an antigen combined with an activator of antigen-presenting cells (especially dendritic cells) which is termed an adjuvant. If the antigen is already present in the host, then many times an immune response can be created simply by administering the adjuvant. The antigen is taken-up by DCs and the adjuvant activates the DCs by interacting with various cell surface receptors (e.g., CD40 or Toll-like receptors, TLRs).

Tumor antigens are presented by DCs within tumors, but tumor-produced factors suppress DC antigen presentation. CD40 ligand (CD40L, CD154, TNFSF5), the natural ligand for CD40, has been used to stimulate DCs within tumors. CD40L, an immunostimulatory member of the TNF superfamily (TNFSF), is produced as a trimeric Type II membrane protein.

DNA vaccines can be used to present antigens and/or induce CD40 stimulation, and such vaccines have been shown to elicit appropriate immune responses. However, plasmids encoding full-length membrane CD40L usually do not augment immune responses to DNA vaccines. Consequently, a number of studies have utilized a soluble 1 trimer form of CD40L (sCD40LT, originally produced by Immunex). However, full CD40L stimulation requires the clustering of receptors in the plane of the membrane, which can only be achieved by multimeric forms of CD40L, reinforcing the observation that TNFSFs in general require multimerization beyond the single trimer level to fully stimulate their corresponding cell types.

As intimated above, there are several ways to stimulate DCs: by stimulating their CD40 receptor or by stimulating their receptors for Toll-like receptor (TLR) agonists. Important TLR agonists include CpG-rich oligonucleotides and the double-stranded RNA mimic, polyinosinic acid:polycytidylic acid (poly-I:C). CpG signals DCs using the MyD88 pathway, whereas poly-I:C uses the TRIF pathway. Recent reports indicate that combining CD40 stimulation plus a single TLR agonist stimulates strong immune responses. Similarly, other reports indicate that combining two TLR agonists together (e.g., CpG + poly-I: C), yields markedly greater responses.

Similar to observations for some infectious agents (e.g., HIV), it has been very difficult to treat model tumors in mice with adjuvant/DNA vaccination modalities. This is especially true in mice presenting B16-F10 tumors. Presently, established B16-F10 tumors can be cured in mice by combining immune cell depletion, infusion of transgenic antitumor CD8+ T cell, vaccination with Vaccinia expressing melanoma antigen, and IL-2. However, in the absence of the above steps, using plasmids encoding adjuvants that augment immune responses to DNA vaccines for the treatment of tumors has met with limited success.

Immunostimulants contribute to vaccine efficacy by upregulating co-stimulatory molecules, inducing supportive cytokines, and favoring immunological memory. Ideally, vaccines should be safe, strongly protective, and durable enough to reduce the need for frequent revaccination.

For more than a century immunotherapy for cancer has offered the promise of cure for tumors and metastases with little or no damage to normal tissues. Despite a record of repeated failures to deliver on that promise, there is a renewed interest in cancer immunotherapy, including vaccinating patients with specific tumor antigens, tumor cells modified to express cytokines, or dendritic cells that present tumor antigens.

If the immune system is capable of recognizing tumor cells without vaccination, then the only element that would be lacking is a strong adjuvant to augment the antitumor response. Several lines of investigation provide evidence that this is the case: (1) CD8+ T cells can be measured in untreated patients with cancer, directed against such antigens as MUC-1, HER-2/neu, gp100, and telomerase; (2) tumor-infiltrating lymphocytes (TIL) from unvaccinated patients can be expanded ex vivo and reinfused into patients, resulting in occasional dramatic responses even in metastatic disease; (3) nonspecific immunostimulants such CTLA-4 blockade can elicit effective antitumor responses; and (4) when immune responses are induced by treatment, patients often respond by producing large numbers of CD8+ T cells with different specificities than were used in treatment, so-called "epitope spreading".

Tumor cells produce a variety of substances that inactivate the immune response. These substances include IL-10, TGF-β, PGE2, and even the angiogenic factor, VEGF. However, anti-tumor CD8+ T cells can be found both within tumors and their draining lymph nodes. These cells require stimuli from mature dendritic cells (DCs) in order to gain effector function, and there is evidence that tumor-associated DCs present tumor antigens. However, the DCs within cancers are typically not mature because of suppressive substances produced by the tumors including IL-10, TNF-β, PGE2, and VEGF described above. Such immature DCs can lead to immunological tolerance mediated by immunosuppressive regulatory T cells (i.e., Tregs). Tregs suppress effective CD8+ T cell responses. Removing or inactivating Tregs augments antitumor immunity. Alternatively, stimuli to mature DCs can lead to effective anti-tumor immunity, exemplified by stimulation of DCs through CD40.

Many approaches have been used to exploit immunostimulants to augment antitumor therapy. For example, repeated peritumoral injections of a "naked" plasmid DNA for IL-12 may control tumor growth in mice. However, DNA vaccines vary in their ability to elicit antibody responses.

### SUMMARY OF THE INVENTION

The present invention relates to immunostimulatory combinations of Tumor Necrosis Factor Receptor Superfamily (TNFRSF) agonists, Toll-Like Receptor (TLR) agonists, "domain present in NAIP, CIITA, HET-E, TP-1(NACHT)-Leucine Rich Repeat (LRR)" or "NLR" agonists, RHR agonists, purinergic receptor agonists, purinergic receptor, and cytokine/chemokine receptor agonists. When used alone at the site of pathology, these combinations provide immunostimulation that induces a host immune response to eliminate pathogens or neoplasms. When used with defined antigens, these combinations can produce focused responses useful as vaccines and for the treatment of neoplastic disorders.

In one embodiment, stimulation of TNFRSF receptors is accomplished via compositions comprising soluble, multimeric tumor necrosis factor superfamily (TNFSF) ligands (e.g., CD40L and glucocorticoid-induced TNFR-related gene ligand (GITRL)) as useful molecular adjuvants for DNA vaccines.

In a related aspect, compositions are disclosed including a nucleic acid encoding a fusion protein comprising a soluble tumor necrosis factor receptor superfamily (TNFRSF) ligand and a multimerizing polypeptide, at least two TLR agonists, and a cationic polymer. Further, such multimerizing polypeptides include, for example, members of the C1q and collectin families, such as Acrp30 or surfactant protein-D (SP-D).

In a related aspect, such compositions may include polymers of the general formula (I):

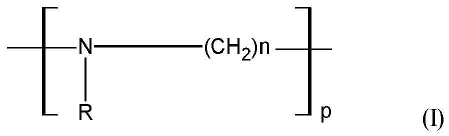

where R is a hydrogen atom or a group of formula

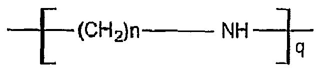

and where the R group is attached to the (CH₂) end to the N atom in the main formula, n is an integer between 2 and 10, and p and q are integers, in which the sum of p + q is such that the average molecular weight of the polymer is between 100 and 10⁷.

In another related aspect, the TNFRSF ligand includes, but is not limited to, CD40L, glucocorticoid-induced TNFR-related gene ligand (GITRL), NGF, GD137L/4-1BBL, TNF-alpha, CD143L/OX40L, CD27L/CD70, FasL, CD30L, TNF-beta/LT-alpha, LT-beta, RANKL, LIGHT, and TRAIL.

In a related aspect, such compositions may include, but are not limited to, an antigen such as MAGE-1, MAGE-2, (MUC-1, tyrosinase, surface Ig, cyclin dependent kinase 4, β-catenin, caspase-8, HPV type 16, E6 and E7 proteins, CD5, CAMPATH-1, CEA, EGFR, FAP-α, tenascin, metalloproteinases, H1V-1 gag, HIV-1 nef, HIV-1 env, HIV-1 gp41-1, HIV-1 p24, HIV-1 gp 120, HIV-2 env, HIV-2 gp 36, HBsAg, HCV core, HCV NS4, HCV NS3, HCV p22 nucleocapsid, HCV NS5, Influenza A, Influenza B, SARS associated spike mosaic S(N), SARS associated spike mosaic S(M), and SARS associated Coronavirus nucleocapsid.

In another embodiment, a method of inducing proliferation of a cell population containing effector T-cells in a subject is disclosed, including contacting the cells of the subject with a composition comprising a nucleic acid encoding a fusion protein comprising a soluble tumor necrosis factor receptor superfamily (TNFRSF) ligand and a multimerizing polypeptide, at least two TLR agonists, and a cationic polymer.

In a related aspect, cells are contacted *ex vivo* and subsequently administered to the subject, alternatively, cells are contacted by administering the composition to the subject. Further, the method may include administering the composition directly into or around a tumor presented by the subject.

In another embodiment, a method of treating a cell proliferation disorder is disclosed, including administering a therapeutically effective amount of a pharmaceutically acceptable composition comprising a cationic polymer mixed with a nucleic acid encoding a fusion protein comprising a soluble tumor necrosis factor receptor superfamily (TNFRSF) ligand and a multimerizing polypeptide, combined with at least two TLR agonists, a cationic polymer, in a pharmaceutically acceptable carrier.

In a related aspect, the cell proliferation disorder is cancer and may include extracting cells from the subject, wherein the cells comprise T lymphocytes (T cells), combining the cells with the composition ex vivo, and administering the mixture to the subject. Alternatively, the composition is administered directly into or around a tumor.

In another embodiment, a pharmaceutical composition is disclosed including a pharmaceutically acceptable carrier and a composition comprising a nucleic acid encoding a fusion protein comprising a soluble tumor necrosis factor receptor superfamily (TNFRSF) ligand and a multimerizing polypeptide, at least two TLR agonists, and a cationic polymer.

In another embodiment, a vaccine is disclosed including a nucleic acid encoding a fusion protein comprising a soluble tumor necrosis factor receptor superfamily (TNFRSF) ligand and a multimerizing polypeptide, at least two TLR agonists, a cationic polymer, an antigen including, but not limited to, MAGE-1, MAGE-2, MUC-1, tyrosinase, surface Ig, cyclin dependent kinase 4, β-catenin, caspase-8, HPV type 16, E6 and E7 proteins, CD5, CAMPATH-1, CEA, EGFR, FAP-α, tenascin, metalloproteinases, HIV-1 gag, HN-1 nef, HIV-1 env, HIV-1 gp41-1, HIV-1 p24, HIV-1 gp 120, HIV-2 env, HN-2 gp 36, HCV core, HCV NS4, HCV NS3, HCV p22 nucleocapsid, HCV NS5, Influenza A, Influenza B, SARS associated spike mosaic S(N), SARS associated spike mosaic S(M), and SARS associated Coronavirus nucleocapsid, and a pharmaceutically acceptable carrier.

In a related aspect, the subject presents an infectious disorder or a neoplastic disorder, where the administration is prophylactic or ameliorative.

In one embodiment, a DNA vaccine containing a combination of one or more nucleic acids is disclosed, where the nucleic acids encode one or more Tumor Necrosis Factor Receptor Superfamily (TNFRSF) agonists and one or more agonists selected from the group consisting of Toll-Like Receptor (TLR) agonists, domain present in NAIP, CIITA, HET-E, TP-1(NACHT)-Leucine Rich Repeat (LRR) or NLR agonists, RIG-Like Helices or RHR agonists, purinergic receptor agonists, cytokine/chemokine receptor agonists, and combinations thereof.

In a related aspect, the one or more nucleic acids encode one or more TNFRSF agonists, one or more TLR agonists, one or more NLR agonists, one or more RHR agonists, one or more purinergic receptor agonists, and one or more cytokine/chemokine receptor agonists. In another aspect, the one or more nucleic acids encode one or more TNFRSF agonists, one or more TLR agonists, and one or more NLR agonists. In another aspect, the one or more nucleic acids encode one or more TNFRSF agonists, one or more TLR agonists, one or more NLR agonists; and one or more cytokine/chemokine receptor agonists.

In another embodiment, a composition is disclosed including a DNA vaccine, one or more polypeptide agonists which includes, but is not limited to, TNFRSF agonists, TLR agonists, RIG-Like Helicases or RHR agonists, purinergic receptor agonists, and cytokine/chemokine receptor agonists, and/or one or more NLR agonists, where the NLR agonist includes, but is not limited to, double stranded RNA, gamma-D-Glu-meso-diaminopimelic acid (DAP) or its derivatives, muramyl dipeptide (MDP) or its derivatives, bacterial DNA and extracellular ATP (ATPe).

In one aspect, the DNA vaccine or composition is used in eliciting an immunotherapeutic response to an infection or neoplastic disease, whereby expression of the combination of agonists in a vertebrate cell elicits a humoral immune response and/or a cell-mediated response, against the infection or neoplastic disease.

In another aspect, the DNA vaccine or composition is used for the manufacture of a medicament for use in eliciting an immunotherapeutic response to an infection or neoplastic disease, whereby expression of the combination of agonists in a vertebrate cell elicits a humoral immune response and/or a cell-mediated response, against the infection or neoplastic disease.

In one embodiment, a method of treating an infection or neoplastic disease is disclosed, including administering a therapeutically effective amount of the DNA vaccine or composition to a subject in need thereof. In a related aspect, the method includes administering an antigen associated with the infection or neoplastic disease.

Exemplary methods and compositions according to this invention, are described in greater detail below.

### BRIEF DESCRIPTION OF THE DR-AWINGS

Figure 1 illustrates soluble multimeric CD40L fusion proteins.

Figure 2 demonstrates that soluble multimeric CD40L is more active than a single trimer in an HIV-1 Gag DNA vaccine (1 < 2 < 4 trimers).

Figure 3 demonstrates that in addition to CD40L, multimeric GITRL increases CTL activity of an HIV Gag vaccine.

Figure 4 demonstrates that an established A20 lymphoma tumor can be cured by peritumoral plasmid injections.

Figure 5 shows the survival benefit of multimeric TNFSF ligands for A20 lymphoma.

Figure 6 demonstrates that TLR 3 stimulation (PolyI:C) synergizes with CD40L against established B16-F10 tumors.

Figure 7 compares the effects of PBS, a control plasmid, and a plasmid containing 2 CD40L trimers on established B16-F10 tumors.

Figure 8 demonstrates that TLR 9 stimulation (CpG) synergizes with CD40L against established B16-F10 tumors.

Figure 9 demonstrates that CD40 stimulation synergizes with TLR 9 against established B16-F10 tumors.

Figure 10 demonstrates that CD40L, TLR 3, and TLR 9 stimulation in combination with JetPEI is effective against established B16-F10 tumors.

Figure 11 shows the survival benefit of the CD40L, TLR 3/TLR 9 agonists, JetPEI combination for B16-F10 tumors.

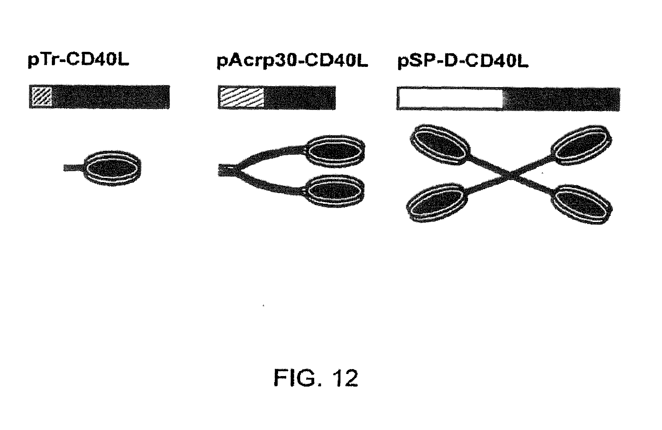

Figure 12 shows 1-, 2-, and 4-trimer soluble forms of the TNFRSF agonist, CD40L. The extracellular domain (ECD) of murine CD40L was fused to an isoleucine zipper to make 1-trimer soluble CD40L. The ECD of murine CD40L was fused to the body of murine Acrp30 (adiponectin) to make 2-trimer soluble CD40L. The ECD of murine CD40L was fused to the body of murine surfactant protein D (SP-D) to make 4-trimer soluble CD40L. The respective plasmid DNAs are termed pTr-CD40L, pAcrp30-CD40L, and pSP-D-CD40L. Similar 4-trimer constructs were made for GITRL (pSP-D-GITRL), RANKL (pSP-D-RANKL), 4-1BBL (pSP-D-4-1BBL), OX40L (pSP-D-OX40L), CD27L/CD70 (pSP-D-CD27L/CD70), BAFF (pSP-D-BAFF) and LIGHT (pSP-D-LIGHT).

Figure 13 demonstrates the adjuvant activity of soluble CD40L in a vaccine is related the valence of the trimers. Plasmids for 1-, 2-, and 4-trimer forms of soluble CD40L were combined with an antigen plasmid for secreted HIV-1 Gag (pScGag) and used to immunize mice in groups of 5.

Figure 14 illustrates the synergy between a TNFRSF agonist and a TLR agonist in a vaccine for malaria. Mice were vaccinated with a DNA vaccine encoding a codon-optimized, secreted form of merozoite surface protein-1 (pMSP-1) or empty vector, pcDNA3.1. As an adjuvant, a plasmid DNA for 4-trimer GITRL (TNFSF18), pSP-D-GITRL, was also used with or without added CpG-ODN. Then the mice were challenged with an injection of Plasmodium yoelii-parasitized red blood cells.

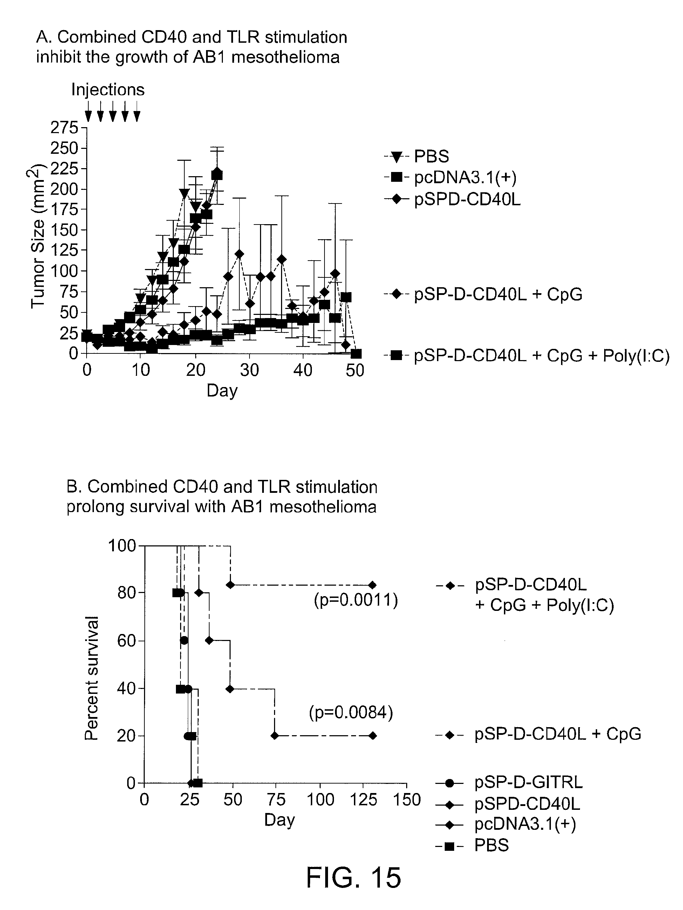

Figure 15 illustrates the synergy between a TNFRSF agonist and two TLR agonists in a tumor immunotherapy protocol against mesothelioma. Mice were injected with mesothelioma cells and a tumor was allowed to form. When the tumor reached ≥ 4 mm in diameter, it was injected on days 0, 2, 4, 6, and 8 (arrows in Panel A) with 50 µl of phosphate-buffer containing 50 µg of either empty plasmid (pcDNA3.1), 4-trimer CD40L (pSP-D-CD40L), or pSP-D-CD40L, and on days 1, 3, 5, 7, and 9 with 50 µl of phosphate-buffered saline containing 25 ug of CpG-ODN 1018 (i.e., a phosphorothioate-linked oligonucleotide having the sequence 5'-TGAACTGTGAACGTTCGAGATTGA-3': SEQ ID NO:7) with or without 25 ug Poly(I:C).

Figure 16 illustrates the synergy between a TNFRSF agonist and two TLR agonists in a tumor immunotherapy protocol against melanoma. Mice were injected with B16-F10 melanoma cells and a tumor was allowed to form. When the tumor reached ≥ 4 mm in diameter, it was injected on days 0, 2, 4, 6, and 8 (arrows in Panel A) with 50 µl of phosphate-buffered saline containing 50 ug of either empty plasmid (pcDNA3.1), 4-trimer CD40L (pSP-D-CD40L), or pSP-D-CD40L, and on days 1, 3, 5, 7, and 9 with 50 µl of phosphate-buffered saline containing 25 ug of CpG-ODN 1018 with or without 25 ug Poly(I:C).

Figure 17 demonstrates the beneficial effect of polyethylenimine on the effectiveness of the plasmid for the TNFRSF agonist given on days 0, 2, 4, 6, and 8, where two TLR agonists in a tumor immunotherapy protocol against melanoma. Mice were given B16-F10 tumors as described in Fig. 16.

Figure 18 illustrates the synergy between a TNFRSF agonist and a cytokine/chemokine receptor agonist in a tumor immunotherapy protocol against melanoma. Mice were injected with B16-F10 melanoma cells and a tumor was allowed to form. When the tumor reached ≥ 4 mm in diameter, it was injected on days 0, 2, 4, 6, and 8 (arrows in Panel A) with 50 µl of phosphate-buffered saline containing either 50 ug of empty plasmid (pcDNA3.1), 4-trimer CD40L (pSP-D-CD40L), and/or a plasmid for MIP-3 alpha (also called CCL20), pMIP3alpha.

Figure 19 illustrates the transfection of muscle and high density expression of correctly folded Env, where after cytolysis, muscle cell fragments move to draining lymph nodes.

Figure 20 graphically illustrates the relative titer of anti-Env IgG from pEnv vaccinated mice.

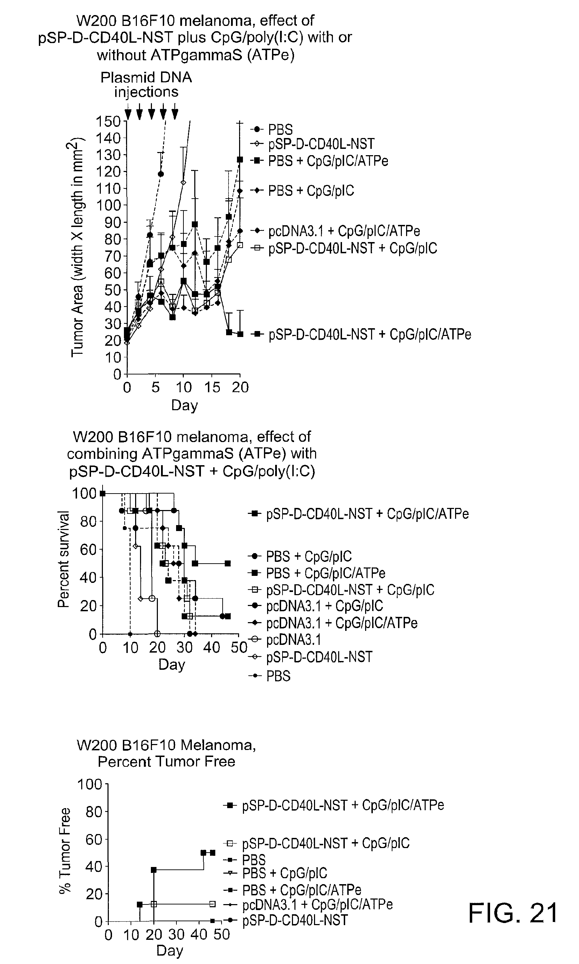

Figure 21 graphically illustrates the synergistic effects of molecular adjuvants and extracellular ATP (ATPe)), a purinergic receptor agonist. Mice were injected with B16-F10 melanoma cells and a tumor was allowed to form. When the tumor reached ≥ 4 mm in diameter, it was injected on days 0, 2, 4, 6, and 8 (arrows in Panel A) with 50 ul of phosphate-buffered saline (PBS) alone orPBS containing 50 ug either empty plasmid (pcDNA3.1) or a plasmid encoding 4-trimer CD40L (pSP-D-CD40L-NST) on days 0, 2, 4, 6, and 8. Also, as indicated, some experimental arms received 50 ul of phosphate-buffered saline containing 25 ug of CpG-ODN 1018 with or without 25 ug Poly(I:C) with or without 100 uM ATPgammaS on days 1, 3,5, 7 and 9. Panel B shows the effects of these treatments on survival, where mice were euthanized if the tumors became larger than 15 mm in mean diameter, ulcerated, or the mice became moribund. Panel C shows the effects of these treatments on the local tumors. Since all of the mice had tumors initially, 0% were tumor-free at day 0 of treatment. The Y-axis shows the % of mice that became tumor-free following treatment in each condition.

### DETAILED DESCRIPTION OF THE INVENTION

Before the present composition, methods, and treatment methodology are described, it is to be understood that this invention is not limited to particular compositions, methods, and experimental conditions described, as such compositions, methods, and conditions may vary. It is also to be understood that the terminology used herein is for purposes of describing particular embodiments only, and is not intended to be limiting, since the scope of the present invention will be limited only in the appended claims.

As used in this specification and the appended claims, the singular forms "a", "an", and "the" include plural references unless the context clearly dictates otherwise. Thus, for example, references to "the method" includes one or more methods, and/or steps of the type described herein which will become apparent to those persons skilled in the art upon reading this disclosure and so forth.

Unless defined otherwise, all technical and scientific terms used herein have the same meaning as commonly understood by one of ordinary skill in the art to which this invention belongs. Although any methods and materials similar or equivalent to those described herein can be used in the practice or testing of the invention, the preferred methods and materials are now described.

The practice of the present invention will employ, unless indicated specifically to the contrary, conventional methods of virology, immunology, microbiology, molecular biology and recombinant DNA techniques within the skill of the art, many of which are described below for the purpose of illustration. Such techniques are explained fully in the literature. See, e.g., Sambrook, et al. Molecular Cloning: A Laboratory Manual (2nd Edition, 1989); Maniatis et al. Molecular Cloning: A Laboratory Manual (1982); DNA Cloning: A Practical Approach, vol. I & II (D. Glover, ed.); Oligonucleotide Synthesis (N. Gait, ed., 1984); Nucleic Acid Hybridization (B. Hames & S. Higgins, eds., 1985); Transcription and Translation (B. Hames & S. Higgins, eds., 1984); Animal Cell Culture (R. Freshney, ed., 1986); Perbal, A Practical Guide to Molecular Cloning (1984).

The term "agonist" refers to a protein, nucleic acid, lipid, carbohydrate, and/or chemical substance that interacts with a cell receptor to produce a stimulatory signal on a cell. Special preference is given to agonists that stimulate immune cells.

The term "antigen" refers to substances from microbes (bacteria, fungi, protozoa, or viruses) or endogenous substances against which a specific immune response can be generated. Examples of microbial antigens include proteins from HIV (Gag, Env, Nef), influenza (HA, N), plasmodia (CSP-1, MSP-1), hepatitis B, hepatitis C, papillomavirus, herpes viruses, orthopoxviruses (such as variola, the agent of smallpox). Carbohydrates can also be antigens, such as those from Streptococci, Meningococci, Mycobacteria, Mycoplasma, Clamydia, Franciscella, Pasteurella, Legionella, and the Bacillus species (including B. anthracis). Lipids can be antigens, such as glycolipids. Nucleic acids can also be antigens. Additionally, complex substances composed of protein, carbohydrate, lipid and/or nucleic acid can be antigens. For endogenous antigens, preference is given to those specific to tumors. Other endogenous antigens include abnormal proteins such as prions or aggregated proteins such as those found in the plaques of Alzheimer's disease.

Antigens can also be directly connected with any of the immunostimulants in this application. The connection can be through a covalent bond or non-covalent binding interactions (such as electrostatic interactions between CpG ODN and a positively charged peptide antigen). As an example, protein antigens can be covalently joined to CpG-containing immunostimulatory oligodeoxynucleotides (ISS-ODNs) or other TLR agonists. Upon vaccination by a suitable method, these antigen-immunostimulant compounds can generate an immune response greater than that generated when the two components are used together without being connected. For example, proteins can be joined to ISS-ODN to generate a stronger vaccine response. Similarly, protein antigens can be covalently bonded to TLR agonist to give a stronger vaccine response.

Antigens can be used in vaccines to either treat or prevent a disease. They can also be used to generate specific immune substances, such as antibodies, which can be used in diagnostic tests or kits. The subject of an antigen-containing vaccine are typically vertebrates, preferably a mammal, more preferably a human.

For the purposes of this description, an antigen is defined as any protein, carbohydrate, lipid, nucleic acid, or mixture of these, or a plurality of these, to which an immune response is desired. It is important to note that it is not always necessary that the antigen be identified in molecular terms. For example, immune responses to tumors can be generated without knowing either in advance or post-hoc which molecules the immune response is directed against. In these cases, the term antigen refers to the substance or substances, known or not known, toward which a specific immune response is directed. The specificity of the immune response provides an operational definition of an antigen, such that immunity generated against one type of tumor may be specific for that tumor type but not another tumor type.

The term "synergy" refers to an activity of administering combinations of proteins, lipids, nucleic acids, carbohydrates, or chemical compounds that is greater than the additive activity of the proteins, lipids, nucleic acids, carbohydrates, or chemical compounds if administered individually.

The term "co-administered" refers to two or more proteins, lipids, nucleic acids, carbohydrates, or chemical compounds of a combination that are administered so that the therapeutic or prophylactic effects of the combination can be greater than the therapeutic effect of either proteins, lipids, nucleic acids, carbohydrates, or chemical compounds administered alone. The two or more proteins, lipids, nucleic acids, carbohydrates, or chemical compounds can be administered simultaneously or sequentially. Simultaneously co-administered proteins, lipids, nucleic acids, carbohydrates, or chemical compounds may be provided in one or more pharmaceutically acceptable compositions. Sequential co-administration includes, but is not limited to, cases in which the proteins, lipids, nucleic acids, carbohydrates, or chemical compounds are administered so that each protein, lipid, nucleic acid, carbohydrate, or chemical compound can be present at the treatment site at the same time.

The term "adjuvant" refers to a compound or mixture that enhances the immune response to an antigen. An adjuvant can serve as a tissue depot that slowly releases the antigen and also as a lymphoid system activator that non-specifically enhances the immune response (Hood et al., Immunology, Second Ed., 1984, Benjamin/Cummings: Menlo Park, Calif., p. 384). Often, a primary challenge with an antigen alone, in the absence of an adjuvant, will fail to elicit a humoral or cellular immune response. Adjuvants include, but are not limited to, complete Freund's adjuvant, incomplete Freund's adjuvant, saponin, mineral gels such as aluminum hydroxide, surface active substances such as lysolecithin, pluronic polyols, polyanions, peptides, oil or hydrocarbon emulsions, keyhole limpet hemocyanins, dinitrophenol, and potentially useful human adjuvants such as BCG (bacille Calmette-Guerin) and Corynebacterium parvum. Preferably, the adjuvant is pharmaceutically acceptable.

In a related aspect, the term "molecular adjuvant" is defined as a protein, lipid, nucleic acid, carbohydrate, or chemical compound for which dendritic cells (DCs), macrophages, B cells, T cells, and/or NK cells have a known receptor whose occupancy leads to a defined sequence of intracellular signal transduction and a change in the phenotype resulting in an improvement in the quantity or quality of the ensuing immune response. In a related aspect, the cells as described above are collectively referred to as "immune cells."

The term "antigen-presenting cell" or "APC" refers to those highly specialized cells that can process antigens and display their peptide fragments on the cell surface together with molecules required for lymphocyte activation. The main antigen-presenting cells for T-cells are DC, macrophages, and B-cells, whereas the main antigen-presenting cells for B-cells are follicular dendritic cells.

The term "dendritic cell" or "DC" is defined as those APCs that are found in T-cell areas of lymphoid tissues. (Banchereau et al., Nature 392:245-251, 1998). DCs are a sparsely distributed, migratory group of bone-marrow-derived leukocytes that are specialized for the uptake, transport, processing and presentation of antigens to T-cells. Non-lymphoid tissues also contain DCs, but these do not stimulate T-cell responses until they are activated and migrate to lymphoid tissues. In general, dendritic cells may be identified based on their typical shape (stellate in situ, with marked cytoplasmic processes, dendrites, visible in vitro); their ability to take up, process and present antigens with high efficiency; and their ability to activate naive T-cell responses. For a general review of murine and human dendritic cells, see Shortman et al., Nat. Rev. Immunol. 2(3):151-61, 2002.

The term "immunogenic" refers to the ability of an antigen to elicit an immune response, either humoral or cell mediated. An "immunogenically effective amount" as used herein refers to the amount of antigen sufficient to elicit an immune response, either a cellular (T cell) or humoral (B cell or antibody) response, as measured by standard assays known to one skilled in the art. The effectiveness of an antigen as an immunogen, can be measured either by proliferation assays, by cytolytic assays, such as chromium release assays to measure the ability of a T cell to lyse its specific target cell, or by measuring the levels of B cell activity by measuring the levels of circulating antibodies specific for the antigen in serum, or by measuring the number of antibody spot-forming cells in the spleen. Furthermore, the level of protection of the immune response may be measured by challenging the immunized host with a replicating virus or cell containing the antigen that has been injected. For example, if the antigen to which an immune response is desired is a virus or a tumor cell, the level of protection induced by the "immunogenically effective amount" of the antigen is measured by detecting the level of survival after virus or tumor cell challenge of the animals. Alternatively, protection can also be measured as the reduction in viral replication or tumor growth following challenge of the animals.

As used herein, the term cytokine/chemokine receptor agonist refers to any of these immunostimulatory proteins. Preference is given to IL-1 (all members of this molecular family, but especially II,-1-beta), IL-2, IL-4, IL-12, IL-13, IL-15, IL-17, IL-18, CCL13, CCL20, IFN-alpha, IFN-beta, IFN-gamma, IFN-lambdal, IFN-lambda2, IFN-lambda3, and GM-CSF.

Cytokines and chemokines are proteins that affect the function of immune cells. A compendium of cytokines is given by Oppenheimer et al. in The Cytokine Reference database (hosted by Academic Press, Burlington MA, USA). Other listings of cytokines are: Cytokines and Cells Online Pathfinder Encyclopedia (COPE) (hosted by Horst Ibelgaufts, Institute of Biochemistry, MPI of Biochemistry, Munich Martinsried, FRG); The Cytokines Web (hosted by PSINIX Information Systems Ltd, China); and The Cytokine Handbook, Vol. 1 and 2, 4th Ed., 2003, (Thompson and Lotze, eds.), Academic Press, San Diego, CA. Some cytokines are termed interleukins (IL) and given a numerical designation such as IL-1 to IL-33. Other cytokines are termed interferons (IFN) and designated using Greek letters. Other cytokines are named for their properties, such as granulocyte-macrophage colony stimulating factor or GM-CSF. Chemokines are generally proteins that cause the chemoattraction of specific cell types. They are classified into four families based on the number and arrangement of cysteines within the proteins: CXC, C, CX3C, and CC. Chemokines include CXCL1-CXCL16, XCL1-XCL2, CX3CL1, and CCL1-CCL28. Special preference is given to CCL3, CCL4, CCL5, CCL13, and CCL20. CCL3 is also called macrophage inflammatory protein 1-alpha, which is a chemoattractant for cells bearing CC-chemokine receptor-1 (CCR1) and CCR5 on such as T cells. CCL4 is also called macrophage inflammatory protein 1-beta, which is a chemoattractant for cells bearing CCR5, such as T cells. CCL5 is also called RANTES, which is a chemoattractant for cells bearing CCR5, such as T cells. CCL3, CCL4, and CCL5 can also block many strains of the human immunodeficiency virus from entering CD4+ T cells and macrophages. CCL13, is also called macrophage chemotactic protein-4, which is a chemoattractant for cells bearing CCR2 and CCR3 such as blood monocytes or dendritic cell precursors. CCL20 is also called macrophage inflammatory protein 3-alpha and is a chemoattractant for cells bearing CCR6, such as immature dendritic cells. CCL20 and also CCL13 have been shown to augment antitumor responses. The use of polymeric delivery agents can augment the antitumor uses of nucleic acid injections, where the nucleic acid encodes these cytokines or chemokines.

As used herein, "vaccine' is defined as an immunostimulatory treatment designed to elicit an immune response against an antigen, whether administered prophylactically or for the treatment of an already existing condition.

In a related aspect, a "genetic vaccine" relates to the use of genetic material (e.g., nucleic acid sequences) encoding a protein of interest which is used as an immunizing agent. This term includes, but is not limited to, nucleic acids transported into host cells within viruses or viral vectors (e.g., modified forms of adenoviruses, poxviruses, rhabdoviruses, alphaviruses, herpesviruses, influenza viruses, retroviruses, or lentiviruses) or bacteria (e.g., modified forms of Salmonella species, Listeria species, or mycobacterial species). The term also includes, but is not limited to, nucleic acids administered directly, such as plasmid DNA, which is referred to as a "DNA vaccine." DNA encoding a protein of interest can also be administered in a non-plasmid form as a linear, double-stranded molecule as a minimalist expression construct. Messenger RNA (mRNA) encoding a protein of interest can also be directly administered. In one aspect, a nucleic acid encoding a protein of interest is administered as a DNA vaccine comprising a plasmid.

In one aspect, the present invention relates to the introduction of exogenous or foreign DNA molecules into an individual's tissues or cells, wherein these molecules encode an exogenous protein capable of eliciting an immune response to the protein. The exogenous nucleic acid sequences may be introduced alone or in the context of an expression vector wherein the sequences are operably linked to promoters and/or enhancers capable of regulating the expression of the encoded proteins. The introduction of exogenous nucleic acid sequences may be performed in the presence of a cell stimulating agent capable of enhancing the uptake or incorporation of the nucleic acid sequences into a cell. Such exogenous nucleic acid sequences may be administered in a composition comprising a biologically compatible or pharmaceutically acceptable carrier. The exogenous nucleic acid sequences may be administered by a variety of means, as described herein, and well known in the art. The DNA is linked to regulatory elements necessary for expression in the cells of the individual. Regulatory elements include a promoter and a polyadenylation signal. Other elements known to skilled artisans may also be included in genetic constructs of the invention, depending on the application. The following references pertain to methods for the direct introduction of nucleic acid sequences into a living animal: Nabel et al., (1990) Science 249:1285-1288; Wolfe et al., (1990) Science 247:1465-1468; Acsadi et al. (1991) Nature 352:815-818; Wolfe et al. (1991) BioTechniques 11(4):474-485; and Felgner and Rhodes, (1991) Nature 349:351-352. Such methods may be used to elicit immunity to a pathogen, absent the risk of infecting an individual with the pathogen. The present invention may be practiced using procedures known in the art, such as those described in PCT International Application Number PCT/US90/01515, wherein methods for immunizing an individual against pathogen infection by directly injecting polynucleotides into the individual's cells in a single step procedure are presented, and in U.S. Pat. Nos. 6,635,624; 6,586,409; 6,413,942; 6,406,705; 6,383,496.

"Enhanced effects" refers to an increased immune response to a vaccine or an increased antitumor response. This includes small increments in effectiveness, fully additive increases that are the sum of all of the component immunostimulants , or synergistic increases that are more than the sum of all of the components.

The NLR proteins form an expanding family. It is sometimes called the CATERPILLER family of proteins (where that term is an acromym for CARD, transcription enhancer, R(purine)-binding, pyrin, lots of leucine repeats). Many of the proteins in this molecular family contain a NACHT domain (where that term is an acronym for a domain present in NAIP, CIITA, HET-E, TP-1). Also, many of the proteins in this molecular family contain a LRR domain (where that term is an acronym for Leucine-Rich Repeat), and several contain pyrin domains. Other names for certain members of this family are NOD-LRR proteins (where NOD is an acronym for nucleotide-binding oligomerization domain). Another name for certain members of this family are NALPs (where the term is an acronym for a NACHT-LRR-PYD-containing protein).

An important feature of NLR stimulation is that it can lead to activation of caspase-1, also known as Interleukin-1-beta Converting Enzyme (ICE). While many stimuli of dendritic cells and macrophages can lead to the synthesis of pro-interleukin-1-beta, ICE is necessary to trim this inactive precursor into the fully mature interleukin-1-beta form (IL-1-beta). For example, CD40L alone does not induce dendritic cells to release significant amounts of IL-1-beta. Similarly, ICE is also needed to convert pro-IL-18 to active IL-18. IL-1-beta and IL-18 are important in activating several types of immune cells, which makes NLR agonists useful components of vaccines and immunostimulators. For example, IL-1-beta synergizes with CD40L to induce human monocyte-derived dendritic cells to make IL-12.

TNFRSF agonists are defined as any substance that interacts with a TNF Receptor SuperFamily (TNFRSF) member to cause a cell-activating response. Typically, this will be a form of the cognate ligand for the TNFRSF receptor, such as a TNF SuperFamily (TNFSF) ligand (e.g., the TNFSF ligand for the CD40 receptor is typically CD40 ligand or CD40L). The gene symbols and common names of exemplary TNFSF ligands are TNF, LTA (lymphotoxin A), LTB (lyniphotoxin B), TNFSF4 (OX-40L), TNFSF5 (CD40L), TNFSF6 (FasL), TNFSF7 (CD27L or CD70 or CD27L/CD70), TNFSF8 (CD30L), TNFSF9 (4-1BBL), TNFSF10 (TRAIL), TNFSF11 (RANKL), TNFSF12 (TWEAK), TNFSF13A (APRIL), TNFSF13B (BAFF), TNFSF14 (LIGHT), TNFSF15 (VEGI), TNFSF18 (GITRL), and others. An updated listing of TNFSF ligands is maintained by the National Center for Biotechnology Information, U.S. National Library of Medicine, Bethesda, MD, USA.

Rather than restrict the definition of TNFRSF agonists to the TNFSF molecules themselves, this application uses 'TNFRSF agonist' as a more general term for any substance that interacts with a TNFRSF receptors to cause a cell-activating response. An online database of TNFRSF receptors is maintained by the Human Genome Nomenclature Committee. Preference is given to multimeric soluble forms of TNFRSF agonists, such as those formed by constructing fusion proteins between the body of collectin or C1q family members and the extracellular domains of the TNFSFs. The construction of these multimeric soluble TNFSFs is art recognized. However, any other substances that interact with TNFRSF receptors to induce a cellular response are also indicated by the term TNFRSF agonist for the purposes of this application. Such substances include the following, with preference given to methods for delivering these TNFRSF agonists in a multimeric soluble form or a form that effectively clusters the TNFRSF receptor in the responding cell type: (1) heat shock proteins (Hsp) such as Hsp70 or peptides derived from Hsp70 that have been reported to activate cells by interacting with CD40. Consequently, Hsps that may interact with TNFRSF receptors are being tested in vaccines and as antitumor agents by Antigenics, Inc. and as derivatives of Hsp70. (2) C4BP or a peptide derived from it has been reported to activate cells by interacting with CD40. (3) Certain anti-receptor antibodies can activate TNFRSF receptors, in which case they are termed "agonistic antibodies." For murine CD40, such agonistic antibodies include monoclonal antibodies IC10, FGK4.5, and their derivatives. For human CD40, such agonistic antibodies include monoclonal antibodies B-B20 (Diaclone, Besançon, France), Mab89 (Immunotech), G28-5 (American Type Culture Collection, Manassas, VA), clone 64 (Tanox Pharma, Amsterdam, The Netherlands), CP-870,893 (Pfizer Ine., New York, NY) and their derivatives. Preference is especially given to derivatives that contain the complementarity determining regions (CDRs) of such agonistic antibodies or variants of these CDRs, whether or not they contain other portions of an antibody molecule. (4) Another type of antireceptor antibody can augment the interaction of suboptimal levels of TNFSF ligands with their TNFRSF receptors. An example is S2C6 (SGN-40, Seattle Genetics, Inc.) which acts to stabilize the interaction of CD40L with the CD40 receptor, and thereby enhance the cellular response to CD40L. (5) Agonistic compounds can also be produced by random mutagenesis methods followed by selection for binding to and activation of a TNFRSF receptor. Such random mutagenesis methods include phage display, peptide libraries, and RNA or DNA aptamer techniques. (6) TNFRSF agonists (including those described above such as C4BP, anti-receptor antibodies, and receptor-binding peptides or aptamers) can also be attached to a multimerizing scaffold.

Examples of such scaffolds include: (1) the "valency platform"; (2) the "multimeric biopolymers"; (3) the "molecular scaffolds"; (4) the multimerization techniques; (5) polyethylene oxide-based multimerizing scaffolds; (6) the oil body-based multimers; (7) multimerizing scaffolds comprised of units with complementary surfaces; (8) the hetero-oligomer scaffolds; (9) genetic fusions between a multimerizing component and another component; (10) tethered ligands; and (11) constructing C3 symmetric peptide scaffolds containing the three "hot spot" residues for the binding of CD40L to CD40 (Lys-GlyTyr-Tyr) (SEQ ID NO: 8).

Using intracellular portions of the CD40, Fas, or other TNFRSF receptor introduced into cells, these molecules can then be selectively multimerized to deliver a signal. For example, when the intracellular signaling portion of CD40 is introduced into dendritic cells and then multimerized, the dendritic cells become activated to present tumor antigens and eradicate tumors. In consideration of these many approaches to stimulating TNFRSF receptors, the term TNFRSF agonist is defined herein to encompass any or all of these activators of TNFRSF receptors and their functional equivalents or derivatives.

Substances that bind to and activate TLRs are called TLR ligands or, equivalently, TLR agonists. It is important to note that there are differences between humans and mice as to which TLR will be activated by any given agonist, and this must be taken into account depending upon the species used. Without restricting this description to any particular embodiments, the following TLR agonists are suitable examples for the immunostimulatory combinations described herein: TLR2/1 agonists and TLR2/6 agonists: TLR2 is typically a heteromeric receptor found in combination with either TLR1 or TLR6. Bacterial lipopeptides are the main agonists for TLR2-containing receptors. These agonists include: mycoplasmal macrophage-activating lipoprotein-2; tripalmitoyl-cysteinyl-seryl-(lysyl)3-lysine (P3CSK4), dipalmitoyl-CSK4 (P2-CSK4), and monopalmitoyl-CSK4 (PCSK4); the tripalmitoyl-S-glyceryl-cysteine (Pam(3)Cys)-modified lipoproteins, including OspA from the Lyme disease spirochete Borrelia burgdorferi; mycobacterial cell wall fractions enriched for lipoarrabinomannan, mycolylarabinogalactan-peptidoglycan complex, or M. tuberculosis total lipids.

TLR3 agonists signal through the TRIF pathway to generate cytokines. The administration of viral genomes or partial genomes that generate dsRNA is another means of activating these pathways. In some cases, even endogenous messenger RNA (mRNA) can stimulate TLR3, and bacterial RNA can be especially stimulatory for dendritic cells. It has also been suggested that RNA stimulates dendritic cells through a nucleotide receptor.

While viral double stranded RNAs (dsRNAs) can be used to stimulate TLR3, the best tested TLR3 agonist is polyriboinosinic-polyribocytidylic acid or Poly(I:C) which is a synthetic form of dsRNA. Poly(I:C) has antitumor effects in mice at a dose of 100 ug intraperitoneally or intravenously and has been extensively tested in humans with cancer. Poly(I:C) was shown to ameliorate herpes simplex keratoconjunctivitis in mice and to reduce the growth of Leishmania in mouse cells. For peptide vaccination, Poly(I:C) was used at a dose of 50 ug subcutaneously. In humans with herpes simplex infection and cancer, Poly(I:C) has been used at a dose of 3-12 mg/kg. Ampligen (poly I:poly C12U) is a mismatched form of dsRNA that has also been tested.

TLR4 can signal cells through both the MyD88 and the TRIF pathways. Its special utility in activating human dendritic cells is art recognized. The classic agonist for TLR4 is bacterial lipopolysaccharide (LPS), which refers to a family of substances containing lipid A and its cogeners. An exemplary form of LPS is E. coli B:O111 (Sigma Chemicals). However, in an effort to make a less toxic form of TLR4 agonist, monophosphoryl lipid A (MPL) compounds have been produced and some are active in humans. The synthetic adjuvant, ASO2 (GlaxoSmithKline, United Kingdom), contains MPL as a component.

The principal agonist for TLR5 is bacterial flagellin.

For TLR7 agonists, these include, but are not limited to, single-stranded RNA; imidazoquinoline compounds such as resiquimod and imiquimod; Loxoribine (7-allyl-7,8-dihydro-8-oxo-guanosine) and related compounds; 7-Thia-8-oxoguanosine, 7-deazaguanosine, and related guanosine analogs; ANA975 (Anadys Pharmaceuticals) and related compounds; SM-360320 (Sumimoto); 3M-01 and 3M-03 (3M Pharmaceuticals); and adenosine analogs such as UC-1V150 (Jin et al., Bioorganic Medicinal Chem Lett (2006) 16:4559-4563, compound 4). It has been observed that TLR7 agonists directly activate plasmacytoid dendritic cells to make IFN-alpha, whereas TLR8 agonists directly activate myeloid dendritic cells, monocytes, and monocyte-derived dendritic cells to make proinflammatory cytokines and chemokines, such as TNF, IL-12, and MIP-1. Nevertheless, many compounds are agonists for both TLR7 and TLR8.

As noted above, many of the compounds that activate TLR7 also activate TLR8. 3M-03 activates both TLR7 and TLR8, but 3M-02 is more specific for TLR8. Again, many compounds are agonists for both TLR7 and TLR8. Poly-G containing 10 guanosine nucleosides connected by phosphorothioate linkages (Poly-G10) is also a TLR8 agonist that may be especially useful as a substance that shuts off the immunosuppressive functions of regulator CD4+CD25+ T cells.

Immunostimulatory oligonucleotides or polynucleotides such as CpG-containing oligodeoxynucleotides (CpG ODN) are the prototype agonists for TLR9. More generally, they are called immunostimulatory sequences of oligodeoxynucleotides (ISS-ODN) because many immunostimulatory oligonucleotides (ODNs) do not contain a CpG motif. Typically, the ODN is a synthetic thiophosphorylate-linked compound. However, many types of DNA and RNA can activate TLR9 including bacterial DNA, liposomal vertebrate DNA, insect DNA, chlamydia polynucleotides and others.

Another class of TLR9 agonists are nucleotide sequences containing a synthetic cytosine-phosphate-2'-deoxy-7-deazaguanosine dinucleotide (CpR), called immunomodulatory oligonucleotides (IMOs) (Hybridon, Inc.). A dumbbell-like covalently-closed structure is also art recognized (dSLIM-30L1) that is an agonist for TLR9. PolyG oligodeoxynucleotides can also be immunostimulatory. Even double-stranded DNA, such as that released from dying cells, can increase an immune response. Plasmid DNA may be especially immunostimulatory. While this may be due to CpG motifs, it is not clear if this is always due to its agonistic activity for TLR9. Nevertheless, this property of plasmid DNA can add to the effectiveness of an immunostimulatory combination when the plasmid encodes a TNFRSF agonist, a TLR agonist (like Hsp60), or a cytokine/chemokine receptor agonist (like interferon-gamma).

The ligand for TLR10 is currently not known.

One agonist for TLR11 is the profilin-like molecule from the protozoan parasite Toxoplasma gondii (PFTG).

NLR agonists include microbial products and synthetic derivatives of them. The best characterized agonists are those for NOD1, NOD2, NALP-3, RIG-I, and MDA5.

NOD1, also called CARD-4, is activated by compounds containing GlcNac-MurNAc-L-Ala-γ-D-Glu-meso-diaminopimelic acid, also called GM-TriDAP, which is a breakdown product of the peptidoglycan in bacterial cell walls. Another NOD 1 activator is MurNAc-L-Ala-γ-D-Glu-meso-diaminopimelic acid, also called M-TriDAP. The minimal motif for NOD 1 activation is γ-D-Glu-meso-DAP, also called iE-DAP, with an exposed DAP stem. Other NOD1 activators are FK156 (D-lactoyl-L-alanyl-gamma-D-glutamyl-(L)-meso-diaminopimelyl-(L)-glycine) and FK565 (heptanoyl-gamma-D-glutamyl-(L)-mesodiaminopimelyl-(D)-alanine). FK565 protected mice from herpes simplex virus when given at intravenous and subcutaneous doses of 0.01, 0.1, and 1 mg/kg or orally at a dose of 1 mg/kg. FK565 also protected mice from cytomegalovirus infection when given at a subcutaneous dose of 15 mg/kg, and was protective against influenza virus using intravenous, subcutaneous, and oral doses of 0.001 to 1 mg/kg.

NOD2, also called CARD-15, is activated by muramyl dipeptide (MDP), MurNAc-L-Ala-D-isoGln, also called GM-Di. Another NOD2 activator is MurNAc-L-Ala-y-D-Glu-L-Lys, also called MtriLYS). MDP has been formulated in liposomes composed of phosphatidylserine and phosphatidylcholine (3:7) ratio and containing 2.5 ug of MDP. When given intravenously, this form of MDP markedly reduces metastases in mice bearing B16-BL6 melanoma tumors. Clinical trials in dogs and humans with osteosarcoma have used a liposomal form of muramyl tripeptide (MTP) phosphatidylethanolamine (MTP-PE) given intravenously at 1-2 mg/mm². Similar studies of MTP-PE liposomes showed mildly positive effects against melanoma in dogs.

NALP-3, also called PYPAF1, has several splice variants, one of which is called cryopyrin. MDP interacts with Nalp3 to stimulate the proteolytic processing of interleukin-1 beta (IL-13) and Interleukin-18 (IL-18), and can be activated by bacterial RNA and extracellular ATP (ATPe), where the latter acts on the purogenic P2X₇ receptor. In one aspect, ATPγS and benzoyl-benzoic ATP are P2X₇ agonists. In a related aspect, ATPe includes, but is not limited to, BenzoylBenzoylATP, or any agonist of the P1, P2X, and P2Y receptor family.

Many of the NLR proteins contain a CARD domain. A recently described CARD-containing protein, variously called IPS-1, MAVS, VISA and Cardif, is an adaptor protein involved in transmitting the activating signals from retinoic acid inducible gene-I (RIG-I) and melanoma differentiation-associated gene 5 (MDA5) which also contain a helicase domain.

"RHL receptors" are defined as class of RIG-Like Helicase proteins. In a related aspect, RHL receptors include, but are not limited to, RIG-I (GenBank Accession No. AF038963) and Melanoma differentiation associated gene 5 (MDA5) (GenBank Accession No. AF095844). RIG-I recognizes 5'-triphosphate RNAs, and dsRNA, such as poly(I:C) are ligands for MDA5. Further, dsRNA containing a triphosphate, which is often produced during the replication of certain viruses, interacts with MDA5 to activate interferon regulatory factor 3 (IRF3), a transcription factor that initiates a program of antiviral defenses in cells and the production of Type I interferons.

Different NLR agonists induce different responses. For example, MDP interacts with Nalp3 to stimulate the proteolytic processing of interleukin-1 beta (IL-1β) and Interleukin-18 (IL-18). Double-stranded RNAs (dsRNA) interacts with MDA5 to activate interferon regulatory factor 3 (IRF3), a transcription factor that initiates a program of antiviral defenses in cells and the production of Type I interferons. As noted above, poly(I:C) is commonly used as a mimic of naturally occurring dsRNAs and extracellular poly(I:C) has been shown to stimulate cells by interacting with TLR3. However, cells without TLR3 on their surface or cells in which TLR signaling has been blocked can still respond to dsRNA from intracellular Sendai virus infection through a TLR3-independent intracellular pathway requiring the expression of retinoic acid-inducible gene I (RIG-I), a putative cellular RNA helicase. For dsRNA, this agonist may signal using either TLR3 or RIG-I or both, depending upon the cell type. For example, myeloid-derived dendritic cells respond to dsRNA using TLR3 whereas plasmacytoid dendritic cells respond to dsRNA using RIG-I. MDA5 is another RNA helicase that also senses intracellular dsRNA and signals similarly to RIG-I.

In summary, the NLR and RLH agonists that have been identified thus far include: γ-D-Glu-meso-DAP, the minimal activator of NOD 1; MurNAc-L-Ala-D-isoGln or muramyl dipeptide (MDP), an activator of NOD2 and NALP-3; MTP-PE liposomes, which is also likely to activate NOD2 and NALP-3; and dsRNA such as Poly(I:C), an activator of RIG-I and MDA5, bacterial DNA, and ATPe (acting through cryopyrin/NALP3).

"Purinergic receptor" is defined as a cell membrane receptor that binds to one or more purines. These receptors were originally divided into P1 receptors which have adenosine as their main ligand, and P2 receptors which have ATP and ADP as their main ligands. The P2 receptors were later divided into the ionotropic P2X and metabotropic P2Y subtypes P1 purinergic receptors (V. Ralevic and G. Burnstock, Receptors for purines and pyrimidines. Pharmacol. Rev. 50:413-492, 1998). The P1 receptors include A1, A2A, A2B and A3. An online database of P1 receptors is hosed by The Neuromuscular Disease Center, Washington University, St. Louis, MO. The P2 receptors are described in an online database hosted by The Human Genome Organization Gene Nomenclature Committee, Department of Biology, University College London, United Kingdom. P2X receptors include the homomeric P2X1, P2X2, P2X3, P2X4, P2X5, P2X7 and P2XL1 channels and the heteromeric P2X2/3 and P2X1/5 channels. P2X receptors are described in an online database hosted by The Neuromuscular Disease Center, Washington University, St. Louis, MO. P2Y receptors include P2Y 1, P2Y2, P2Y4, P2Y6, P2Y 11, P2Y 12, P2Y 13 and P2Y 14. An online listing of P2Y receptors is maintained by The International Union of Basic and Clinical Pharmacology (IUPHAR), University of Kansas Medical Center, Kansas City, KS. In one aspect, the receptor is P2X7. Stimulation of P2X7 can induce many types of tumor cells to undergo apoptotic cell death (N. White and G. Burnstock, P2 receptors and cancer, Trends Pharmacol. Sci. 27:211-217, 2006). Stimulation of P2X7 by ATP activates caspase 1 which in turn causes the proteolytic activation of pro-interleukin-1 and pro-interleukin-18 into active interleukin-1 and active interleukin-18, respectively (D. Ferrari et al, The P2X7 Receptor: A Key Player in IL-1 Processing and Release, J. Immunol. 176:3877-83, 2006).

"Purinergic receptor agonist" is defined as a substance that activates purinergic receptors. Special preference is given to agonists for P2X receptors, most preferably P2X7. Agonists for P2X7 include, but are not limited to, adenosine 5'-triphosphate (ATP), ATPgammaS (a non-hydrolyzable form of ATP), 2'- and 3'-O-(4-benzyol-benzoyl)ATP (BzATP), with ATPgammaS being preferred. When ATP is administered so that acts on a purinergic receptor at the surface of a cell, it is called 'extracellular ATP' or 'ATPe.' Another agonist for P2X7 is the human cathelicidin-derived peptide LL37/nCAP-18 (GenBank Accession No. M_004345). ATPγS has been shown to increase the immune-mediated delayed type hypersensitivity response in mouse skin. ATP has been infused intravenously into humans with non-small cell lung cancer with a maximum tolerated dose of 50 ug/kg per minute.

"Ectonucleotidases" are defined as enzymes that break down extracellular ATP. They are sometimes called "ecto-ATPases." Several enzyme families have ectonucleotidase activity including: extonucleoside triphosphate diphosphohydrolases (E-NTPDases) of which NTPDase 1, 2, 3 and 8 are extracellular; ectonucleotide pyrophosphatase (E-NPP) of three types, E-NPP-1, E-NPP-2/autotaxin, ad E-NPP-3; alkaline phosphatase; ecto-5'-nucleotidase; and ectonucleoside diphosphokinase (E-NDPK). These degradative enzymes are significant in that they can degrade ATP and ATP analogs and thereby limit the effects of these purinergic agonists. Conversely, agents that inhibit these ectonucleotidases can prolong and enhance the effectiveness of purinergic agonists.

Cytokine/chemokine receptor agonists can be used alone as immunostimulants. For example, IL-2 (10 ug) and IFN-gamma (10 ug) have been injected into lepromatous leprosy lesions in humans at a dose of 10 ug with good therapeutic effects. An effective dose of GM-CSF in dogs was 15 ug/kg s.c. daily over 9 weeks, which enhanced the ability of monocytes to control tumor cell growth in vitro. Similarly, cytokines have been included in DNA vaccines to enhance the immune response.

IL-2 is available commercially as Proleukin (Chiron Corp.) in a preparation of 22 million units/vial. A typical schedule of administration is 600,000 units given intravenously over 15 minutes every 8 hours for a maximum of 14 doses. IFN-gamma-1b is available commercially as Actimmune (InterMune, Inc.) in a preparation of 30 million units/ml. A typical schedule of administration is 1 million units/square meter, given three times/week by subcutaneous injection. GM-CSF is available commercially as Leukine (Berlex, Inc.) in a preparation of 500 ug/ml. A typical schedule of administration is 250 ug/square meter given intravenously over 4 hours.

All cytokines can also be given as the nucleic acids that encode them. Preference is given to plasmid DNAs, since their administration results in a sustained release of the protein at the vaccination or tumor immunotherapy site which creates high concentration in draining lymph nodes while minimizing whole body exposure to these potentially toxic agents.

Combinations of immunostimulatory agents have many potential uses, for example, TLR agonists can be combined with agents that block inhibitors of DC activation. Further, tumors can be injected with CpG ODN and neutralizing antibody against IL-10. Other combinations are known in the art. Preference is given to combinations of immunostimulatory agents that are synergistic, including the combinations that follow.

The combination of an imidazoquinoline TLR7 and/or TLR8 agonist with an ISS-ODN has been described, including the use of combinations of TLR agonists to induce dendritic cell maturation in vitro.

By itself, CD40L is a weak stimulator of IL-12 production by dendritic cells. CD40L combined with dsRNA activate plasmacytoid DCs to produce Interleukin-12 (IL-12) and Type I interferons, which in turn activate type 1 helper T cell responses. CD40L and CpG ODN (TLR9 agonist) synergistically activate dendritic cells to make IL-12. Agonistic anti-CD40 antibody plus Poly(I:C) have synergistic anti-tumor effects in mice. The addition of Poly(I:C) to dendritic cells cultured with IL-1-beta, TNF, and IFN-gamma are especially stimulatory for the production of IL-12 and the generation of type 1 helper T cell responses has been shown.

A NOD1 activator, GM-TriDAP was found to synergize with a TLR1/2 agonist (Pam3CysLys4) TLR2/6 agonist (MALP2), a TLR4 agonist (LPS), a TLR7/8 agonist (resiquimod) for cytokine production by human peripheral blood mononuclear cells. In human monocytes and dendritic cells, a NOD1 activator (M-triDAP) was found to synergize with a TLR4 agonist (purified LPS) for cytokine production.

A NOD2 activator, MDP, was found to synergize with a TLR2 agonist (either Pam3Cys or MALP2), TLR3 agonist (Poly(I:C)), or TLR4 agonist (LPS) for cytokine production in mouse macrophages. Another study found that MDP activation of NOD2 synergized with the TLR9 agonist, ISS-ODN, for cytokine production by human peripheral blood mononuclear cells. In the human THP-1 cell line, MDP synergized with a TLR1/2 agonist (Pam3CysLys4), a TLR4 agonist (Lipid A), or a TLR9 agonist (ISS-ODN) for IL-8 production. In human monocytes and dendritic cells, NOD2 activators (either MDP or MtriLYS) were found to synergize with a TLR4 agonist (purified LPS) for cytokine production. In mice, MDP synergizes with agonists for TLR2, TLR4, or TLR9.

For human monocyte-derived dendritic cells in culture, stimulation with MDP (NOD2 agonist) and FK565 (NOD1 agonist) in combination with lipid A, poly(I:C), and CpG DNA, but not with Pam3CSSNA, synergistically induced interleukin-12 (IL-12) p70 and gamma interferon (IFN-gamma), but not IL-18, in culture supernatants and induced IL-15 on the cell surface.

The ability of mononuclear blood cells from dogs to control the growth of tumor cells in vitro was synergistically increased by the combination of lipopolysaccharide (LPS) and MDP.

IFN-gamma, and also IL-3 and GM-CSF, upregulates the CD40 receptor on human monocytes and dramatically enhances the ability of CD40L to induce IL-6, IL-8, TNF on these cells. Importantly, IFN-gamma greatly augments the ability of CD40L to induce the production of IL-12 in dendritic cells. However, additional cytokines such as IL-4, IL-13, and GM-CSF can also synergize with CD40L for the production of IL-12 by dendritic cells. IL-4 and TNF can also synergize for the treatment of tumors.

IFN-gamma primes mouse macrophages to produce IL-12 after LPS stimulation. Plasmid DNA encoding IFN-beta combined with Poly(I:C) in a cationic lipid formulation reduced metastases in a colon cancer model in mice. The combination of ISS-ODN with cytokines such as IL-2, IL-12 and IFN-ganuna, IFN-alpha, IFN-beta or combinations thereof, has been demonstrated.

In vitro, the combination of IL-2 and FK565 led to synergistic activation of lymphokine-activated killer (LAK) cells that were capable of killing tumor cells. IFN-gamma and muramyl dipeptide (MDP) liposomes synergistically induced the tumor cell-killing activity of human monocytes in vitro. GM-CSF and muramyl tripeptide liposomes (L-MTP-PE) were synergistic in inducing anti-tumor killing by alveolar macrophages in dogs, where the L-MTP-PE was administered intravenously at 1-2 mg/m².

The selection of antigen for enhanced DC delivery and modulation of the immune response thereto may be any antigen for which either an enhanced immune response is desirable, or for which tolerance of the immune system to the antigen is desired. In the case of a desired enhanced immune response to particular antigens of interest, such antigens include, but are not limited to, infectious disease antigens for which a protective immune response may be elicited are exemplary. For example, the antigens from HIV under consideration are the proteins gag, env, pol, tat, rev, nef, reverse transcriptase, and other HIV components. The E6 and E7 proteins from human papilloma virus are also under consideration. Furthermore, the EBNA1 antigen from herpes simplex virus is also under consideration. Other viral antigens for consideration are hepatitis viral antigens such as the S, M, and L proteins of hepatitis B virus, the pre-S antigen of hepatitis B virus, and other hepatitis, e.g., hepatitis A, B, and C, viral components such as hepatitis C viral RNA; influenza viral antigens such as hemagglutinin, neuraminidase, nucleoprotein, M2, and other influenza viral components; measles viral antigens such as the measles virus fusion protein and other measles virus components; rubella viral antigens such as proteins E1 and E2 and other rubella virus components; rotaviral antigens such as VP7sc and other rotaviral components; cytomegaloviral antigens such as envelope glycoprotein B and other cytomegaloviral antigen components; respiratory syncytial viral antigens such as the RSV fusion protein, the M2 protein and other respiratory syncytial viral antigen components; herpes simplex viral antigens such as immediate early proteins, glycoprotein D, and other herpes simplex viral antigen components; varicella zoster viral antigens such as gpI, gpII, and other varicella zoster viral antigen components; Japanese encephalitis viral antigens such as proteins E, M-E, M-E-NS 1, NS 1, NS 1-NS2A, 80% E, and other Japanese encephalitis viral antigen components; rabies viral antigens such as rabies glycoprotein, rabies nucleoprotein and other rabies viral antigen components; West Nile virus prM and E proteins; and Ebola envelope protein. See Fundamental Virology, Second Edition, eds. Knipe, D.M. and, Howley P.M. (Lippincott Williams & Wilkins, New York, 2001) for additional examples of viral antigens. In addition, bacterial antigens are also disclosed. Bacterial antigens which can be used in the compositions and methods of the invention include, but are not limited to, pertussis bacterial antigens such as pertussis toxin, filamentous hemagglutinin, pertactin, FIM2, FIM3, adenylate cyclase and other pertussis bacterial antigen components; diptheria bacterial antigens such as diptheria toxin or toxoid and other diptheria bacterial antigen components; tetanus bacterial antigens such as tetanus toxin or toxoid and other tetanus bacterial antigen components; streptococcal bacterial antigens such as M proteins and other streptococcal bacterial antigen components; Staphylococcal bacterial antigens such as IsdA, IsdB, SdrD, and SdrE; gram-negative bacilli bacterial antigens such as lipopolysaccharides, flagellin, and other gram-negative bacterial antigen components; Mycobacterium tuberculosis bacterial antigens such as mycolic acid, heat shock protein 65 (HSP65), the 30 kDa major secreted protein, antigen 85A, ESAT-6, and other mycobacterial antigen components; Helicobacter pylori bacterial antigen components; pneumococcal bacterial antigens such as pneumolysin, pneumococcal capsular polysaccharides and other pneumococcal bacterial antigen components; haemophilus influenza bacterial antigens such as capsular polysaccharides and other haemophilus influenza bacterial antigen components; anthrax bacterial antigens such as anthrax protective antigen, anthrax lethal factor, and other anthrax bacterial antigen components; the F1 and V proteins from Yersinia pestis; rickettsiae bacterial antigens such as romps and other rickettsiae bacterial antigen components. Also included with the bacterial antigens described herein are any other bacterial, mycobacterial, mycoplasmal, rickettsial, or chlamydial antigens. Examples of protozoa and other parasitic antigens include, but are not limited to, plasmodium falciparum antigens such as merozoite surface antigens, sporozoite surface antigens, circumsporozoite antigens, gametocyte/gamete surface antigens, blood-stage antigen pf 1 55/RESA and other plasmodial antigen components; toxoplasma antigens such as SAG-1, p30 and other toxoplasma antigen components; schistosomae antigens such as glutathione-S-transferase, paramyosin, and other schistosomal antigen components; leishmania major and other leishmaniae antigens such as gp63, lipophosphoglycan and its associated protein and other leishmanial antigen components; and trypanosoma cruzi antigens such as the 75-77 kDa antigen, the 56 kDa antigen and other trypanosomal antigen components. Examples of fungal antigens include, but are not limited to, antigens from Candida species, Aspergillus species, Blastomyces species, Histoplasma species, Coccidiodomycosis species, Malassezia furfur and other species, Exophiala werneckii and other species, Piedraia hortai and other species, Trichosporum beigelii and other species, Microsporum species, Trichophyton species, Epidermophyton species, Sporothrix schenckii and other species, Fonsecaea pedrosoi and other species, Wangiella dermatitidis and other species, Pseudallescheria boydii and other species, Madurella grisea and other species, Rhizopus species, Absidia species, and Mucor species. Examples of prion disease antigens include PrP, beta-amyloid, and other prion-associated proteins.

In addition to the infectious and parasitic agents mentioned above, another area for desirable enhanced immunogenicity to a non-infectious agent is in the area of dysproliferative diseases, including but not limited to cancer, in which cells expressing cancer antigens are desirably eliminated from the body. Tumor antigens which can be used in the compositions and methods of the invention include, but are not limited to, prostate specific antigen (PSA), breast, ovarian, testicular, melanoma, telomerase; multidrug resistance proteins such as P-glycoprotein; MAGE-1, alpha fetoprotein, carcinoembryonic antigen, mutant p53, papillomavirus antigens, gangliosides or other carbohydrate-containing components of melanoma or other tumor cells. It is contemplated by the invention that antigens from any type of tumor cell can be used in the compositions and methods described herein. The antigen may be a cancer cell, or immunogenic materials isolated from a cancer cell, such as membrane proteins. Included are survivin and telomerase universal antigens and the MAGE family of cancer testis antigens. Antigens which have been shown to be involved in autoimmunity and could be used in the methods of the present invention to induce tolerance include, but are not limited to, myelin basic protein, myelin oligodendrocyte glycoprotein and proteolipid protein of multiple sclerosis and CII collagen protein of rheumatoid arthritis.

The antigen may be a portion of an infectious agent such as HZV-1, EBV, HBV, influenza virus, SARS virus, poxviruses, malaria, or HSV, by way of non-limiting examples, for which vaccines that mobilize strong T-cell mediated immunity (via dendritic cells) are needed.

The term "tumor" denotes at least one cell or cell mass in the form of a tissue neoformation, in particular in the form of a spontaneous, autonomous and irreversible excess growth, which is more or less disinhibited, of endogenous tissue, which growth is as a rule associated with the more or less pronounced loss of specific cell and tissue functions. This cell or cell mass is not effectively inhibited, in regard to its growth, by itself or by the regulatory mechanisms of the host organism, e.g. melanoma or carcinoma. Tumor antigens not only include antigens present in or on the malignant cells themselves, but also include antigens present on the stromal supporting tissue of tumors including endothelial cells and other blood vessel components.

In a related aspect, neoplastic refers to abnormal new growth and thus means the same as tumor, which may be benign or malignant. Further, such neoplasia would include cell proliferation disorders.

As used herein, the term "polypeptide" "is used in its conventional meaning, i.e., as a sequence of amino acids. The polypeptides are not limited to a specific length of the product; thus, peptides, oligopeptides, and proteins are included within the definition of polypeptide, and such terms may be used interchangeably herein unless specifically indicated otherwise. This term also does not refer to or exclude post-expression modifications of the polypeptide, for example, glycosylations, acetylations, phosphorylations and the like, as well as other modifications known in the art, both naturally occurring and non-naturally occurring. A polypeptide may be an entire protein, or a subsequence thereof. Particular polypeptides of interest in the context of this invention are amino acid subsequences comprising epitopes, i.e., antigenic determinants substantially responsible for the immunogenic properties of a polypeptide and being capable of evoking an immune response.

In one embodiment, polypeptides of interest include, but are not limited to, CD40L (GenBank Acc. Nos. P63305, P63304, Q918D8, Q9BDN3, Q9BDM7); glucocorticoid-induced TNFR related gene ligand (GIRTL) (GenBank Acc. No. Q9Y5U5); NGF (GenBank Acc. Nos. AAA40599, AAA30666, AAA72805, CAA37703, AAB58676); CD137L14-1BBL, (GenBank Acc. Nos. Nip_033430, P41274); TNF-alpha (GenBank Ace. Nos. NP_001003244, NP_038721, AAB06492, AAB01775); CD143L/OX40L (GenBank Acc. Nos. P23510, AAX43997, NP_003317); CD27L/CD70 (GenBank Acc. Nos. P32970,055237); FasL (GenBank Acc. Nos. P63308, P63307, P63308); CD30L (GenBank Acc. Nos. NP_001235, AAH93630, P32971); TNF-beta/LT-alpha (GenBank Acc. Nos. AAA18593, NP_034865); LT-beta (GenBank Acc. Nos. Q5TM22, Q9TSV8); TRAIL (GenBank Acc. Nos. AAC52345, AAC50332); BAFF (GenBank Acc. No. Q9Y275); LIGHT (GenBank Acc. Nos. AAQ89171, AAC39563) RANKL (GenBank Acc. Nos. AAB86811, NP_003692); Acrp30 (GenBank Acc. Nos. AAZ81421, AAH92565, AAK13417, AA80543); and surfactant protein-D (SP-D) (Genbank Acc. Nos. NP_033186, AAB25038, AAB25037, AAH03705).

In one embodiment, polypeptides are defined by structural domains. For example, the signaling domain, which is associated with transduction upon receptor binding and is found in the cytoplasmic compartment of cells, is defined as a region of a protein molecule delimited on the basis of function and is related to a receptor's cytoplasmic substrate. Such signaling domains include, for example, the cytoplasmic domain of TNFSFRs CD40.

In another aspect, the present invention provides variants of the polypeptide compositions described herein. Polypeptide variants generally encompassed by the present invention will typically exhibit at least about 70%, 75%, 80%, 85%, 90%, 91%, 92%, 93%, 94%, 95%, 96%, 97%, 98%, or 99% or more identity (determined as described below), along its length, to a polypeptide sequences set forth herein.

Within other illustrative embodiments, a polypeptide may be a fusion polypeptide that comprises multiple polypeptides as described herein, or that comprises at least one polypeptide as described herein and an unrelated sequence, such as a known bacterial protein. A fusion partner may, for example, assist in providing T helper epitopes (an immunological fusion partner), preferably T helper epitopes recognized by humans, or may assist in expressing the protein (an expression enhancer) at higher yields than the native recombinant protein. Certain fusion partners enhance formation of multimers. In a related aspect, polypeptides which "multimerize" and can serve as fusion partners for the aggregation of proteins of interest include collectins (collagenous lectins) and ficolins (see, e.g., Ohashi and Erikson, J Biol Chem (2004) 279(8):6534-6539), Clq family proteins such as Acrp30.

In one embodiment, CD40L has been expressed as a soluble, multimeric molecule. In a related aspect, a DNA vaccine approach is disclosed which DNA encodes 2- and 4-trimer multimers of CD40L. For example, such fusion proteins may be produced by combining the extracellular domain (ECD) of a TNFSF ligand with a multimerizing domain, typically taken from Clq and collectin family members, where the carbohydrate recognition domain (CRD) of the Clq/collectin family member has been replaced by the ECD of the TNFSF ligand. In another embodiment, a fusion protein is envisaged including an antigen of interest, a signaling domain of a tumor necrosis factor superfamily receptor (TNFSFR) or a Toll-like receptor (TLR), and a clustering peptide. In a related aspect, fusion proteins as disclosed in the present invention can include translational enhancer elements (TEEs). As used herein, "translational enhancer element (TEE)," including grammatical variations thereof, means cis-acting sequences that increase the amount of protein induced per unit mRNA. In a related aspect, TEEs include, HCV-IRES, IRESes, and IRES-elements, including, but not limited to, Gtx sequences (e.g., Gtx9-nt, Gtx8-nt, Gtx7-nt). In another related aspect, TEEs may include N-18 random nucleotides which when operably linked to a cistron, increase the amount of protein induced per unit mRNA.

In a another related aspect, sequences for such elements include, but are not limited to, GenBank accession numbers AX205123 and AX205116 (Gtx IRES element), D 17763 (HCV-IRES, 5'-untranslated region).

Several other TNFSF ligands are also candidate molecular adjuvants/fusion partners. In one embodiment, ligands for GITR (Glucoconicoid-Induced TNF receptors-Related) are envisaged because GITR is expressed on CD4 + CD25 + regulatory T cells (Tregs). GITR stimulation of Tregs turns off their immunosuppressive effects and augments immune responses. In a related aspect, a 4-trimer soluble multimeric form of GITRL is also a potent molecular adjuvant for CD8+ T cells.

Fusion polypeptides may generally be prepared using standard techniques, including chemical conjugation. Preferably, a fusion polypeptide is expressed as a recombinant polypeptide, allowing the production of increased levels, relative to a non-fused polypeptide, in an expression system. Briefly, DNA sequences encoding the polypeptide components may be assembled separately, and ligated into an appropriate expression vector. The 3' end of the DNA sequence encoding one polypeptide component is ligated, with or without a peptide linker, to the 5' end of a DNA sequence encoding the second polypeptide component so that the reading frames of the sequences are in phase. This permits translation into a single fusion polypeptide that retains the biological activity of both component polypeptides.

A peptide linker sequence may be employed to separate the first and second polypeptide components by a distance sufficient to ensure that each polypeptide folds into its secondary and tertiary structures. Such a peptide linker sequence is incorporated into the fusion polypeptide using standard techniques well known in the art. Suitable peptide linker sequences may be chosen based on the following factors: (1) their ability to adopt a flexible extended conformation; (2) their inability to adopt a secondary structure that could interact with functional epitopes on the first and second polypeptides; and (3) the lack of hydrophobic or charged residues that might react with the polypeptide functional epitopes. Preferred peptide linker sequences contain Gly, Asn, and Ser residues. Other near neutral amino acids, such as Thr and Ala may also be used in the linker sequence. Amino acid sequences which may be usefully employed as linkers include those disclosed in Maratea et al., Gene 40:39-46, 1985; Murphy et al., Proc. Natl. Acad. Sci. USA 83:8258-8262, 1986; U.S. Pat. No. 4,935,233 and U.S. Pat. No. 4,751,7 80. The linker sequence may generally be from 1 to about 50 amino acids in length. Linker sequences are not required when the first and second polypeptides have non-essential N-terminal amino acid regions that can be used to separate the functional domains and prevent steric interference.

The ligated DNA sequences are operably linked to suitable transcriptional or translational regulatory elements. The regulatory elements responsible for expression of DNA are located only 5' to the DNA sequence encoding the first polypeptides. Similarly, stop codons required to end translation and transcription termination signals are only present 3' to the DNA sequence encoding the secondary, tertiary, or quaternary, etc., polypeptide (i.e., a stop codon will be present on the ultimate polypeptide depending on the number of distinct polypeptides making up a chimeric protein molecule).

The present invention, in other aspects, provides polynucleotide compositions. The terms "DNA" and "polynucleotide" are used essentially interchangeably herein to refer to a DNA molecule that has been isolated free of total genomic DNA of a particular species. "Isolated," as used herein, means that a polynucleotide is substantially away from other coding sequences, and that the DNA molecule does not contain large portions of unrelated coding DNA, such as large chromosomal fragments or other functional genes or polypeptide coding regions. Of course, this refers to the DNA molecule as originally isolated, and does not exclude genes or coding regions later added to the segment by the hand of man.

As will be understood by those skilled in the art, the polynucleotide compositions of this invention can include genomic sequences, extra-genomic and plasmid-encoded sequences and smaller engineered gene segments that express, or may be adapted to express, proteins, polypeptides, peptides and the like. Such segments may be naturally isolated, or modified synthetically by the hand of man.

As will be also recognized by the skilled artisan, polynucleotides of the invention may be single-stranded or double-stranded, and may be DNA (genomic, cDNA or synthetic) or RNA molecules. RNA molecules may include HnRNA molecules, which contain introns and correspond to a DNA molecule in a one-to-one manner, and mRNA molecules, which do not contain introns. Additional coding or noncoding sequences may, but need not, be present within a polynucleotide of the present invention, and a polynucleotide may, but need not, be linked to other molecules and/or support materials.

Polynucleotides may comprise a native sequence (i.e., an endogenous sequence that encodes a polypeptide/protein of the invention or a portion thereof) or may comprise a sequence that encodes a variant or derivative, including an immunogenic variant or derivative, of such a sequence.

Optimal alignment of polypeptide or nucleic acid sequences for comparison may be conducted using the Megalign program in the Lasergene suite of bioinformatics software (DNASTAR, Inc., Madison, Wis.), using default parameters. This program embodies several alignment schemes described in the following references: Dayhoff, M. O. (1978) A model of evolutionary change in proteins--Matrices for detecting distant relationships. In Dayhoff, M. O. (ed.) Atlas of Protein Sequence and Structure, National Biomedical Research Foundation, Washington D.C. Vol. 5, Suppl. 3, pp. 345-358; Hein J. (1990) Unified Approach to Alignment and Phylogenes pp. 626-645 Methods in Enzymology vol. 183, Academic Press, Inc., San Diego, Calif.; Higgins, D. G. and Sharp, P. M. (1989) CABIOS 5:151-153; Myers, E. W. and Muller W. (1988) CABIOS 4:11-17; Robinson, E. D. (1971) Comb. Theor 11:105; Saitou, N. Nei, M. (1987) Mol. Biol. Evol. 4:406-425; Sneath, P. H. A. and Sokal, R. R. (1973) Numerical Taxonomy--the Principles and Practice of Numerical Taxonomy, Freeman Press, San Francisco, Calif.; Wilbur, W. J. and Lipman, D. J. (1983) Proc. Natl. Acad., Sci. USA 80:726-730.

Alternatively, optimal alignment of sequences for comparison may be conducted by the local identity algorithm of Smith and Waterman (1981) Add. APL. Math 2:482, by the identity alignment algorithm of Needleman and Wunsch (1970) J. Mol. Biol. 48:443, by the search for similarity methods of Pearson and Lipman (1988) Proc. Natl. Acad. Sci. USA 85: 2444, by computerized implementations of these algorithms (GAP, BESTFIT, BLAST, FASTA, and TFASTA in the Wisconsin Genetics Software Package, Genetics Computer Group (GCG), 575 Science Dr., Madison, Wis.), or by inspection.

One example of algorithms that are suitable for determining percent sequence identity and sequence similarity are the BLAST and BLAST 2.0 algorithms, which are described in Altschul et al. (1977) Nucl. Acids Res. 25:3389-3402 and Altschul et al. (1990) J. Mol. Biol. 215:403-410, respectively. BLAST and BLAST 2.0 can be used, for example with the parameters described herein, to determine percent sequence identity for the polynucleotides and polypeptides of the invention. Software for performing BLAST analyses is publicly available through the National Center for Biotechnology Information. For amino acid sequences, a scoring matrix can be used to calculate the cumulative score. Extension of the word hits in each direction are halted when: the cumulative alignment score falls off by the quantity X from its maximum achieved value; the cumulative score goes to zero or below, due to the accumulation of one or more negative-scoring residue alignments; or the end of either sequence is reached. The BLAST algorithm parameters W, T and X determine the sensitivity and speed of the alignment.

The invention also relates to the use of the cationic polymers of, for example, formula (I) which can be used in combination with the disclosed nucleic acid compositions.

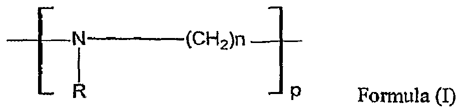

In one embodiment, in the formula (I), R is a hydrogen atom or a group of formula

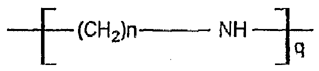

and the R group is attached to the (CH₂) end to the N atom in the main formula, n is an integer between 2 and 10, and p and q are integers, in which the sum ofp + q is such that the average molecular weight of the polymer is between 100 and 10⁷. By way of non-limiting examples, useful R groups for such polymers include those given by D.G. Anderson et al., A polymer library approach to suicide gene therapy for cancer, Proc Natl Acad Sci USA, 101:16028-16033,2004. In one aspect, amino alcohols from libraries of poly(beta-amino esters) comprise the R group. In one aspect, 2-(pyridyldithio)-ethylamine (PDA) comprises the R group. In another aspect, poly(beta-amino ester)s with thiol-reactive side chains comprise the R group. Such constituent R groups are known in the art.

In one aspect, polyethylenimine (PEI) and polypropylenimine (PEI) polymers have advantageous properties.

In a related aspect, polymers for carrying out the present invention are those whose molecular weight is between 10³ and 5 x 10⁶. As an example, this would include a polyethylenimine of average molecular weight 50,000 Da (PEI50K) or a polyethylenimine of average molecular weight 800,000 Da (PEI800K).

The polymers used in the context of the present invention may be obtained in different ways. They may, in the first place, be synthesized chemically from the corresponding monomer under anionic polymerization conditions (for example polymerization of ethylenimine), or by reduction of polyamides obtained by polycondensation of diacids with diamines, or alternatively by reduction of imines obtained by polycondensation of dialdehydes with diamines. Moreover, a number of these polymers are commercially available, such as, in particular, PEI50K or PEI800K.

In the compositions of the present invention, the nucleic acid can be either a deoxyribonucleic acid or a ribonucleic acid. The sequences in question can be of natural or artificial origin, and in particular genomic DNA, cDNA, mRNA, tRNA, rRNA, hybrid sequences or synthetic or semi-synthetic sequences. In addition, the nucleic acid can be very variable in size, ranging from oligonucleotide to chromosome. These nucleic acids may be of human, animal, vegetable, bacterial, viral, and the like, origin. They may be obtained by any technique known to a person skilled in the art, and in particular by the screening of libraries, by chemical synthesis or alternatively by mixed methods including the chemical or enzymatic modification of sequences obtained by the screening of libraries. They can, moreover, be incorporated into vectors, such as plasmid vectors. In order to express a desired polypeptide, the nucleotide sequences encoding the polypeptide, or functional equivalents, may be inserted into appropriate expression vector, i.e., a vector which contains the necessary elements for the transcription and translation of the inserted coding sequence. Methods which are well known to those skilled in the art may be used to construct expression vectors containing sequences encoding a polypeptide of interest and appropriate transcriptional and translational control elements. These methods include in vitro recombinant DNA techniques, synthetic techniques, and in vivo genetic recombination. Such techniques are described, for example, in Sambrook, J. et al. (1989) Molecular Cloning, A Laboratory Manual, Cold Spring Harbor Press, Plainview, N.Y., and Ausubel, F. M. et al. (1989) Current Protocols in Molecular Biology, John Wiley & Sons, New York. N.Y.

A variety of expression vector/host systems may be utilized to contain and express polynucleotide sequences. These include, but are not limited to, microorganisms such as bacteria transformed with recombinant bacteriophage, plasmid, or cosmid DNA expression vectors; yeast transformed with yeast expression vectors; insect cell systems infected with virus expression vectors (e.g., baculovirus); plant cell systems transformed with virus expression vectors (e.g., cauliflower mosaic virus, CaMV; tobacco mosaic virus, TMV) or with bacterial expression vectors (e.g., Ti or pBR322 plasmids); or animal cell systems.

The "control elements" or "regulatory sequences" present in an expression vector are those non-translated regions of the vector--enhancers, promoters, 5' and 3' untranslated regions--which interact with host cellular proteins to carry out transcription and translation. Such elements may vary in their strength and specificity. Depending on the vector system and host utilized, any number of suitable transcription and translation elements, including constitutive and inducible promoters, may be used. For example, when cloning in bacterial systems, inducible promoters such as the hybrid lacZ promoter of the pBLUESGRIPT phagemid (Stratagene, La Jolla, Calif.) or pSPORT1 plasmid (Gibco BRL, Gaithersburg, Md.) and the like may be used. In mammalian cell systems, promoters from mammalian genes or from mammalian viruses are generally preferred. By way of non-limiting examples, promoters include those from CMV, beta-actin, EF2alpha, RSV LTR, HIV LTR, HTLV-1 LTR, and composite promoters (D.H. Barouch et al, A human T-cell leukemia virus type 1 regulatory element enhances the immunogenicity of human immunodeficiency virus type 1 DNA vaccines in mice and nonhuman primates, J. Virol. 79: 8828-8834, 2005). In one aspect, the promoter is a CMV promoter or a promoter comprising portions of the chicken beta-actin promoter (H. Niwa et al, Efficient selection for high-expression transfectants with a novel eukaryotic vector, Gene 108:193-199, 1991). If it is necessary to generate a cell line that contains multiple copies of the sequence encoding a polypeptide, vectors based on SV40 or EBV may be advantageously used with an appropriate selectable marker.

In bacterial systems, any of a number of expression vectors may be selected depending upon the use intended for the expressed polypeptide. For example, when large quantities are needed, for example for the induction of antibodies, vectors which direct high level expression of fusion proteins that are readily purified may be used. Such vectors include, but are not limited to, the multifunctional E. coli cloning and expression vectors such as pBLUESCRIPT (Stratagene), in which the sequence encoding the polypeptide of interest may be ligated into the vector in frame with sequences for the amino-terminal Met and the subsequent 7 residues of beta-galactosidase so that a hybrid protein is produced; pIN vectors (Van Heeke, G. and S. M. Schuster (1989) J. Biol. Chem. 264:5503-5509); and the like. pGEX Vectors (Promega, Madison, WI) may also be used to express foreign polypeptides as fusion proteins with glutathione S-transferase (GST). In general, such fusion proteins are soluble and can easily be purified from lysed cells by adsorption to glutathione-agarose beads followed by elution in the presence of free glutathione. Proteins made in such systems may be designed to include heparin, thrombin, or factor XA protease cleavage sites so that the cloned polypeptide of interest can be released from the GST moiety at will.

In the yeast, Saccharomyces cerevisiae, a number of vectors containing constitutive or inducible promoters such as alpha factor, alcohol oxidase, and PGH may be used. For reviews, see Ausubel et al. (supra) and Grant et al. (1987) Methods Enzymol. 153:516-544.

An insect system may also be used to express a polypeptide of interest. For example, in one such system, Autographa califomica nuclear polyhedrosis virus (AcNPV) is used as a vector to express foreign genes in Spodoptera frugiperda cells or in Trichoplusia larvae. The sequences encoding the polypeptide may be cloned into a non-essential region of the virus, such as the polyhedrin gene, and placed under control of the polyhedrin promoter. Successful insertion of the polypeptide-encoding sequence will render the polyhedrin gene inactive and produce recombinant virus lacking coat protein. The recombinant viruses may then be used to infect, for example, S. frugiperda cells or Trichoplusia larvae in which the polypeptide of interest may be expressed (Engelhard, E. K. et al. (1994) Proc. Natl. Acad. Sci. 91:3224-3227).

In mammalian host cells, a number of viral-based expression systems are generally available. For example, in cases where an adenovirus is used as an expression vector, sequences encoding a polypeptide of interest may be ligated into an adenovirus transcription/translation complex consisting of the late promoter and tripartite leader sequence. Insertion in a non-essential E1 or E3 region of the viral genome may be used to obtain a viable virus which is capable of expressing the polypeptide in infected host cells (Logan, J. and Shenk, T. (1984) Proc. Natl. Acad. Sci. 81:3655-3659). In addition, transcription enhancers, such as the Rous sarcoma virus (RSV) enhancer, may be used to increase expression in mammalian host cells.

Specific initiation signals may also be used to achieve more efficient translation of sequences encoding a polypeptide of interest. Such signals include the ATG initiation codon and adjacent sequences. In cases where sequences encoding the polypeptide, its initiation codon, and upstream sequences are inserted into the appropriate expression vector, no additional transcriptional or translational control signals may be needed. However, in cases where only coding sequence, or a portion thereof, is inserted, exogenous translational control signals including the ATG initiation codon should be provided. Furthermore, the initiation codon should be in the correct reading frame to ensure translation of the entire insert. Exogenous translational elements and initiation codons may be of various origins, both natural and synthetic. The efficiency of expression may be enhanced by the inclusion of enhancers which are appropriate for the particular cell system which is used, such as those described in the literature (Scharf, D. et al. (1994) Results Probl. Cell Differ. 20:125-162).

In addition, a host cell strain may be chosen for its ability to modulate the expression of the inserted sequences or to process the expressed protein in the desired fashion. Such modifications of the polypeptide include, but are not limited to, acetylation, carboxylation, glycosylation, phosphorylation, lipidation, and acylation. Post-translational processing which cleaves a "prepro" form of the protein may also be used to facilitate correct insertion, folding and/or function. Different host cells such as CHO, COS, HeLa, MDCK, HEK293, and WI38, which have specific cellular machinery and characteristic mechanisms for such post-translational activities, may be chosen to ensure the correct modification and processing of the foreign protein.

The general class of gene or nucleic acid delivery that does not rely on microbial delivery, usually with viruses, has been called 'non-viral gene delivery'. An alternative designation is 'synthetic vectors' or 'artificial viruses' for gene delivery. These typically involve polymers which form complexes, nanoparticles (defined as less than 1 micron in diameter), or even microparticles (defined as 1 micron in diameter or greater) with DNA plasmids and other nucleic acids. Many kinds of polymers have been described that enhance the expression of genes encoded by nucleic acids in cells

In one aspect, polyethylenimine (PEI) can be used as a delivery agent. Polyethylenimine (PEI) is one of the most well established polymers for DNA delivery. PEI is positively charged which allows it to complex with negatively charged DNA. In its mannosylated form, it directs plasmid DNA into resting macrophages and dendritic cells which endocytose it using their mannose receptors (sold as Man jetPEI by QBioGene, Inc.). Due to its amine groups, PEI effectively buffers the normally acidic pH in endosomal vesicles, thereby serving as a "proton sponge" that prevents acid damage to the DNA cargo. Many variations on PEI have been described.

In another aspect, cationic lipids can be used as delivery agents for nucleic acids. Cationic lipids and related compounds have been used to enhance the effectiveness of vaccines and the expression of genes encoded by nucleic acids in cells. DNA or RNA can also be encapsulated into microspheres comprised of an aminoalkyl glucosaminide 4-phosphate (AGP). In some cases, lipid-DNA complexes ("lipoplexes") have direct inflammatory activity that is immunostimulatory and augments the antitumor effect of the plasmid DNA.

Cationic polymers such as poly-L-lysine, poly-L-glutamate, or block co-polymers may also be delivery agents for nucleic acids. In one instance, poly-L-arginine was found to synergize with oligodeoxynucleotides containing CpG-motifs (CpG-ODN) for enhanced and prolonged immune responses and prevented the CpG-ODN-induced systemic release of pro-inflammatory cytokines. Pharmaceutical compositions comprising an antigen, an immunogenic oligodeoxynucleotide containing CpG motifs (CpG-ODN), and a polycationic polymer are known in the art.

In a related aspect, CpG-ODN refers to a single-stranded oligodeoxynucleotide produced using phosphorothioate linkages and containing an unmethylated cytosine-guanosine motif.

In one aspect, dendrimeric polymer delivery agents include Starburst polymers (Dow Chemical). In another aspect, poloxamine delivery agents may be used, including both poloxamer and polxamine compositions.

Poly-lactide-co-glycolide (PLGA) is used to make surgical sutures. It can also be formulated to deliver vaccine components. For example, a plasmid DNA encoding an HIV protein was formulated with PLGA with cetyl trimethyl ammonium bromide (CTAB), and the resulting PLG-CTAB-DNA microparticles were found to elicit an improved immune response. PLGA can also be combined with polyethylenimine (PEI) to make microspheres for DNA delivery. Microparticles formed from PLGA and other materials that incorporate DNA and TLR agonists have been developed by Chiron. The polymer component was selected (1) from the group consisting of a poly(a-hydroxy acid), a polyhydroxy butyric acid, a polycaprolactone, a polyorthoester, a polyanhydride and a polycyanoacrylate and (2) a detergent, and used to deliver a polynucleotide, a polynucleoside, a polypeptide, an immunomodulator, an antigen, and an adjuvant.

Another type of gene delivery polymer is formed from beta-amino esters. Agents in this series, such as C32, U28, and JJ28, were identified using a combinatorial library approach. C32 may be especially useful for tumor immunotherapy as it has been shown to increase plasmid DNA gene expression in tumors 4-fold. To complex plasmid DNA to C32, U28, or JJ28, the polymer is first dissolved in DMSO (100 mg/ml). DNA (50 µg) is then suspended in 25 µl of 25 mM sodium acetate buffer (pH 5.0) and mixed with the polymer solution (1,500 µg or 25 µg), also diluted in 25 µl of 25 mM sodium acetate buffer (pH 5.0). After incubation of the polymer/DNA mixture at room temperature for 5 min, 10 µl of 30% glucose in PBS is added to the 50-µl polymer/DNA mixture. If 50 µg of DNA is used with 1,500 µg of polymer, this is referred to as a 1:30 ratio. If 50 µg of DNA is used with 25 µg of polymer, this is referred to as a 2:1 ratio. In previous studies, the 1:30 ratio worked well for intratumoral injections whereas the 2:1 ratio worked best for i.m. DNA vaccination.

For DNA vaccination, one approach for targeting DNA to DCs is to adsorb it onto cationic poly(lactic-co-glycolic acid) (PLGA) particles, which then targets the DNA to phagocytic APCs and enhances CD8+ T cell responses and antibody titers by 100-fold and 1,000-fold respectively. Despite this advantage, even low-molecular-weight PLGA systems require up to 13 days to fully release encapsulated DNA after DC uptake in vitro. This period is too long as most DCs die within 7 days after activation and migration to draining lymph nodes. Furthermore, PLGA microparticles can produce an extremely low pH microclimate (pH < 3.5) after only 3 days in an aqueous environment. This level of acidity has been shown to severely reduce the activity of plasmid DNA. PLGA microparticles also remain confined to phagolysosomal vesicles, which limits gene expression in the transfected DCs. Consequently, a biodegradable, pH-sensitive poly-amino ester (PBAE) can be included in combination with PLGA, so that the microparticles instantaneously release their payload following intracellular pH changes. The encapsulation of a DNA vaccine within these hybrid PLGA/PBAE microparticles strongly enhanced CD8+ T cell responses and also stimulated DCs to upregulate CD40. To prepare plasmid DNA microencapsulated with PBAE and PLGA, 1 mg of plasmid DNA is added to an aqueous solution of 1 mM EDTA and 300 nM D(+)-Lactose, and then emulsified in a sonicator with 200 mg of a PBAE/PLGA mixture in CH₂Cl₂. The resulting emulsion is then added to a solution of 50% poly(vinyl alchohol) and 0.2 M NaCl, then added to a second solution of poly(vinyl alcohol) for 3 hours, washed by repeated centrifugation, and then lyophylized for storage at - 20 °C. Two types of PBAE/PLGA mixtures are preferred: 15% PBAE/85% PLGA and 25% PBAE/75% PLGA. Although the 25% PBAE mixture was significantly more stimulatory for DCs in vitro, the 15% and 25% PBAE mixtures were nearly equivalent when used for intradermal DNA vaccination. In either case, the final lyophylized microparticle preparation is resuspended for use in PBS at a concentration of 10 µg/50 µl, where the 10 µg refers to the amount of DNA in the particles.

Peritumoral injections of "naked" plasmid DNA for IL-12 have been used for antitumor treatment in mice. Further, the use of poly[alpha-(4-aminobutyl)-1-glycolic acid] (PAGA) to deliver IL-12 plasmid DNAs to tumor-bearing mice is known in the art. Moreover, the use of water-soluble lipopolymer (WSLP) and an interleukin-12 (IL-12) expression plasmid for enhanced delivery of the IL-12 gene, using branched polyethylenimine and cholesteryl chloroformate has also been described. Polyethylenimine-based vesicle-polymer hybrid gene delivery as another way to deliver plasmid DNA expression vectors, including the use of poly(propylenimine) dendrimers as delivery agents Also, polyethylene glycol (PEG) copolymers were found to improve plasmid DNA delivery, including various kinds of polymers that can be used for the controlled release of plasmid DNA and other nucleic acids. Such molecules include poly(lactic acid) and its derivatives, PEGylated poly(lactic acid), poly(lactic-co-glycolic acid) and its derivatives, poly(ortho esters) and their derivatives, PEGylated poly(ortho esters), poly(caprolactone) and its derivatives, PEGylated poly(caprolactone), polylysine and its derivatives, PEGylated polylysine, poly(ethylene imine) and its derivatives, PEGylated poly(ethylene imine), poly(acrylic acid) and its derivatives, PEGylated poly(acrylic acid), poly(urethane) and its derivatives, PEGylated poly(urethane), and combinations of all of these. One object of the present invention is the use of polymeric lipid-protein-sugar microparticles for the delivery of nucleic acids. A further object is the use of polymers that are hydrolyzable inside of cellular endosomes, so that their nucleic acid cargo is appropriately released in the intracellular environment. The general utility of using biodegradable particles to deliver nucleic acids is known in the art.

Self-assembling particle delivery systems are often composite substances, including self-assembling particles that can be made as polyplexes between nucleic acids and a hybrid polymer composed of mannose-polyethylene glycol (PEG)-PAMAM-G3.0, -G4.0, or -G5.0, where PAMAM refers to a branching dendrimer of poly(amidoamine) and the G indicates the number of branches. Combining a solution of these linear-dendritic hybrid polymers with plasmid DNA resulted in self-assembled particles about 200 nm in diameter with the DNA in the center and the mannose residues on the outside. In this case, mannose is used to form the outer shell of this nanoparticle because immature DCs and macrophages avidly take up mannosylated substances using their mannose receptors (which are downregulated upon DC maturation) and possibly other mannose-binding receptors such as DC-SIGN. Using the P388D1 macrophage cell line, the resulting polyplexes of a luciferase plasmid with Man-PEG-PAMAM-G5.0 or - G6.0 resulted in 4-fold more gene expression than plasmid complexation with commercially available JetPET (QBioGene, Inc.), whereas the G4.0 polymer was equivalent to JetPEI. The -G6.0 polymer was mildly toxic to these cells, but the G5.0 polymer was essentially nontoxic at concentrations 100X greater than the toxic dose of JetPEI.

Immunostimulatory attributes of polymer delivery systems. Polymeric gene delivery systems need not be biologically inert. Indeed, they may be even more effective if they are immunostimulatory in their own right, in which case they may be preferred for vaccination and tumor immunotherapy. For example, polymers many have intrinsic anticancer effects. Polypropylenimine (PPI) dendrimers have been observed to augment the antitumor effects of TNF plasmid DNA. Interestingly, the PPI dendrimers alone had some antitumor effects, as did linear polyethylenimine (PEI) and polyamidoamine dendrimer, including that PPI dendrimers induce gene expression in transfected cells, a property that could be useful in immunostimulation or antitumor activity. Using different polymers, microencapsulation of plasmid DNA in poly(lactic-co-glycolic acid) (PLGA)/poly-amino ester (PBAE) mixtures leads to direct activation of dendritic cells.

In one aspect, nucleic acids are delivered by electroporation. Elecroporation uses electrical pulses to introduce proteins, nucleic acids, lipids, carbohydrates, or mixtures thereof into the host to produce an effect. A typical use of electroporation is to introduce a nucleic acid into the host so that the protein encoded by the nucleic acid is efficiently produced.

In another aspect, nucleic acids are delivered by article bombardment. Powderject (Norvartis Pharmaceutical Corporation) has developed methods to coat gold particles with nucleic acids and other substances and then forcibly introduce them into the host by particle bombardment. For nucleic acids encoding antigens, this results in an improved immune response to the antigens.

It is one objective of the present invention to combine of ISS-ODN with improved delivery agents. For example, CpG-ODN combined with a polycationic polymer improved the response to vaccination. Alternatively, CpG-ODN can be mixed into an oil-in-water emulsion to form vesicles which improves its immunostimulatory capacity. CpG-ODN can also be incorporated into a microcarrier complex less than 10 microns in size. CpG-ODN adsorbed onto polylactide-co-glycolide microparticles improved the immune response to an anthrax vaccine. Adsorption onto cationic PLGA microparticles provides enhanced efficacy for tuberculosis DNA vaccines. It is one objective of the present invention to form delivery agents from PLGA and other materials that incorporate DNA and TLR agonists such as CpG-ODN.

In some embodiments, the immunostimulatory combination may further include an antigen. When present in the immunostimulatory combination, the antigen may be administered in an amount that, in combination with the other components of the combination, is effective to generate an immune response against the antigen. For example, the antigen can be administered in an amount from about 100 ng/kg to about 100 mg/kg. In some embodiments, the antigen may be administered in an amount from about 10 µg/kg to about 10 mg/kg. In some embodiments, the antigen may be administered in an amount from about 1 mg/kg to about 5 mg/kg. The particular amount of antigen that constitutes an amount effective to generate an immune response, however, depends to some extent upon certain factors such as, for example, the particular antigen being administered; the particular agonist being administered and the amount thereof; the particular agonist being administered and the amount thereof; the state of the immune system; the method and order of administration of the agonist and the antigen; the species to which the formulation is being administered; and the desired therapeutic result. Accordingly, it is not practical to set forth generally the amount that constitutes an effective amount of the antigen. Those of ordinary skill in the art, however, can readily determine the appropriate amount with due consideration of such factors.

The antigen can be any material capable of raising a Th1 immune response, which may include one or more of, for example, a CD8+ T cell response, an NK T cell response, a γ/8 T cell response, or a Th1 antibody response. Suitable antigens include but are not limited to peptides; polypeptides; lipids; glycolipids; polysaccharides; carbohydrates; polynucleotides; prions; live or inactivated bacteria, viruses or fungi; and bacterial, viral, fungal, protozoal, tumor-derived, or organism-derived antigens, toxins or toxoids.

Furthermore, certain currently experimental antigens, especially materials such as recombinant proteins, glycoproteins, and peptides that do not raise a strong immune response, can be used in connection with adjuvant combinations of the invention. Exemplary experimental subunit antigens include those related to viral disease such as adenovirus, AIDS, chicken pox, cytomegalovirus, dengue, feline leukemia, fowl plague, hepatitis A, hepatitis B, HSV-1, HSV-2, hog cholera, influenza A, influenza B, Japanese encephalitis, measles, parainfluenza, rabies, respiratory syncytial virus, rotavirus, wart, and yellow fever.

In one embodiment, the antigen may be a cancer antigen or a tumor antigen. The terms cancer antigen and tumor antigen are used interchangeably and refer to an antigen that is differentially expressed by cancer cells. Therefore, cancer antigens can be exploited to differentially target an immune response against cancer cells. Cancer antigens may thus potentially stimulate tumor-specific immune responses. Certain cancer antigens are encoded, though not necessarily expressed, by normal cells. Some of these antigens may be characterized as normally silent (i.e., not expressed) in normal cells, those that are expressed only at certain stages of differentiation, and those that are temporally expressed (e.g., embryonic and fetal antigens). Other cancer antigens can be encoded by mutant cellular genes such as, for example, oncogenes (e.g., activated ras oncogene), suppressor genes (e.g., mutant p53), or fusion proteins resulting from internal deletions or chromosomal translocations. Still other cancer antigens can be encoded by viral genes such as those carried by RNA and DNA tumor viruses.

Examples of tumor antigens include MAGE, MART-1/Melan-A, gp100, Dipeptidyl peptidase IV (DPPUV), adenosine deaminase-binding protein (ADAbp), cyclophilin b, Colorectal associated antigen (CRC)-C017-1A/GA733, Carcinoembryonic Antigen (CEA) and its antigenic epitopes CAP-1 and CAP-2, etv6, am11, Prostate Specific Antigen (PSA) and its antigenic epitopes PSA-1, PSA-2, and PSA-3, prostate-specific membrane antigen (PSMA), T-cell receptor/CD3-.zeta. chain, MAGE-family of tumor antigens (e.g., MAGE-A1, MAGE-A2, MAGE-A3, MAGE-A4, MAGE-A5, MAGE-A6, MAGE-A7, MAGE-A8, MAGE-A9, MAGE-A10, MAGE-A11, MAGE-A12, MAGE-Xp2 (MAGE-B2), MAGE-Xp3 (MAGE-B3), MAGE-Xp4 (MAGE-B4), MAGE-C1, MAGE-C2, MAGE-C3, MAGE-C4, MAGE-C5), GAGE-family of tumor antigens (e.g., GAGE-1, GAGE-2, GAGE-3, GAGE-4, GAGE-5, GAGE-6, GAGE-7, GAGE-8, GAGE-9), BAGE, RAGE, LAGE-1, NAG, GnT-V, MUM-1, CDK4, tyrosinase, p53, MUC family, HER2/neu, p21ras, RCAS1, α-fetoprotein, E-cadherin, α-catenin, β-catenin, γ-catenin, p 120ctn, gp100^{Pmell17}, PRAME, NY-ESO-1, cdc27, adenomatous polyposis coli protein (APC), fodrin, Connexin 37, Ig-idiotype, p 15, gp75, GM2 and GD2 gangliosides, viral products such as human papilloma virus proteins, Smad family of tumor antigens, Imp-1, P1A, EBV-encoded nuclear antigen (EBNA)-1, brain glycogen phosphorylase, SSX-1, SSX-2 (HOM-MEL-40), SSX-3, SSX-4, SSX-5, SCP-1 and CT-7, and c-erbB-2.

Cancers or tumors and specific tumor antigens associated with such tumors (but not exclusively), include acute lymphoblastic leukemia (etv6, am11, cyclophilin b), B cell lymphoma (Ig-idiotype), glioma (E-cadherin, α-catenin, β-catenin, γ-catenin, p120ctn), bladder cancer (p21ras), biliary cancer (p21ras), breast cancer (MUC family, HER2/neu, c-erbB-2), cervical carcinoma (p53, p21ras), colon carcinoma (p21ras, HER2/neu, c-erbB-2, MUC family), colorectal cancer (Colorectal associated antigen (CRC)-CO17-1A/GA733, APC), choriocarcinoma (CEA), epithelial cell cancer (cyclophilin b), gastric cancer (HER2/neu, c-erbB-2, ga733 glycoprotein), hepatocellular cancer (α-fetoprotein), Hodgkins lymphoma (Imp-1, EBNA-1), lung cancer (CEA, MAGE-3, NY-ESO-1), lymphoid cell-derived leukemia (cyclophilin b), melanoma (p5 protein, gp75, oncofetal antigen, GM2 and GD2 gangliosides, Melan-A/MART-1, cdc27, MAGE-3, p21ras, gp100^{Pmll17}), myeloma (MUC family, p21ras), non-small cell lung carcinoma (HER2/neu, c-erbB-2), nasopharyngeal cancer (Imp-1, EBNA-1), ovarian cancer (MUC family, HER2/neu, c-erbB-2), prostate cancer (Prostate Specific Antigen (PSA) and its antigenic epitopes PSA-1, PSA-2, and PSA-3, PSMA, HER2/neu, c-erbB-2, ga733 glycoprotein), renal cancer (HER2/neu, c-erbB-2), squamous cell cancers of the cervix and esophagus (viral products such as human papilloma virus proteins), testicular cancer (NY-ESO-1), and T cell leukemia (HTLV-1 epitopes).

Immunostimulatory combinations of the invention that include an antigen may form a vaccine. Such vaccines can contain additional pharmaceutically acceptable ingredients, excipients, carriers, and the like well known to those skilled in the art.

Immunostimulatory combinations of the invention can be administered to animals, e.g., mammals (human and non-human), fowl, and the like according to conventional methods well known to those skilled in the art (e.g., orally, subcutaneously, nasally, topically).

The invention also provides therapeutic and/or prophylactic methods that include administering an immunostimulatory combination of the invention to a subject.

Unless a specific sequence of administration is provided, components of the immunostimulatory combination may be administered simultaneously with the antigen (together in admixture or separately, e.g., orally or by separate injection) or subsequent to administering one or more other components of the immunostimulatory combination.

A therapeutic combination can be provided in further combination with one or more pharmaceutically acceptable carriers. Because the combination as disclosed and antigen (if present in the combination) may be co-administered sequentially, at different sites, and/or by different routes, a therapeutic combination may be provided in two or more formulations. When provided in two or more formulations, each formulation can include a carrier similar or different than the carrier or carriers included in the remaining formulations. Alternatively, the combination as disclosed, and antigen (if present in the combination) may be provided in a single formulation, which can include a single carrier or a combination of carriers.

Each component or mixture of components may be administered in any suitable conventional dosage form such as, for example, tablets, lozenges, parenteral formulations, syrups, creams, ointments, aerosol formulations, transdermal patches, transmucosal patches and the like.

Therapeutic immunostimulatory combinations can be administered as the single therapeutic agent in the treatment regimen. Alternatively, a therapeutic immunostimulatory combination of the invention may be administered in combination with another therapeutic combination of the invention, with one or more pharmaceutical compositions, or with other active agents such as antivirals, antibiotics, and the like.

The invention also provides a method of treating a viral infection in an animal and a method of treating a neoplastic disease in an animal comprising administering a therapeutically effective amount of an immunostimulatory combination of the invention to the animal. A therapeutically effective amount to treat or inhibit a viral infection is an amount that will cause a reduction in one or more of the manifestations of viral infection, such as viral lesions, viral load, rate of virus production, and mortality as compared to untreated control animals. A therapeutically effective amount of a combination to treat a neoplastic disease is an amount that will cause, for example, a reduction in tumor size, a reduction in the number of tumor foci, or slow the growth of a tumor, as compared to untreated animals.

Treatments according to the present invention may include one or more than one immunization. When the treatment includes more than one immunization, the treatment can include any suitable number of immunizations administered at any suitable frequency. The number and frequency of immunizations in a treatment regimen depend at least in part upon one or more factors including but not limited to the condition being treated and the stage thereof, the state of the subject's immune system, the particular agonists being administered and the amount thereof, and the particular antigen being administered (if present) and the amount thereof.

The phrase "pharmaceutically acceptable" refers to molecular entities and compositions that are physiologically tolerable and do not typically produce an allergic or similar untoward reaction, such as gastric upset, dizziness and the like, when administered to a human. In one embodiment, as used herein, the term "pharmaceutically acceptable" means approved by a regulatory agency of the Federal or a state government or listed in the U.S. Pharmacopeia or other generally recognized pharmacopeia for use in animals, and more particularly in humans. The term "carrier" refers to a diluent, adjuvant, excipient, or vehicle with which the compound is administered. Such pharmaceutical carriers can be sterile liquids, such as water and oils, including those of petroleum, animal, vegetable or synthetic origin, such as peanut oil, soybean oil, mineral oil, sesame oil and the like. Water or aqueous solution saline solutions and aqueous dextrose and glycerol solutions are preferably employed as carriers, particularly for injectable solutions. Suitable pharmaceutical carriers are described in "Remington's Pharmaceutical Sciences" by E. W. Martin.

In additional embodiments, the present invention concerns formulation of one or more of the polynucleotides, TLR agonists, and/or cationic polymer compositions disclosed herein in combination with pharmaceutically-acceptable carriers for administration to a cell or an animal, either alone, or in combination with one or more other modalities of therapy.

In another embodiment, vaccines may be delivered by incorporating DNA encoding a fusion protein into a biological agent. As used herein, a "biological agent" is a prokaryotic cell, eukaryotic cell, or virus. Such agents also include tumor cells. Such agents as disclosed herein can be combined with pharmaceutically-acceptable carriers for administration to a cell or an animal, either alone, or in combination with one or more other modalities of therapy. In other embodiments, DNA vaccines encoding such a fusion protein is formulated with pharmaceutically-acceptable carriers for administration to a cell or an animal, either alone, or in combination with one or more other modalities of therapy.

In a related aspect, such pharmaceutical compositions may be administered to a subject as a prophylactic or ameliorative modality. As used herein, "ameliorative," means to improve or relieve a subject of symptoms associated with a disorder, and includes curing such a disorder. For example, the vaccines as disclosed in the present invention can be administered to a subject before onset of an infection or after the subject has been infected.

It will be understood that, if desired, a composition as disclosed herein may be administered in combination with other agents as well, such as, e.g., other proteins or polypeptides or various pharmaceutically-active agents. In fact, there is virtually no limit to other components that may also be included, given that the additional agents do not cause a significant adverse effect upon contact with the target cells or host tissues. The compositions may thus be delivered along with various other agents as required in the particular instance. Such compositions may be purified from host cells or other biological sources, or alternatively may be chemically synthesized as described herein. Likewise, such compositions may further comprise substituted or derivatized RNA or DNA compositions.

Therefore, in another aspect of the present invention, pharmaceutical compositions are provided comprising one or more of the polynucleotide, TLR agonists, and/or cationic polymer compositions described herein in combination with a physiologically acceptable carrier. In certain preferred embodiments, the pharmaceutical compositions of the invention comprise immunogenic polynucleotide and/or polypeptide compositions of the invention for use in prophylactic and therapeutic vaccine applications. Vaccine preparation is generally described in, for example, M. F. Powell and M. J. Newman, eds., "Vaccine Design (the subunit and adjuvant approach)," Plenum Press (NY, 1995). Generally, such compositions will comprise one or more polynucleotide and/or polypeptide compositions of the present invention in combination with one or more immunostimulants.

It will be apparent that any of the pharmaceutical compositions described herein can contain pharmaceutically acceptable salts of the polynucleotides and polypeptides of the invention. Such salts can be prepared, for example, from pharmaceutically acceptable non-toxic bases, including organic bases (e.g., salts of primary, secondary and tertiary amines and basic amino acids) and inorganic bases (e.g., sodium, potassium, lithium, ammonium, calcium and magnesium salts).

In another embodiment, illustrative immunogenic compositions, e.g., vaccine compositions, of the present invention comprise DNA encoding one or more of the polypeptides as described above, such that the polypeptide is generated in situ. As noted above, the polynucleotide may be administered within any of a variety of delivery systems known to those of ordinary skill in the art. Indeed, numerous gene delivery techniques are well known in the art, such as those described by Rolland, Crit. Rev. Therap. Drug Carrier Systems 15:143-198, 1998. Appropriate polynucleotide expression systems will, of course, contain the necessary regulatory DNA regulatory sequences for expression in a patient (such as a suitable promoter and terminating signal). Alternatively, biological delivery systems may involve the administration of a infectious agent or neoplastic cell or tissue that expresses an immunogenic portion of the polypeptide on its cell surface or secretes such an epitope.

In certain embodiments, polynucleotides encoding immunogenic polypeptides described herein are introduced into suitable mammalian host cells for expression using any of a number of known viral-based systems. In one embodiment, retroviruses or lentiviruses provide a convenient and effective platform for gene delivery systems. A selected nucleotide sequence encoding a polypeptide of the present invention can be inserted into a vector and packaged in retroviral or lentiviral particles using techniques known in the art. The recombinant virus can then be isolated and delivered to a subject. A number of illustrative retroviral systems have been described (e.g., U.S. Pat. No. 5,219,740; Miller and Rosman (1989) BioTechniques 7:980-990; Miller, A. D. (1990) Human Gene Therapy 1:5-14; Scarpa et al. (1991) Virology 180:849-852; Bums et al. (1993) Proc. Natl. Acad. Sci. USA 90:8033-8037; and Boris-Lawrie and Temin (1993) Cur. Opin. Genet. Develop. 3:102-109. An illustrative lentiviral system has been described by L. Naldini et al, Proc. Natl. Acad. Sci. USA 93: 11382-11388, 1996.

In addition, a number of illustrative adenovirus-based systems have also been described. Unlike retroviruses which integrate into the host genome, adenoviruses persist extrachromosomally thus minimizing the risks associated with insertional mutagenesis (Haj-Ahmad and Graham (1986) J. Virol. 57:267-274; Bett et al. (1993) J. Virol. 67:5911-5921; Mittereder et al. (1994) Human Gene Therapy 5:717-729; Seth et al. (1994) J. Virol. 68:933-940; Barr et al. (1994) Gene Therapy 1:51-58; Berkner, K. L. (1988) BioTechniques 6:616-629; and Rich et al. (1993) Human Gene Therapy 4:461-476).

Various adeno-associated virus (AAV) vector systems have also been developed for polynucleotide delivery. AAV vectors can be readily constructed using techniques well known in the art. See, e.g., U.S. Pat. Nos. 5,173,414 and 5,139,941; International Publication Nos. WO 92/01070 and WO 93/03769; Lebkowski et al. (1988) Molec. Cell. Biol. 8:3988-3996; Vincent et al. (1990) Vaccines 90 (Cold Spring Harbor Laboratory Press); Carter, B. J. (1992) Current Opinion in Biotechnology 3:533-539; Muzyczka, N. (1992) Current Topics in Microbiol. and Immunol. 158:97-129; Kotin, R. M. (1994) Human Gene Therapy 5:793-801; Shelling and Smith (1994) Gene Therapy 1:165-169; and Zhou et al. (1994) J. Exp. Med. 179:1867-1875.

Additional viral vectors useful for delivering the polynucleotides encoding polypeptides of the present invention by gene transfer include those derived from the pox family of viruses, such as vaccinia virus and avian poxvirus. By way of example, vaccinia virus recombinants expressing the novel molecules can be constructed as follows. The DNA encoding a polypeptide is first inserted into an appropriate vector so that it is adjacent to a vaccinia promoter and flanking vaccinia DNA sequences, such as the sequence encoding thymidine kinase (TK). This vector is then used to transfect cells which are simultaneously infected with vaccinia. Homologous recombination serves to insert the vaccinia promoter plus the gene encoding the polypeptide of interest into the viral genome. The resulting TK⁽⁻⁾ recombinant can be selected by culturing the cells in the presence of 5-bromodeoxyuridine and picking viral plaques resistant thereto.

A vaccinia-based infection/transfection system can be conveniently used to provide for inducible, transient expression or coexpression of one or more polypeptides described herein in host cells of an organism. In this particular system, cells are first infected in vitro with a vaccinia virus recombinant that encodes the bacteriophage T7 RNA polymerase. This polymerase displays exquisite specificity in that it only transcribes templates bearing T7 promoters. Following infection, cells are transfected with the polynucleotide or polynucleotides of interest, driven by a T7 promoter. The polymerase expressed in the cytoplasm from the vaccinia virus recombinant transcribes the transfected DNA into RNA which is then translated into polypeptide by the host translational machinery. The method provides for high level, transient, cytoplasmic production of large quantities of RNA and its translation products. See, e.g., Elroy-Stein and Moss, Proc. Natl. Acad. Sci. USA (1990) 87:6743-6747; Fuerst et al. Proc. Natl. Acad. Sci. USA (1986) 83:8122-8126.

Alternatively, avipoxviruses, such as the fowlpox and canarypox viruses, can also be used to deliver the coding sequences of interest. Recombinant avipox viruses, expressing immunogens from mammalian pathogens, are known to confer protective immunity when administered to non-avian species. The use of an Avipox vector is particularly desirable in human and other mammalian species since members of the Avipox genus can only productively replicate in susceptible avian species and therefore are not infective in mammalian cells. Methods for producing recombinant Avipoxviruses are known in the art and employ genetic recombination, as described above with respect to the production of vaccinia viruses. See, e.g., WO 91/12882; WO 89/03429; and WO 92/03545.

Any of a number of alphavirus vectors can also be used for delivery of polynucleotide compositions of the present invention, such as those vectors described in U.S. Pat. Nos. 5,843,723; 6,015,686; 6,008,035 and 6,015,694. Certain vectors based on Venezuelan Equine Encephalitis (VEE) can also be used, illustrative examples of which can be found in U.S. Pat. Nos. 5,505,947 and 5,643,576.

Moreover, molecular conjugate vectors, such as the adenovirus chimeric vectors described in Michael et al. J. Biol. Chem. (1993) 268:6866-6869 and Wagner et al. Proc. Natl. Acad. Sci. USA (1992) 89:6099-6103, can also be used for gene delivery under the invention.

Additional illustrative information on these and other known viral-based delivery systems can be found, for example, in Fisher-Hoch et al., Proc. Natl. Acad. Sci. USA 86:317-321, 1989; Flexner et al., Ann. N.Y. Acad. Sci. 569:86-103, 1989; Flexner et al., Vaccine 8:17-21, 1990; U.S. Pat. Nos. 4,603,112, 4,769,330, and 5,017,487; WO 89/01973; U.S. Pat. No. 4,777,127; GB 2,200,651; EP 0,345,242; WO 91/02805; Berkner, Biotechniques 6:616-627, 1988; Rosenfeld et al., Science 252:431-434, 1991; Kolls et al., Proc. Natl. Acad. Sci. USA 91:215-219, 1994; Kass-Eisler et al., Proc. Natl. Acad. Sci. USA 90:11498-11502, 1993; Guzman et al., Circulation 88:2838-2848, 1993; and Guzman et al., Cir. Res. 73:1202-1207, 1993.

In certain embodiments, a polynucleotide may be integrated into the genome of a target cell. This integration may be in the specific location and orientation via homologous recombination (gene replacement) or it may be integrated in a random, non-specific location (gene augmentation). In yet further embodiments, the polynucleotide may be stably maintained in the cell as a separate, episomal segment of DNA. Such polynucleotide segments or "episomes" encode sequences sufficient to permit maintenance and replication independent of or in synchronization with the host cell cycle. The manner in which the expression construct is delivered to a cell and where in the cell the polynucleotide remains is dependent on the type of expression construct employed.

In another embodiment of the invention, a polynucleotide is administered/delivered as "naked" DNA, for example as described in Ulmer et al., Science 259:1745-1749, 1993 and reviewed by Cohen, Science 259:1691-1692, 1993. The uptake of naked DNA may be increased by coating the DNA onto biodegradable beads, which are efficiently transported into the cells.

In still another embodiment, a composition of the present invention can be delivered via a particle bombardment approach, many of which have been described. In one illustrative example, gas-driven particle acceleration can be achieved with devices such as those manufactured by Powderject Pharmaceuticals PLC (Oxford, UK) and Powderject Vaccines Inc. (Madison, Wis.), both now part of the Chiron division ofNovartis, some examples of which are described in U.S. Pat. Nos. 5,846,796; 6,010,478; 5,865,796; 5,584,807; and EP Patent No. 0500 799. This approach offers a needle-free delivery approach wherein a dry powder formulation of microscopic particles, such as polynucleotide or polypeptide particles, are accelerated to high speed within a helium gas jet generated by a hand held device, propelling the particles into a target tissue of interest.

In a related embodiment, other devices and methods that may be useful for gas-driven needle-less injection of compositions of the present invention include those provided by Bioject, Inc. (Portland, Oreg.), some examples of which are described in U.S. Pat. Nos. 4,790,824; 5,064,413; 5,312,335; 5,383,851; 5,399,163; 5,520,639 and 5,993,412.

Typically, these formulations will contain at least about 0.1% of the active compound or more, although the percentage of the active ingredient(s) may, of course, be varied and may conveniently be between about 1 or 2% and about 60% or 70% or more of the weight or volume of the total formulation. Naturally, the amount of active compound(s) in each therapeutically useful composition may be prepared is such a way that a suitable dosage will be obtained in any given unit dose of the compound. Factors such as solubility, bioavailability, biological half-life, route of administration, product shelf life, as well as other pharmacological considerations will be contemplated by one skilled in the art of preparing such pharmaceutical formulations, and as such, a variety of dosages and treatment regimens may be desirable.

For oral administration the compositions of the present invention may alternatively be incorporated with one or more excipients in the form of a mouthwash, dentifrice, buccal tablet, oral spray, or sublingual orally-administered formulation. Alternatively, the active ingredient may be incorporated into an oral solution such as one containing sodium borate, glycerin and potassium bicarbonate, or dispersed in a dentifrice, or added in a therapeutically-effective amount to a composition that may include water, binders, abrasives, flavoring agents, foaming agents, and humectants. Alternatively the compositions may be fashioned into a tablet or solution form that may be placed under the tongue or otherwise dissolved in the mouth. Vaccine formulations can also be delivered to the nasal mucosa, aerosolized for inhalational delivery, or delivered to the mucosal surfaces of the female and male genital track or the rectum. Vaccine formations may also be formulated for transdermal delivery.

In certain circumstances it will be desirable to deliver the pharmaceutical compositions disclosed herein parenterally, intravenously, intramuscularly, or even intraperitoneally. Such approaches are well known to the skilled artisan, some of which are further described, for example, in U.S. Pat. No. 5,543,158; U.S. Pat. No. 5,641,515 and U.S. Pat. No. 5,399,363. In certain embodiments, solutions of the active compounds as free base or pharmacologically acceptable salts may be prepared in water suitably mixed with a surfactant, such as hydroxypropylcellulose. Dispersions may also be prepared in glycerol, liquid polyethylene glycols, and mixtures thereof and in oils. Under ordinary conditions of storage and use, these preparations generally will contain a preservative to prevent the growth of microorganisms.

Illustrative pharmaceutical forms suitable for injectable use include sterile aqueous solutions or dispersions and sterile powders for the extemporaneous preparation of sterile injectable solutions or dispersions (for example, see U.S. Pat. No. 5,466,468). In all cases the form must be sterile and must be fluid to the extent that easy syringability exists. It must be stable under the conditions of manufacture and storage and must be preserved against the contaminating action of microorganisms, such as bacteria and fungi. The carrier can be a solvent or dispersion medium containing, for example, water, ethanol, polyol (e.g., glycerol, propylene glycol, and liquid polyethylene glycol, and the like), suitable mixtures thereof, and/or vegetable oils. Proper fluidity may be maintained, for example, by the use of a coating, such as lecithin, by the maintenance of the required particle size in the case of dispersion and/or by the use of surfactants. The prevention of the action of microorganisms can be facilitated by various antibacterial and antifungal agents, for example, parabens, chlorobutanol, phenol, sorbic acid, thimerosal, and the like. In many cases, it will be preferable to include isotonic agents, for example, sugars or sodium chloride. Prolonged absorption of the injectable compositions can be brought about by the use in the compositions of agents delaying absorption, for example, aluminum monostearate and gelatin.

In one embodiment, for parenteral administration in an aqueous solution, the solution should be suitably buffered if necessary and the liquid diluent first rendered isotonic with sufficient saline or glucose. These particular aqueous solutions are especially suitable for intravenous, intramuscular, subcutaneous and intraperitoneal administration. In this connection, a sterile aqueous medium that can be employed will be known to those of skill in the art in light of the present disclosure. For example, one dosage may be dissolved in 1 ml of isotonic NaCl solution and either added to 1000 ml of hypodermoclysis fluid or injected at the proposed site of infusion, (see for example, "Remington's Pharmaceutical Sciences" 15th Edition, pages 1035-1038 and 1570-1580). Some variation in dosage will necessarily occur depending on the condition of the subject being treated. Moreover, for human administration, preparations will of course preferably meet sterility, pyrogenicity, and the general safety and purity standards as required by FDA Office of Biologics standards.

In another embodiment of the invention, the compositions disclosed herein may be formulated in a neutral or salt form. Illustrative pharmaceutically-acceptable salts include the acid addition salts (formed with the free amino groups of the protein) and which are formed with inorganic acids such as, for example, hydrochloric or phosphoric acids, or such organic acids as acetic, oxalic, tartaric, mandelic, and the like. Salts formed with the free carboxyl groups can also be derived from inorganic bases such as, for example, sodium, potassium, ammonium, calcium, or ferric hydroxides, and such organic bases as isopropylamine, trimethylamine, histidine, procaine and the like. Upon formulation, solutions will be administered in a manner compatible with the dosage formulation and in such amount as is therapeutically effective.

The carriers can further comprise any and all solvents, dispersion media, vehicles, coatings, diluents, antibacterial and antifungal agents, isotonic and absorption delaying agents, buffers, carrier solutions, suspensions, colloids, and the like. The use of such media and agents for pharmaceutical active substances is well known in the art. Except insofar as any conventional media or agent is incompatible with the active ingredient, its use in the therapeutic compositions is contemplated. Supplementary active ingredients can also be incorporated into the compositions. The phrase "pharmaceutically-acceptable" refers to molecular entities and compositions that do not produce an allergic or similar untoward reaction when administered to a human.

In certain embodiments, the pharmaceutical compositions may be delivered by intranasal sprays, inhalation, and/or other aerosol delivery vehicles. Methods for delivering genes, nucleic acids, and peptide compositions directly to the lungs via nasal aerosol sprays has been described, e.g., in U.S. Pat. No. 5,756,353 and U.S. Pat. No. 5,804,212. Likewise, the delivery of drugs using intranasal microparticle resins (Takenaga et al., J Controlled Release (1998) 52(1-2):81-7) and lysophosphatidyl-glycerol compounds (U.S. Pat. No. 5,725,871) are also well-known in the pharmaceutical arts. Likewise, illustrative transmucosal drug delivery in the form of a polytetrafluoroetheylene support matrix is described in U.S. Pat. No. 5,780,045.

In certain embodiments, liposomes, nanocapsules, microparticles, lipid particles, vesicles, and the like, are used for the introduction of the compositions of the present invention into suitable host cells/organisms. In particular, the compositions of the present invention may be formulated for delivery either encapsulated in a lipid particle, a liposome, a vesicle, a nanosphere, or a nanoparticle or the like. Alternatively, compositions of the present invention can be bound, either covalently or non-covalently, to the surface of such carrier vehicles.

The formation and use of liposome and liposome-like preparations as potential drug carriers is generally known to those of skill in the art (see for example, Lasic, Trends Biotechnol (1998) 16(7):307-21; Takakura, Nippon Rinsho (1998) 56(3):691-5; Chandran et al., Indian J Exp Biol (1997) 35(8):801-9; Margalit, Crit Rev Ther Drug Carrier Syst (1995) 12(2-3):233-61; U.S. Pat. No. 5,567,434; U.S. Pat. No. 5,552,157; U.S. Pat. No. 5,565,213; U.S. Pat. No. 5,738,868 and U.S. Pat. No. 5,795,587.

Liposomes have been used successfully with a number of cell types that are normally difficult to transfect by other procedures, including T cell suspensions, primary hepatocyte cultures and PC 12 cells (Renneisen et al., J Biol. Chem (1990) 265(27):16337-42; Muller et al., DNA Cell Biol (1990) 9(3):221-9). In addition, liposomes are free of the DNA length constraints that are typical of viral-based delivery systems. Liposomes have been used effectively to introduce genes, various drugs, radiotherapeutic agents, enzymes, viruses, transcription factors, allosteric effectors and the like, into a variety of cultured cell lines and animals. Furthermore, the use of liposomes does not appear to be associated with autoimmune responses or unacceptable toxicity after systemic delivery.

In certain embodiments, liposomes are formed from phospholipids that are dispersed in an aqueous medium and spontaneously form multilamellar concentric bilayer vesicles (also termed multilamellar vesicles (MLVs).

Alternatively, in other embodiments, the invention provides for pharmaceutically-acceptable nanocapsule formulations of the compositions of the present invention. Nanocapsules can generally entrap compounds in a stable and reproducible way (see, for example, Quintanar-Guerrero et al., Drug Dev India Pharm (1998) 24(12):1113-28). To avoid side effects due to intracellular polymeric overloading, such ultrafine particles (sized around 0.1 µm) may be designed using polymers able to be degraded in vivo. Such particles can be made as described, for example, by Couvreur et al., Crit Rev Ther Drug Carrier Syst. 1988; 5(1):1-20; zur Muhlen et al., Eur J Pharm Biopharm (1998) 45(2):149-55; Zambaux et al., J Controlled Release (1998) 50(1-3):31-40; and U.S. Pat. No. 5,145,684.

The following examples are intended to illustrate but not limit the invention.

### EXAMPLES

Methods

A soluble, multimeric from of CD40L was produced by fusing the extracellular domain of CD40L to the body of two proteins, pulmonary surfactant protein D (SP-D, 4 trimers) or ACRP 30 (2 trimers). In mice, DNA vaccination with a plasmid encoding an HIV antigen was significantly augmented by co-injecting pSP-D-CD40L, pACRP-30-CD40L, pSP-D-GITRL, but not by single trimer pTrCD40L or full length platform CD40L. To test for antitumor activity, 50 mg of DNA was injected into either B16-F10 melanoma or A20 lymphoma. The growth of B16-F10 was significantly slowed by peritumoral injections of pSP-D-CD40L. More dramatic effects were seen with A20 tumors. This method for producing soluble, multimeric members of the TNF superfamily (TNFSF) enhances the feasibility of using CD40L, GITRL, and other TNFSF ligands as effective treatments for established tumors.

The present invention observed effects of direct peritumoral injection of the present constructs without antigen, as tumors are known to already contain dendritic cells that present tumor antigens. However, these DCs are suppressed by the local tumor environment and therefore, require some form of immunostimulation in order to activate CD8+ antitumor immunity.

In order to enhance the antitumor effects of soluble multimeric CD40L, tumors were injected with adjuvant plasmid along with a number of TLR agonists. The results indicate that stimulation of TLR 9 or TLR 3 can synergize with CD40 stimulation to produce significant antitumor effects in vivo.

Example 1. Plasmid Construction.

Plasmids were constructed in the pcDNA3.1 expression vector (Invitrogen, Carlsbad, CA). Membrane CD40L (p-Mem-CD40L), the full-length natural form of murine CD40L, was cloned by RT-PCR from antiCD3/anti -CD 26-stimulated murine spleen cells.

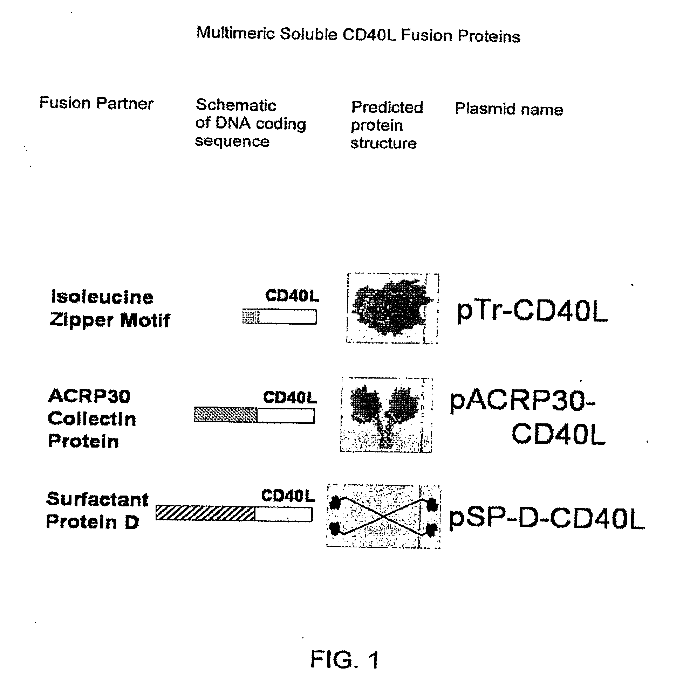

1-trimer soluble CD40L (pTr-CD40L) was constructed using PCR resulting in an isoleucine zipper fused to the extracellular domain of murine CD40L (including the stalk). The construct was subsequently cloned into the pcDNA3.1 expression vector (Figure 1).

For the 2-trimer soluble CD40L (pAcrp30-CD40L), the body of murine Acrp30 was fused to the extracellular domain of murine CD40L. Acrp30 is a V-shaped molecule with two trimeric arms that can present two trimeric TNFSF extracellular domains (Figure 1).

For the 4-trimer soluble CD40L, and GITRL (pSP-D-CD40L and pSP-D-GITRL), the body of murine surfactant protein D (SP-D) was fused to the extracellular domains of murine CD40L or GITRL (including their stalks). SP-D is a plus-sign shaped molecule with four trimeric arms that can present four trimeric TNFSF extracellular domains (Figure 1).

Plasmids were also constructed using the pVAX1 expression vector (Invitrogen), specifically plasmids that express murine SP-D-CD40L or SP-D-GITRL.

Further, plasmids were constructed using the pVAX1 expression vector that express macaque SP-D-CD40L, ACRP30-CD40L, or SP-D-GITRL.

As an improvement upon the previous pSP-D-CD40L plasmid in the pcDNA3.1 vector, the insert was transferred to pCAGEN (AddGene, Inc., Cambridge, MA). pCAGEN is a derivative of pCAGGS, which contains elements of the chicken beta-actin promoter that has been found to increase expression in a variety of tissues (H. Niwa et al, Efficient selection for high-expression transfectants with a novel eukaryotic vector, Gene 108:193-199, 1991). As a further modification, the signal sequence was changed from that of surfactant protein-D (SP-D) to that of human tissue plasminogen activator (GenBank Acc. Nos. P00750 and NP_127509). In addition, it has been noted that CD40L can be cleaved by proteinases in its extracellular stalk region Pietravalle, F. et al, Eur. J. Immunol. 26:725-728, 1996). To avoid this cleavage, the stalk region of murine CD40L was deleted. The final version is termed pSP-D-CD40L-NST (where NST refers to No Stalk/tPA signal sequence). Upon transfecting this plasmid into 293T cells in vitro, about 8 times more CD40L protein was detected by ELISA than with the pSP-D-CD40L constructs described above.

Toxicity

Mice appeared normal throughout the vaccination experiments. The histology of the i.m. injection sites 48 hours after vaccination with pSP-D-CD40L in the pcDNA3.1 vector showed no inflammation, and lung histology was normal at the conclusion of the experiments. Unlike may other adjuvants, spleen size and cell numbers were not increased by pSP-D-CD40L. Furthermore, when the pScGag antigen plasmid and the pSP-D-CD40L adjuvant plasmid were not mixed but instead were injected separately into opposite quadriceps, there were no vaccine responses, indicating that pSP-D-CD40L (unlike TLR agonists) does not induce systemic immune activation. After tumor injection, mice appeared normal and showed no signs of autoimmune response, including loss of pigment.

HIV-1 Gag Vaccine Methods

Vaccine plasmids

A plasmid for secreted, codon-optimized HIV GAG (pScGag) was used as the test antigen. Similar results were obtained with an HIV envelope plasmid and a plasmid for the MSP-1 protein of Plasmodium yoelii.

Mouse vaccinations

BALB/c mice were injected i.m. in both quadriceps every other week X 3 with a combination of pScGag (80 µg) plus CD40L plasmid or empty control vector (20 µg) suspended in phosphate-buffered saline at a total DNA concentration of 1 ug/ul. Fifty µl were injected into each quadriceps muscle using a 28G insulin syringe. The mice were sedated with isofluorane gas anesthesia to prevent them from moving during the brief injection.

Immunoassays

Two weeks after the last vaccination, mice were euthanized. Splenocyte CD8+ T cell activity was determined by CTL activity using peptide-pulsed P815 cells. Splenic CTLs were measured after restimulation for 5 days and tested for killing of P815 targets pulsed with the H-2Kd immunodominant peptide, AMQMLKETI (SEQ ID NO:6).

Example 2. Soluble, Multimeric CD40L and GIRTL as DNA Vaccine Adjuvants.

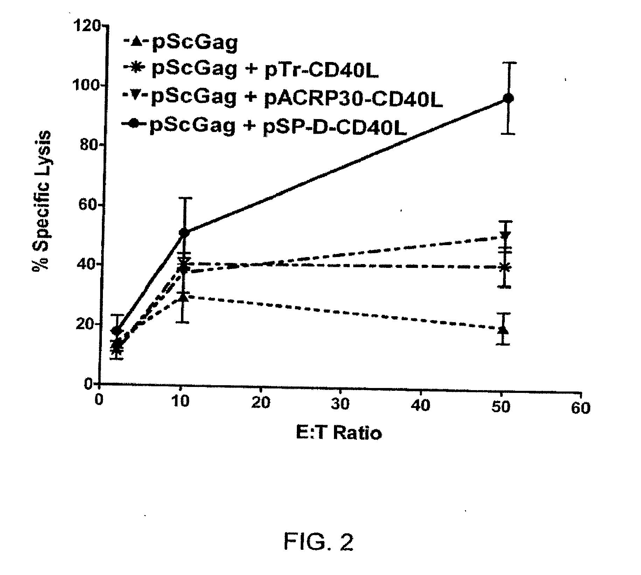

The 4-trimer soluble CD40L dramatically enhances the cytotoxic CD8+ T cell responses to a DNA vaccine (Figure 2). As found by others, the addition of a plasmid for full-length membrane CD40L (pMemCD40L) had no enhancing effects. In contrast, a plasmid for 4-trimer soluble CD40L (pSP-D-CD40L) dramatically increased the CTL response to Gag compared to 2-trimer and 1-trimer forms.

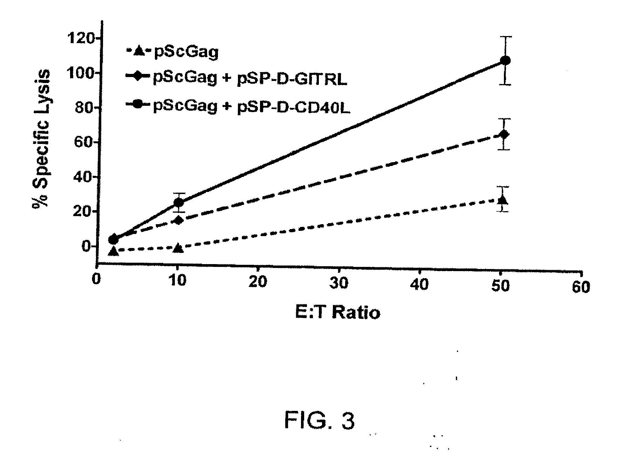

The addition of a 4-trimer soluble GITRL to DNA vaccination significantly increased CTLs (Figure 3). However, these CD8+ T cell responses were not as strong as those produced using 4-trimer CD40L as a molecular adjuvant.

Example 3. Tumor Immunotherapy Methods.

BALB/c mice were injected with 1 X 10⁶ A 20 tumor cells s.c. and C57B5/6 mice were injected with 1 X10⁶ B16-F10 tumor cells s.c. After a palpable tumor appeared (> 4mm), 50 µl PBS or plasmid DNA (50 µg) with or without TLR agonist molecules was injected into and around the tumors every other day X 5. Tumors were measured every other day. Mice were sacrificed when they showed signs of stress or tumors became larger than 1.5 cm X 1.5 cm.

Cure of an established A20 lymphoma tumor bv peri-tumoral plasmid injections.

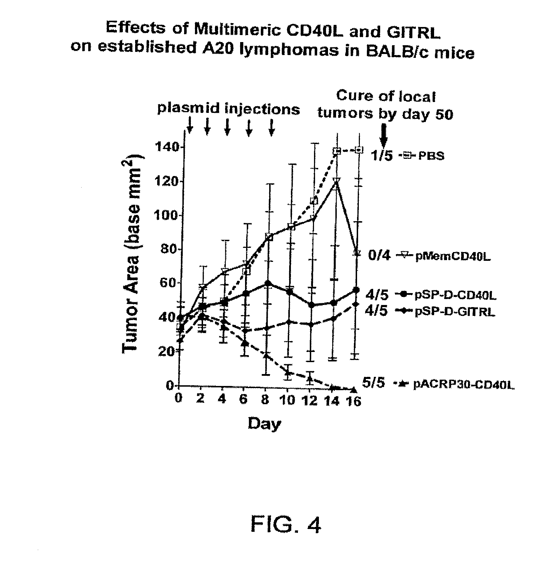

For A20 lymphoma tumors, plasmids for non-secreted membrane CD40L (pMemCD40L), 2-trimer soluble CD40L (pAcrp30-CD40L), 4-trimer soluble CD40L (pSP-D-CD40L) and 4-trimer soluble GITRL (pSP-D-GITRL) were injected. All of these plasmids used the pcDNA3.1 vector. All three soluble multimeric TNFSF plasmids had anti-tumor activity, and in 4/5 cases were able to cure mice of local tumors (Figure 4). The plasmid for membrane CD40L (pMemCD40L) was inactive (Figure 4).

Survival benefits of multimeric TNFSF ligands for A20 lymphoma.

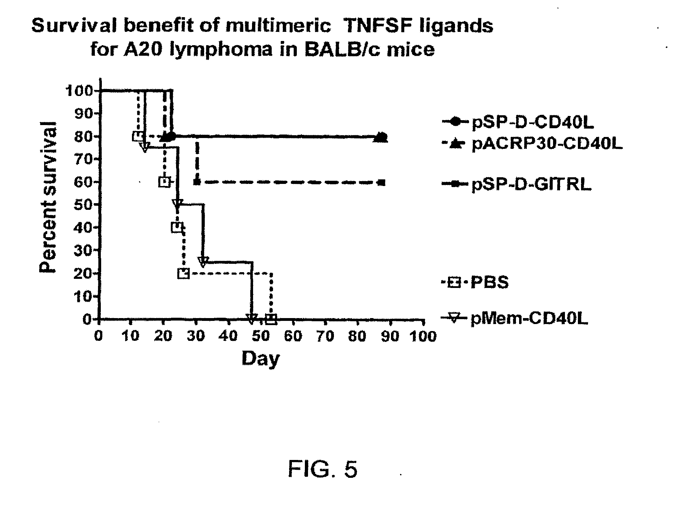

Both pAcrp30-CD40L and pSP-D-CD40L were able to cure 80 % of mice (p<0.05). pSP-D-GITRL was able to cure 80% of mice (Figure 5). Cure was defined as tumor-free survival 90 days after treatment.

TLR 3 stimulation (Poly I:C) synergized with CD40L against established B16-F10 tumors.

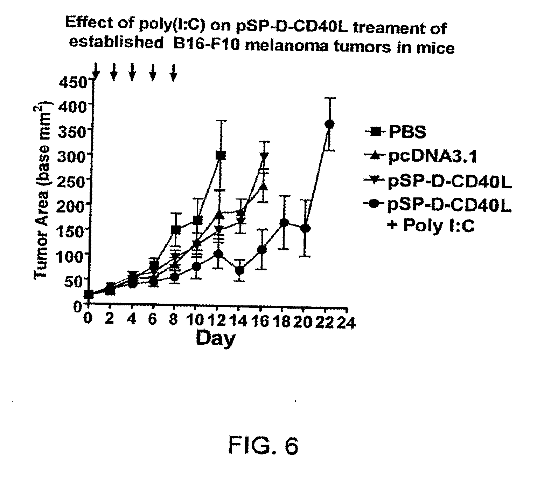

Combining poly (I:C) and pSP-D-CD40L led to a significant antitumor effect (p<0.05) compared to either control plasmid or pSP-D-CD40-L alone (Figure 6). In this example, pSP-D-CD40L (50 ug in 50 ul PBS) was injected peritumorally on days 0, 2, 4, 6, and 8, and poly(I:C) (25 ug in 50 ul PBS) was injected on days 1, 3, 5, 7, and 9.

Two trimer pAcrp30-CD40L induces strong anti-tumor immunity.

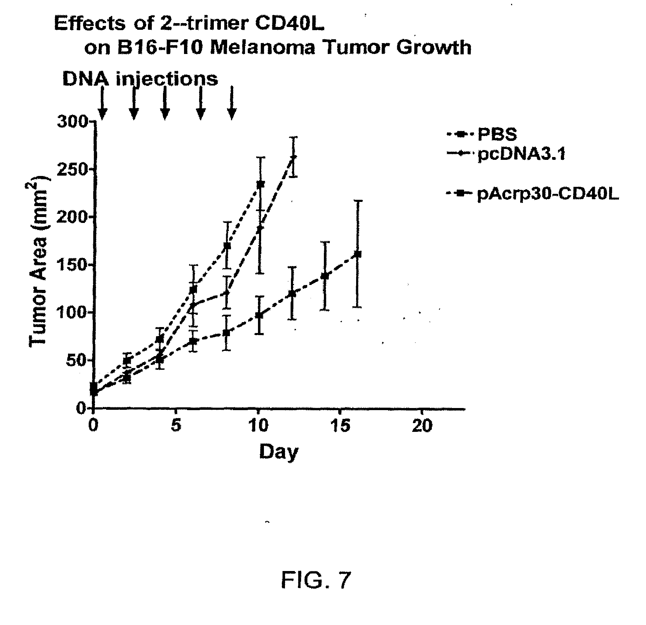

After noting strong anti-tumor effects of pAcrp30-CD-40L in the A20 lymphoma, we examined its effect on a B16-F10 tumor. While there was a significant reduction (p<0.05) in local tumor size (Figure 7), survival was not significantly enhanced.

TLR 9 stimulation (CpG) synergizes with CD40L, against established B16-F10 tumors.

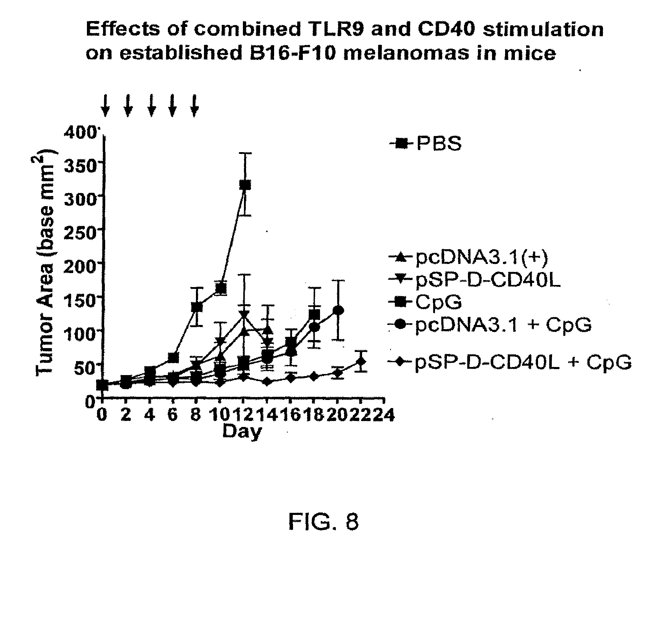

The pSP-D-CD40L plasmid was unable to significantly alter tumor progression (Figure 8). However, the combination of CpG and pSP-D-CD40L had significant anti-tumor activity (p<0.05). In this example, pSP-D-CD40L (50 ug in 50 ul PBS) was injected on days 0, 2,4,6, and 8, and CpG-ODN 1018(25 ug in 50 ul PBS) was injected peritumorally on days 1, 3, 5, 7, and 9.

CD40 stimulation synergizes with TLR 9 against established B16-F10 tumors.

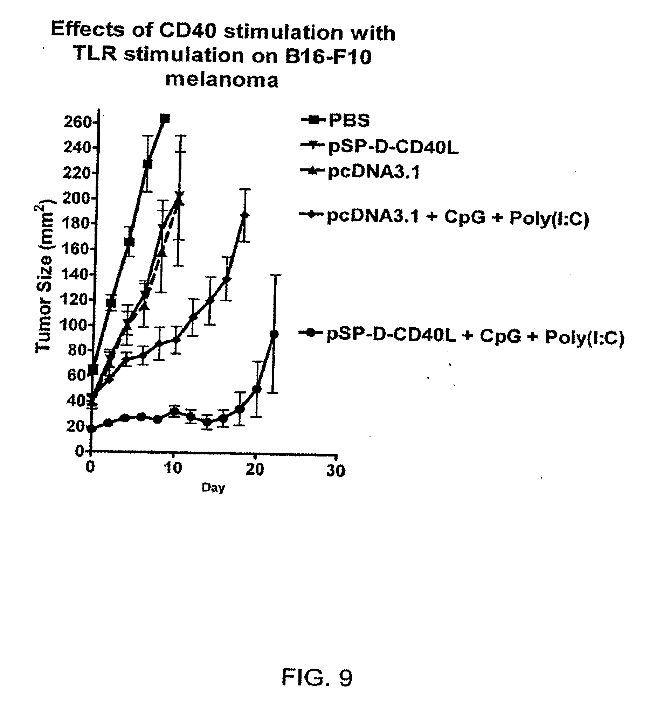

In order to control for the effect of CpG alone and CpG with empty plasmid, a second experiment was performed. Again the combination of pSP-D-CD40L and CpG had significant antitumor activity(p>0.05) compared with both CpG alone and pcDNA3.1 combined with CpG (Figure 9).

### TLR Agonists Synergize with CD40L Adjuvant.

Only TLR 3 and TLR 9 synergize with CD40 stimulation for anti-tumor effects on established B16-F10 tumors (Table 1).

**Table 1. TLR Agonist Effects with CD40 Stimulation**

| **TLR** | **TLR AGONIST** | **EFFECT** |
|---|---|---|
| TLR 1/2 | Pam3CSK4 | - |
| TLR 2/6 | FSL1 | - |
| TLR 2/6 | MALP2 | - |
| TLR 3 | Poly I: C | + |
| TLR 4 | MPL | - |
| TLR 7/8 | Imiquimod | - |
| TLR 9 | CpG 1018 | +++ |

### Example 4. Tumor Immunotherapy Methods Using Soluble, Multimeric CD40L And TLR Agonists In Combination With A Cationic Polymer.

C57B5/6 mice were injected with 1 X10⁶ B16-F10 tumor cells s.c. After a palpable tumor appeared (> 4mm), 50 µl PBS or plasmid DNA (50 µg) with or without TLR agonist, in the presence or absence of cationic polymer (JetPEI™ Qbiogene Irvine, CA) molecules, was injected into and around the tumors every other day X 5. Tumors were measured every other day. Mice were sacrificed when they showed signs of stress or tumors became larger than 1.5 cm X 1.5 cm.

Anti-tumor effect of TNFSF plasmid/TLR agonists in combination with a cationic polymer.

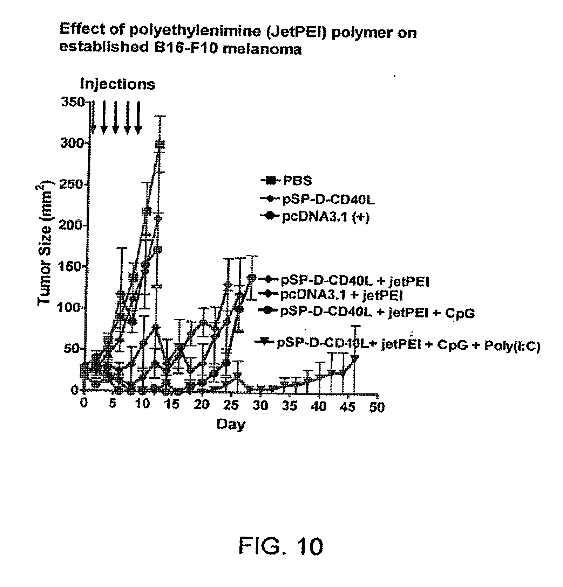

As expected, soluble multimeric TNFSF plasmids in combination with the TLR agonists had anti-tumor activity (Figure 10). However, addition of the cationic polymer to the TNFSF-plasmid/TLR agonists combination showed strikingly enhanced activity (Figure 10).

Survival benefits of TNFSF plasmid/TLR agonists-polymer combination for B16-F10 tumors.

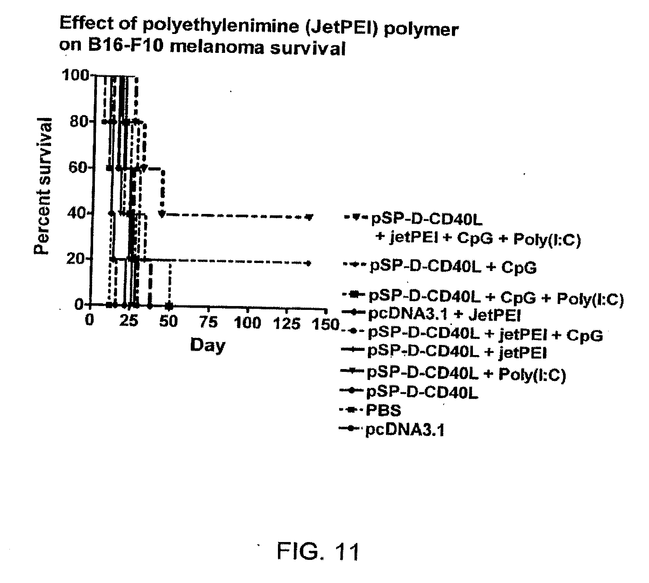

The pSP-D-CD40L + CpG + poly-I:C + polymer combination was able to cure 80 % of mice (p<0.05)(Figure 11). Cure was defined as tumor-free survival 90 days after treatment.

**Example 5: Soluble, multimeric CD40L can be produced as a fusion protein with surfactant protein D**.

CD40L, like many TNFSF ligands, must be presented as a multimer (many trimers) in order to stimulate receptor-bearing cells. To make a single trimer form of CD40L, an isoleucine zipper was used was genetically fused to the extracellular domain. To make multimeric soluble TNFSF ligands, the extracellular domain of the TNFSF ligand was genetically fused to the body of surfactant protein D (SP-D) or Acrp30 (adiponectin), two naturally multimeric proteins in the collectin and C1q families respectively (Fig. 12).

**Example 6: The valence of CD40L trimers is directly related to its adjuvanticity in a DNA vaccine**.

Three forms of soluble CD40L were produced, as shown in Fig. 12: 1-trimer CD40L containing an isoleucine zipper (pTr-CD40L); 2-trimer CD40L produced as a fusion protein with the body of Acrp30 (pAcrp30-CD40L); and 4-trimer CD40L produced as a fusion protein with the body of surfactant protein D (pSP-D-CD40L).

Methods: Plasmids were propagated in E. coli strains XL1 blue or TOP10. Supercoiled plasmid DNA was isolated by anion-exchange chromatography resin (EndoFree Plasmid MaxiKit, QIAgen, Inc, Valencia, CA). After initial experiments indicated that control vector isolated by this method still retained immunostimulatory activity compared to buffer alone, subsequent plasmid isolations were further purified by Triton X-114 extraction. Briefly, Triton X-114 (Sigma) was pre-equilibrated by adding 10 volumes TE buffer followed by a 6 hour incubation at 4°C, incubation overnight at 37°C, then removal of the upper (aqueous) phase and any turbid material at the interface. This procedure was repeated for a total of three extractions, and the detergent stored at 4°C. After completing the EndoFree kit purification protocol, plasmid DNA was suspended in TE Buffer at a concentration of 0.8 mg/ml. Sodium acetate, pH 5.2 was added to a final concentration of 0.3 M. A total of 0.03 volumes of pre-equilibrated Triton X-114 was added and the sample vortexed thoroughly. After 15 minute incubation on ice, the sample was heated to 37 C for 10 minutes to allow the two phases to separate, followed by centrifugation at 400 x g for 2 minutes at room temperature or above. The aqueous upper phase was then transferred to a new tube and an additional volume of Triton X-114 was added for a total of three extractions. Plasmid DNA was recovered by the addition of 0.7 volumes of room temperature isopropanol and centrifugation at maximum speed for 10 minutes. The pellet was washed with cold 70% ethanol (endotoxin free) then dried and resuspended in endotoxin-free 10 mM Tris-HCl/l mM EDTA, pH 7.5 TE buffer at a concentration of 5-7 mg/ml. Prior to use, the DNA was diluted in phosphate-buffered saline As an antigen plasmid, a secreted form of HIV-1 Gag (pScGag) was used. Each immunization consisted of 100 µl of phosphate-buffered saline containing 80 µg of antigen plasmid pScGag plus 20 ug of one of the CD40L or GITRL plasmids. As controls, some mice were either not immunized ("naive") or immunized with 80 ug of antigen plasmid without an adjuvant plasmid (which was replaced in this instance with 20 ug of the empty expression vector, pcDNA3.1, as filler DNA). Female BALB/cByJ mice, 6- to 9-weeks old (The Jackson Laboratory, Bar Harbor, ME) were studied in groups of five. Three immunizations were performed under isofluorane anesthesia at 2-week intervals by injecting 50 ul of plasmid DNA solution into the quadriceps of each hind limb (100 ug DNA total) using an insulin syringe with a 28 gauge needle. Two weeks following the last vaccination, the mice were euthanized with pentobarbital and spleen cells were collected. Assays for cytotoxic activity against Gag peptide-pulsed P815 cells and overnight ELISPOT assays for the production of interferon-gamma were performed by standard methods as described.

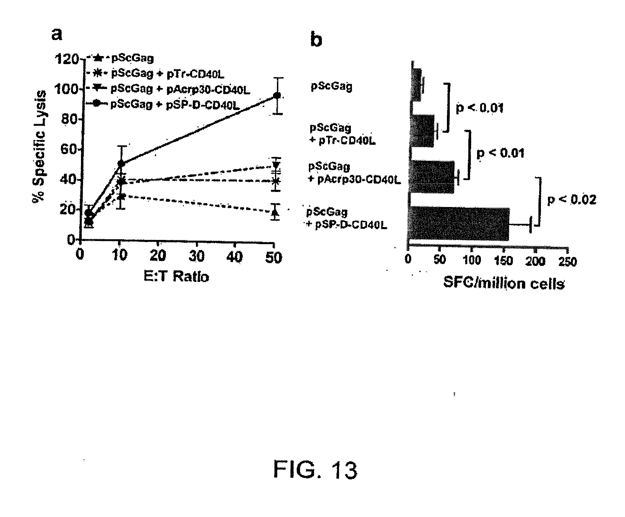

Results: When combined with an antigen plasmid, pScGag, the adjuvanticity of CD40L was directly related to the number of trimers (4 > 2 > 1) as shown in Fig. 13. For this reason, the 4-trimer and 2-trimer forms of TNFRSF ligands are preferred, especially the 4-trimer form produced as a fusion protein with SP-D.

**Example 7: Combined TNFRSF agonist and TLR agonist in a DNA vaccine for malaria.**

Methods: Plasmid DNA was prepared as above using either pcDNA3.1 (empty plasmid), pSP-D-CD40L, pSP-D-GITRL (a 4-trimer form of soluble GITR ligand), and pMSP-1 (a secreted, codon optimized form of merozoite surface protein-1 from *Plasmodium yoelii*) BALB /cByJ mice were vaccinated as before every two weeks X 3 intramuscularly with a total of 80 ug of pMSP-1 antigen plasmid and 20 ug of either pcDNA3.1 or pSP-D-GITRL. In addition, 25 ug of ISS-ODN was mixed with the injections in certain groups so that both plasmid DNA and ISS-ODN were present in the same injection. The specific ISS-ODN used was a phosphothioate version of 5'-TGACTGTGAACGTTCGAGATGA-3' (SEQ ID NO:5), also called ODN 1018, a B Class ISS-ODN. Two weeks after the last vaccination, the mice were challenged with an intraperitoneal injection of 2 X 10E4 *P. yoelii* 17XL-parasitized red blood cells. Mice were followed daily and euthanized when they appeared moribundi. Survival was plotted as a Kaplan-Meier graph.

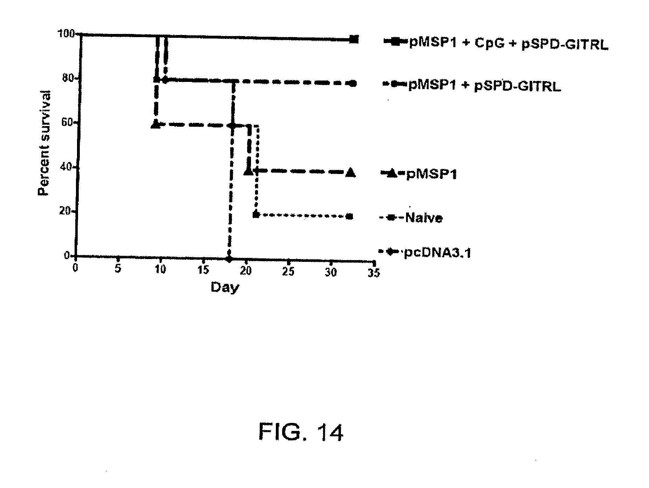

Results: As shown in Fig. 14, the combination of pSP-D-GITRL with ISS-ODN (labeled as 'CpG') protected 100% of mice from death due to malaria.

**Example 8: Combined TNFRSF agonist and two TLR agonists to treat mesothelioma tumors**.

Methods: AB1 murine mesothelioma cells were cultured in RPMI 1640 with 10% fetal bovine serum. Cells were detached with trypsin-EDTA and washed in phosphate-buffered saline (PBS). Then, 10E4 cells in 100 ul PBS were injected subcutaneously over the abdomen of female BALB/cByJ mice, 6- to 9-weeks old (Jackson Laboratories). Once tumors ≥ 4 mm in diameter had formed, the tumors were injected with 50 µl of phosphate-buffered saline containing 50 ug of pSP-D-CD40L with or without 25 ug Poly(I:C) and/or 25 ug CpG ODN 1018 every other day X 5 using an insulin syringe with a 28G needle.

Results: As shown in Fig. 15, the combination of one TNFRSF agonist (i.e., pSP-D-CD40L) combined with two TLR agonists (i.e., CpG or ISS-ODN plus Poly(I:C)) had superior antitumor effects compared to pSP-D-CD40L alone, which had no effects by itself against this tumor.

**Example 9: Combined TNFRSF agonist and two TLR agonists to treat melanoma tumors.**

Methods: B16-F10 melanoma cells were cultured in RPMI 1640 with 10% fetal bovine serum. Cells were detached with trypsin-EDTA and washed in phosphate-buffered saline (PBS). Then, 10E5 cells in 100 ul PBS were injected subcutaneously over the abdomen of female C57BL/6 female mice, 6- to 9-weeks old (Jackson Laboratories). Once tumors ≥ 4 mm in diameter had formed, the tumors were injected with 50 µl of phosphate-buffered saline containing 50 ug of pSP-D-CD40L with or without 25 ug Poly(I:C) and/or 25 ug CpG-ODN 1018 every other day X 5 using an insulin syringe with a 28G needle.

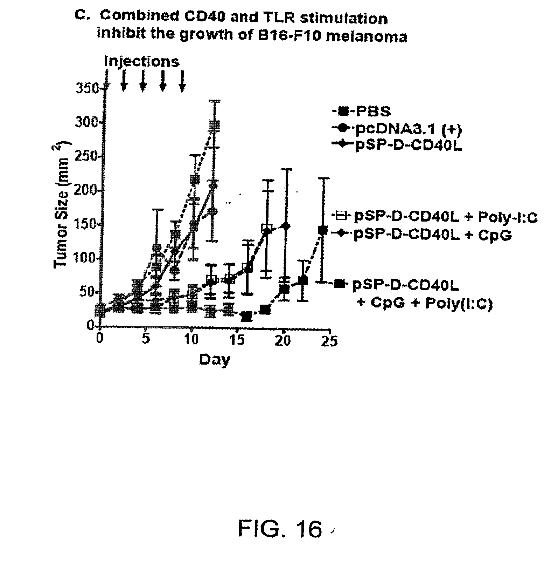

Results: As shown in Fig. 16, the combination of one TNFRSF agonist (i.e., pSP-D-CD40L) combined with two TLR agonists (i.e., CpG or ISS-ODN plus Poly(I:C)) had superior antitumor effects compared to pSP-D-CD40L alone, which had no effects by itself against this tumor.

**Example 10: Combined TNFRSF agonist, two TLR agonists, and polymer delivery to treat melanoma tumors.**

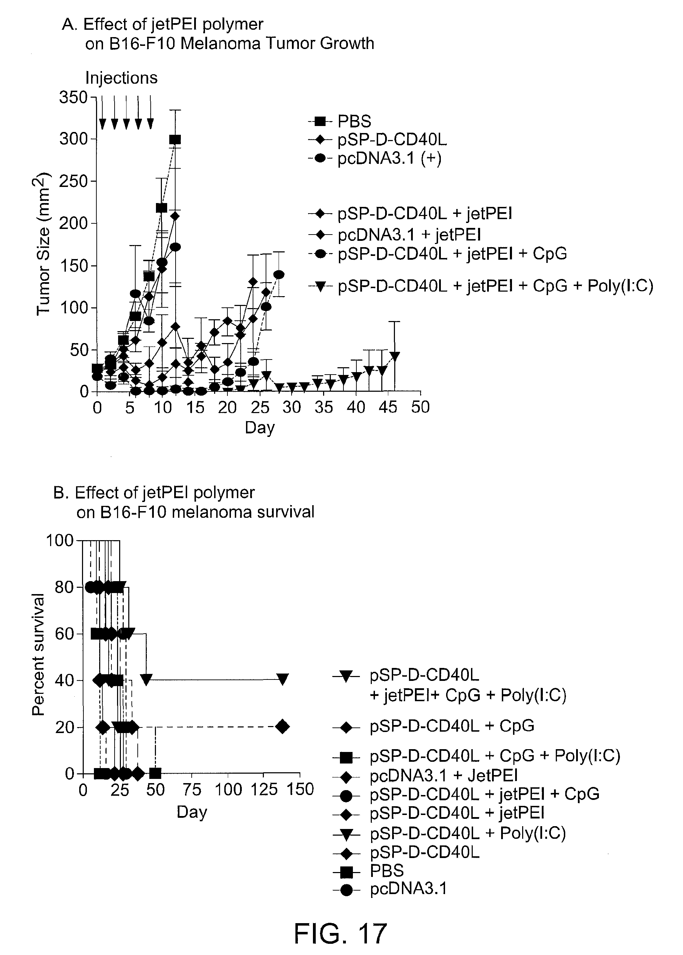

Methods: B16-F10 melanoma cells were cultured in RPMI 1640 with 10% fetal bovine serum. Cells were detached with trypsin-EDTA and washed in phosphate-buffered saline (PBS). Then, 10E5 B16-F10 cells in 100 ul PBS were injected subcutaneously over the abdomen of female C57BL/6 female mice, 6-to 9-weeks old (Jackson Laboratories). Once tumors ≥ 4 mm in diameter had formed, the tumors were injected with 50 ug of pcDNA3.1 or pSP-D-CD40L, with or without polyethylenimine (JetPEI, QBioGene, Inc.). To make the DNA/PEI mix, 50 ug of plasmid DNA was diluted with 5% glucose to a volume of 50 ul. In a separate tube, 10 ul of jetPEI was mixed with 5% glucose to a volume of 50 ul. Each tube was vortexed, and then the two tubes were combined and allowed to incubate at room temperature for 15 minutes. Mice were given peritumoral injections of 50 ug of either plain pcDNA3.1 or pSP-D-CD40L in 50 ul PBS every other day X 5 (days 0, 2, 3, 6, and 8), where some of the mice receive the DNA/JetPEI mix. Because CpG ODN precipitated in the DNA/JetPEI mix, solutions of CpG ODN 1018 and/or Poly(I:C) 25 ug each in 50 ul PBS were injected peritumorally on the days between DNA ± JetPEI injections (days 1, 3, 5, 7, and 9). Results: As shown in Fig. 17, the combination of one TNFRSF agonist (i.e., pSP-D-CD40L) using polymer delivery with JetPEI combined with two TLR agonists (i.e., CpG or ISS-ODN plus Poly(I:C)) had superior antitumor effects.

**Example 11: Combined TNFRSF agonist and cytokine/chemokine receptor agonist to treat melanoma tumors**.

Methods: As in Example 10, B16-F10 melanoma tumors were established in C57BL/6 mice. Once tumors ≥ 4 mm in diameter had formed, the tumors were injected with 50 ul of PBS alone or PBS containing 50 ug of pcDNA3.1, pSP-CD40L-NST, or pSP-D-CD40L-NST combined with 25 ug of a plasmid for murine MTP3alpha (CCL20, pMIP3alpha; GenBank Accession No. AF099052) on days 0, 2, 4, 6, and 8. Selected mice were also injected with 50 µl PBS containing 25 ug ISS-ODN (CpG 1018) and 25 ug poly(I:C) on days 1, 3, 5, 7, and 9.

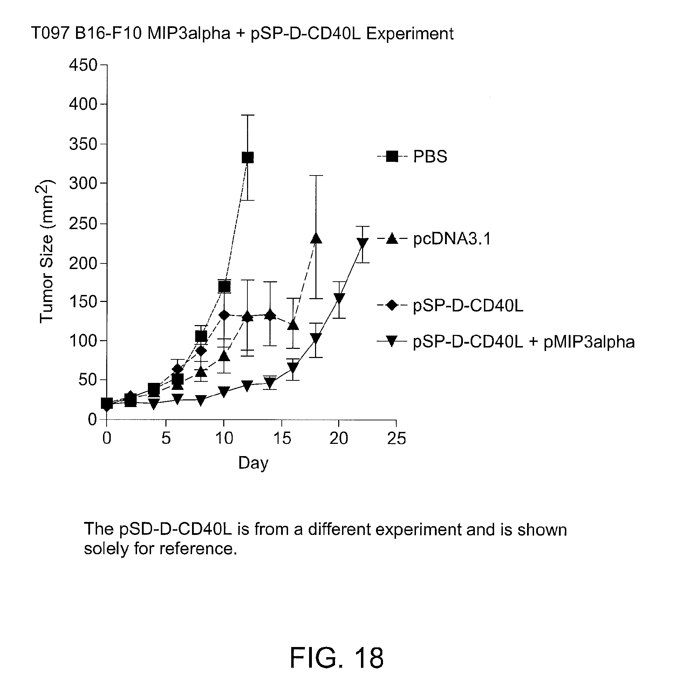

Results: The expectation was that MIP3alpha would attract dendritic cells to the site of the tumor and its draining lymph nodes and the pSP-D-CD40L would stimulate these dendritic cells. As shown in the Kaplan-Meier plot in Fig. 18, there was a survival benefit to the pSP-D CD40L-NST/pMIO-3alpha + CpG/poly)I:C) combination vs. the same treatment without pMIP-3alpha.

**EXAMPLE 12: Combination DNA vaccine against Env in mice**.

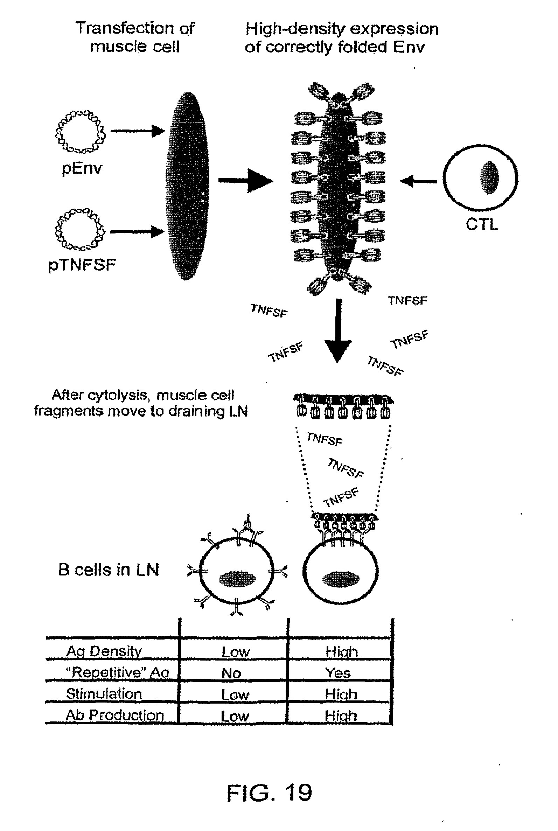

DNA vaccines vary in their ability to elicit antibody responses, for example it is known that HBsAg DNA vaccines need to contain strong CTL epitope in order to elicit a sufficient humoral response. It has been observed previously that CTLs can augment antibody response to a DNA vaccine by 1,000 fold. Under specific conditions, CTLs and Abs could be directed against different proteins so long as both antigens were present in the same vaccination. While not being bound by theory, it was hypothesized that CTLs act to liberate cell-bound Ags at the vaccine site so that the Ags can move to B cells in the draining lymph node (See FIG. 19).

Again, while not being bound by theory, it seems that repetitive Ags generate higher antibody titers than unordered Ags. Further, data suggests that high-density Ags complex with more B cell receptors and generate stronger activating signals than low-density Ags. This suggests that CTL-generated membrane fragments from high-density Env-expressing transfected muscle cells may be way to deliver Env to B cells. In one aspect, cells transfected with Env plasmid (pEnv, a plasmid encoding a codon-optimized form of the gp160 protein from subtype C strain 96ZM651 in pcDNA3.1 using the DC5 signal sequence, p96ZM651gp160-CD5-opt) are used to generate properly folded Env immunogen and to elicit neutralizing antibody response.

In order to determine if CTLs will augment Ab response to an HIV vaccine, BALB/c mice were first vaccinated against Gag using plasmids for secreted Gag (pScGag) plus 4-trimer soluble CD40L (pSP-D-CD40L). Vaccination with pScGag+ pSP-D-CD40L intramuscularly (i.m.) every two weeks X 3 generates strong CTL responses against Gag. Other mice were left unvaccinated to serve as controls without anti-Gag CTLs.

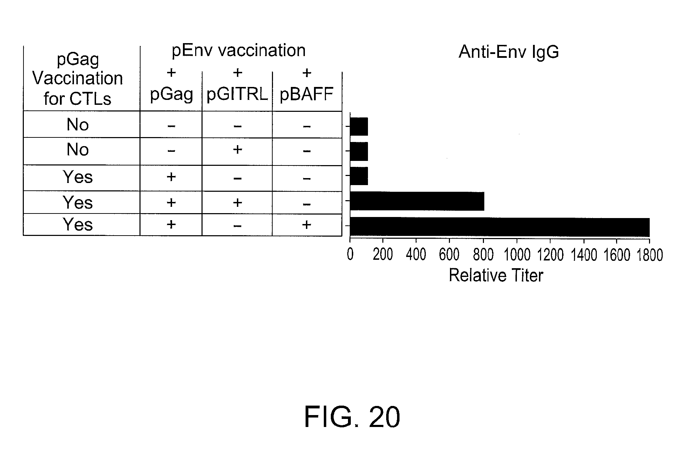

Results: Two weeks after the last Gag vaccination, the mice were vaccinated once with a plasmid for Env (pEnv, described above) with or without a plasmid for non-secreted Gag (p96ZM651 gag-opt, abbreviated pGag, also subtype C, but mutated to contain the AMQMLKETI sequence (SEQ ID NO:6) that is the immunodominant MHC-I epitope in BALB/c mice). Also, some vaccinations with pEnv ± pGag DNA included either 4-trimer soluble GITRL (pSP-D-GIRTL) or BAFF (pSP-D-BAFF). One week after this single Env plasmid vaccination, venous blood was collected and anti-Env IgG was determined by ELISA. The graph shows the mean titer of 5 mice per group (Figure 20).

**Example 13: Extracellular ATP (ATPe) as a component of an immunostimulatory combination.**

ATPγS, a nonhydrolyzable form of ATP was added to various agonists (pSP-D-CD40L + CpG + poly(I:C) + ATPe) and the combination was injected intratumorally in to mice presenting established B16F10 melanomas. Tumors were formed by injecting B16F10 tumor cells s.c. in C57BL/6 mice as before. When tumors were observed to be ≥ 4 mm in mean diameter, tumors were injected on days 0, 2, 4, 6, and 8, with various agonists (listed above) in combination with or without ATPγS. Treatments were limited to five injections into or around the tumors every other day. Tumor size was measured every other day and plotted as the product of two orthogonal diameters (i.e., "tumor area"). If a tumor disappeared, then the mouse was considered tumor-free so long as the tumor never recurred during the 44 days of observation nor the mouse died of a distant tumor.

Results: Figure 21 shows 9 experimental arms where the effects of various immunostimulatory combinations on established tumors is demonstrated. Top Panel: Treatment with the quadruple combination (i.e., pSP-D-CD40L-NST + CpG + poly(I:C) + ATPe) showed a decrease in tumor size by day 20, whereas the tumors grew in all of the other treatment arms. Middle Panel: Kaplan-Meier survival plot showing that the quadruple combination showed a marked survival benefit over the other experimental conditions. By the log-rank test, the quadruple combination that included pSP-D-CDL-NST was significantly better than the same combination except substituting pcDNA3.1 for pSP-DCD40L-NST in this combination, p=0.03. Bottom Panel: Percent tumor free mice was increased only in the quadruple combination group that included both pSP-D-CD40L-NST and ATPγS (ATPe).

**OTHER IMMUNOSTIMULATORY COMBINATIONS**

Beyond these examples, the following include other immunostimulatory combinations.
**Condition #1: One TNFRSF agonist.** CD40
   agonist (e.g., pSP-D-CD40L
   GITR agonist (e.g., pSP-D-GITRL)
   RANKL agonist (e.g., pSP-D-RANKL)
   CD27 agonist (e.g., pSP-D-CD70)
   OX40 agonist (e.g., pSP-D-OX40L)
   4-1BB agonist (e.g., pSP-D-4-1BBL) HVEM agonist (e.g., pSP-D-
   LIGHT)
**Condition #2: Two TNFRSF agonists.**
   CD40 agonist plus GITR agonist (e.g., pSP-D-CD40L plus pSP-D-GITRL)
   CD40 agonist plus RANKL agonist (e.g., pSP-D-CD40L plus pSP-D-RANKL) CD40 agonist plus CD27
   agonist (e.g., pSP-D-CD40L plus pSP-D-CD70) CD40 agonist plus OX40 agonist (e.g., pSP-D-CD40L plus pSP-D-OX40L) CD40 agonist plus 4-1BB agonist (e.g., pSP-D-CD40L plus pSP-D-4-1BBL) CD40 agonist plus HVEM agonist (e.g., pSP-D-CD40L plus pSP-D-LIGHT)
**Condition #3: One TNFRSF agonist plus one TLR agonist.**
   CD40 agonist (e.g., pSP-D-CD40L) plus TLR3 agonist (e.g. Poly(I:C))
   CD40 agonist (e.g., pSP-D-CD40L) plus TLR4 agonist (e.g., MPL)
   CD40 agonist (e.g., pSP-D-CD40L) plus TLR7 agonist (e.g., isatoribine) CD40 agonist (e.g., pSP-D-
   CD40L) plus TLR8 agonist (e.g., resiquimod) CD40 agonist (e.g., pSP-D-CD40L) plus TLR9 agonist (e.g., ISS-ODN)
   GITR agonist (e.g., pSP-D-GITRL) plus TLR3 agonist (e.g. Poly(I:C))
   GITR agonist (e.g., pSP-D-GITRL) plus TLR4 agonist (e.g., MPL)
   GITR agonist (e.g., pSP-D-GITRL) plus TLR7 agonist (e.g., isatoribine)
   GITR agonist (e.g., pSP-D-GITRL) plus TLR8 agonist (e.g., resiquimod) GITR agonist (e.g., pSP-D-GITRL) plus TLR9 agonist (e.g., ISS-ODN) RANK agonist (e.g., pSP-D-RANKL) plus TLR3 agonist (e.g. Poly(I:C)) RANK agonist (e.g., pSP-D-RANKL) plus TLR4 agonist (e.g., MPL)
   RANK agonist (e.g., pSP-D-RANKL) plus TLR7 agonist (e.g., isatoribine) RANK agonist (e.g., pSP-D-RANKL) plus TLR8 agonist (e.g., resiquimod) RANK agonist (e.g., pSP-D-RANKL) plus TLR9 agonist (e.g., ISS-ODN) OX40 agonist (e.g., pSP-D-OX40L) plus TLR3 agonist (e.g. Poly(I:C))
   OX40 agonist (e.g., pSP-D-OX40L) plus TLR4 agonist (e.g., MPL)
   OX40 agonist (e.g., pSP-D-OX40L) plus TLR7 agonist (e.g., isatoribine) OX40 agonist (e.g., pSP-D-OX40L) plus TLR8 agonist (e.g., resiquimod) OX40 agonist (e.g., pSP-D-OX40L) plus TLR9 agonist (e.g., ISS-ODN)
   4-1BB agonist (e.g., pSP-D-4-1BBL) plus TLR3 agonist (e.g. Poly(I:C))
   4-1BB agonist (e,g., pSP-D-4-1BBL) plus TLR4 agonist (e.g., MPL)
   4-1BB agonist (e.g., pSP-D-4-1BBL) plus TLR7 agonist (e.g., isatoribine)
   4-1BB agonist (e.g., pSP-D-4-1BBL) plus TLR8 agonist (e.g., resiquimod)
   4-1BB agonist (e.g., pSP-D-4-1BBL) plus TLR9 agonist (e.g., ISS-ODN) CD27 agonist (e.g., pSP-D-CD70) plus TLR3 agonist (e.g. Poly(I:C))
   CD27 agonist (e.g., pSP-D-CD70) plus TLR4 agonist (e.g., MPL)
   CD27 agonist (e.g., pSP-D-CD70) plus TLR7 agonist (e.g., isatoribine)
   CD27 agonist (e.g., pSP-D-CD70) plus TLR8 agonist (e.g., resiquimod)
   CD27 agonist (e.g., pSP-D-CD70) plus TLR9 agonist (e.g., ISS-ODN)
   HVEM agonist (e.g., pSP-D-LIGHT) plus TLR3 agonist (e.g. Poly(I:C)) HVEM agonist (e.g., pSP-D-LIGHT) plus TLR4 agonist (e.g., MPL)
   HVEM agonist (e.g., pSP-D-LIGHT) plus TLR7 agonist (e.g., isatoribine) HVEM
   agonist (e.g., pSP-D-LIGHT) plus TLR8 agonist (e.g., resiquimod) HVEM agonist
   (e.g., pSP-D-LIGHT) plus TLR9 agonist (e.g., ISS-ODN)
**Condition #4: One TNFRSF agonist plus two TLR agonists.**
   CD40 agonist (e.g., pSP-D-CD40L) plus TLR3 agonist (e.g., Poly(I:C)) plus TLR4 agonist (e.g., MPL)
   CD40 agonist (e.g., pSP-D-CD40L) plus TLR3 agonist (e.g., Poly(I:C)) plus TLR7 agonist (e.g., isatoribine)
   CD40 agonist (e.g., pSP-D-CD40L) plus TLR3 agonist (e.g., Poly(I:C)) plus TLR8 agonist (e.g., resiquimod)
   CD40 agonist (e.g., pSP-D-CD40L) plus TLR3 agonist (e.g., Poly(I:C)) plus TLR9 agonist (e.g., ISS-ODN)
   CD40 agonist (e.g., pSP-D-CD40L) plus TLR4 agonist (e.g., MPL) plus TLR7 agonist (e.g., isatoribine)
   CD40 agonist (e.g., pSP-D-CD40L) plus TLR4 agonist (e.g., MPL) plus TLR8 agonist (e.g., resiquimod)
   CD40 agonist (e.g., pSP-D-CD40L) plus TLR4 agonist (e.g., MPL) plus TLR9 agonist (e.g., ISS-ODN)
   GITR agonist (e.g., pSP-D-GITRL) plus TLR3 agonist (e.g., Poly(I:C)) plus TLR4 agonist (e.g., MPL)
   GITR agonist (e.g., pSP-D-GITRL) plus TLR3 agonist (e.g., Poly(I:C)) plus TLR7 agonist (e.g., isatoribine)
   GITR agonist (e.g., pSP-D-GITRL) plus TLR3 agonist (e.g., Poly(I:C)) plus TLR8 agonist (e.g., resiquimod)
   GITR agonist (e.g., pSP-D-GITRL) plus TLR3 agonist (e.g., Poly(I:C)) plus TLR9 agonist (e.g., ISS-ODN)
   GITR agonist (e.g., pSP-D-GITRL) plus TLR4 agonist (e.g., MPL) plus TLR7 agonist (e.g., isatoribine)
   GITR agonist (e.g., pSP-D-GITRL) plus TLR4 agonist (e.g., MPL) plus TLR8 agonist (e.g., resiquimod)
   GITR agonist (e.g., pSP-D-GITRL) plus TLR4 agonist (e.g., MPL) plus TLR9 agonist (e.g., ISS-ODN)
   RANK agonist (e.g., pSP-D-RANKL) plus TLR3 agonist (e.g., Poly(I:C)) plus TLR4 agonist (e.g., MPL)
   RANK agonist (e.g., pSP-D-RANKL) plus TLR3 agonist (e.g., Poly(I:C)) plus TLR7 agonist (e.g., isatoribine)
   RANK agonist (e.g., pSP-D-RANKL) plus TLR3 agonist (e.g., Poly(I:C)) plus TLR8 agonist (e.g., resiquimod)
   RANK agonist (e.g., pSP-D-RANKL) plus TLR3 agonist (e.g., Poly(I:C)) plus TLR9 agonist (e.g., ISS-ODN)
   RANK agonist (e.g., pSP-D-RANKL) plus TLR4 agonist (e.g., MPL) plus TLR7 agonist (e.g., isatoribine)
   RANK agonist (e.g., pSP-D-RANKL) plus TLR4 agonist (e.g., MPL) plus TLR8 agonist (e.g., resiquimod)
   RANK agonist (e.g., pSP-D-RANKL) plus TLR4 agonist (e.g., MPL) plus TLR9 agonist (e.g.,ISS-ODN)
   OX40L agonist (e.g., pSP-D-OX40L) plus TLR3 agonist (e.g., Poly(I:C)) plus TLR4 agonist (e.g., MPL)
   OX40 agonist (e.g., pSP-D-OX40L) plus TLR3 agonist (e.g., Poly(I:C)) plus TLR7 agonist (e.g., isatoribine)
   OX40 agonist (e.g., pSP-D-OX40L) plus TLR3 agonist (e.g., Poly(I:C)) plus TLR8 agonist (e.g., resiquimod)
   OX40 agonist (e.g., pSP-D-OX40L) plus TLR3 agonist (e.g., Poly(I:C)) plus TLR9 agonist (e,g., ISS-ODN)
   OX40L agonist (e.g., pSP-D-OX40L) plus TLR4 agonist (e.g., MPL) plus TLR7 agonist (e.g., isatoribine)
   OX40 agonist (e.g., pSP-D-OX40L) plus TLR4 agonist (e.g., MPL) plus TLR8 agonist (e.g., resiquimod)
   OX40 agonist (e.g., pSP-D-OX40L) plus TLR4 agonist (e.g., MPL) plus TLR9 agonist (e.g., ISS-ODN)
   4-1BB agonist (e.g., pSP-D-4-1BBL) plus TLR3 agonist (e.g., Poly(I:C)) plus TLR4 agonist (e.g., MPL)
   4-1 BB agonist (e.g., pSP-D-4-1BBL) plus TLR3 agonist (e.g., Poly(I:C)) plus TLR7 agonist (e.g., isatoribine)
   4-1BB agonist (e.g., pSP-D-4-1BBL) plus TLR3 agonist (e.g., Poly(I:C)) plus TLR8 agonist (e.g., resiquimod)
   4-1BB agonist (e.g., pSP-D-4-1BBL) plus TLR3 agonist (e.g., Poly(I;C)) plus TLR9 agonist (e.g., ISS-ODN)
   4-1BB agonist (e.g., pSP-D-4-1BBL) plus TLR4 agonist (e.g., MPL) plus TLR7 agonist (e.g., isatoribine)
   4-1BB agonist (e.g., pSP-D-4-IBBL) plus TLR4 agonist (e.g., MPL) plus TLR8 agonist (e.g., resiquimod)
   4-1BB agonist (e.g., pSP-D-4-IBBL) plus TLR4 agonist (e.g., MPL) plus TLR9 agonist (e.g., ISS-ODN)
   CD27 agonist (e.g., pSP-D-CD70) plus TLR3 agonist (e.g., Poly(I:C)) plus TLR4 agonist (e.g., MPL)
   CD27 agonist (e.g., pSP-D-CD70) plus TLR3 agonist (e.g., Poly(I:C)) plus TLR7 agonist (e.g., isatoribine)
   CD27 agonist (e.g., pSP-D-CD70) plus TLR3 agonist (e.g., Poly(I:C)) plus TLR8 agonist (e.g., resiquimod)
   CD27 agonist (e.g., pSP-D-CD70) plus TLR3 agonist (e.g., Poly(I:C)) plus TLR9 agonist (e.g., ISS-ODN)
   CD27 agonist (e.g., pSP-D-CD70) plus TLR4 agonist (e.g., MPL) plus TLR7 agonist (e.g., isatoribine)
   CD27 agonist (e.g., pSP-D-CD70) plus TLR4 agonist (e.g., MPL) plus TLR8 agonist (e.g., resiquimod)
   CD27 agonist (e.g., pSP-D-CD70) plus TLR4 agonist (e.g., MPL) plus TLR9 agonist (e.g., ISS-ODN)
   HVEM agonist (e.g., pSP-D-LIGHT) plus TLR3 agonist (e.g., Poly(I:C)) plus TLR4 agonist (e.g., MPL)
   HVEM agonist (e.g., pSP-D-LIGHT) plus TLR3 agonist (e.g., Poly(I:C)) plus TLR7 agonist (e.g., isatoribine)
   HVEM agonist (e.g., pSP-D-LIGHT) plus TLR3 agonist (e.g., Poly(I:C)) plus TLR8 agonist (e.g., resiquimod)
   HVEM agonist (e.g., pSP-D-LIGHT) plus TLR3 agonist (e.g., Poly(I:C)) plus TLR9 agonist (e.g., ISS-ODN)
   HVEM agonist (e.g., pSP-D-LIGHT) plus TLR4 agonist (e.g., MPL) plus TLR7 agonist (e.g., isatoribine)
   HVEM agonist (e.g., pSP-D-LIGHT) plus TLR4 agonist (e.g., MPL) plus TLR8 agonist (e.g., resiquimod)
   HVEM agonist (e.g., pSP-D-LIGHT) plus TLR4 agonist (e.g., MPL) plus TLR9 agonist (e.g., ISS-ODN)
**Condition #5: Two TNFRSF agonists plus one TLR agonist.**
   CD40 agonist plus GITR agonist (e.g., pSP-D-CD40L plus pSP-D-GITRL) plus TLR3 agonist (e.g., Poly(I:C))
   CD40 agonist plus GITR agonist (e.g., pSP-D-CD40L plus pSP-D-GITRL) plus TLR4 agonist (e.g., MPL)
   CD40 agonist plus GITR agonist (e.g., pSP-D-CD40L plus pSP-D-GITRL) plus TLR7 agonist (e.g., isatoribine)
   CD40 agonist plus GITR agonist (e.g., pSP-D-CD40L plus pSP-D-GITRL) plus TLR8 agonist (e.g., resiquimod)
   CD40 agonist plus GITR agonist (e.g., pSP-D-CD40L plus pSP-D-GITRL) plus TLR9 agonist (e.g., ISS-ODN)
   CD40 agonist plus RANKL (e.g., pSP-D-CD40L plus pSP-D-RANKL) plus TLR3 agonist (e.g., Poly(I:C))
   CD40 agonist plus RANKL (e.g., pSP-D-CD40L plus pSP-D-RANKL) plus TLR4 agonist (e.g., MPL)
   CD40 agonist plus RANK (e.g., pSP-D-CD40L plus pSP-D-RANKL) plus TLR7 agonist (e.g., isatoribine)
   CD40 agonist plus RANKL (e.g., pSP-D-CD40L plus pSP-D-RANKL) plus TLR8 agonist (e.g., resiquimod)
   CD40 agonist plus RANKL (e.g., pSP-D-CD40L plus pSP-D-RANKL) plus TLR9 agonist (e.g., ISS-ODN)
   CD40 agonist plus CD27 agonist (e.g., pSP-D-CD40L plus pSP-D-CD70) plus TLR3 agonist (e.g., Poly(I:C))
   CD40 agonist plus CD27 agonist (e.g., pSP-D-CD40L plus pSP-D-CD70) plus TLR4 agonist (e.g., MPL)
   CD40 agonist plus CD27 agonist (e.g., pSP-D-CD40L plus pSP-D-CD70) plus TLR7 agonist (e.g., isatoribine)
   CD40 agonist plus CD27 agonist (e.g., pSP-D-CD40L plus pSP-D-CD70) plus TLR8 agonist (e.g., resiquimod)
   CD40 agonist plus CD27 agonist (e.g., pSP-D-CD40L plus pSP-D-CD70) plus TLR9 agonist (e.g., ISS-ODN)
   CD40 agonist plus OX40 agonist (e.g., pSP-D-CD40L plus pSP-D-OX40L) plus TLR3 agonist (e.g., Poly(I:C))
   CD40 agonist plus OX40 agonist (e.g., pSP-D-CD40L plus pSP-D-OX40L) plus TLR4 agonist (e.g., MPL)
   CD40 agonist plus OX40 agonist (e.g., pSP-D-CD40L plus pSP-D-OX40L) plus TLR7 agonist (e.g., isatoribine)
   CD40 agonist plus OX40 agonist (e.g., pSP-D-CD40L plus pSP-D-OX40L) plus TLR8 agonist (e.g., resiquimod)
   CD40 agonist plus OX40 agonist (e.g., pSP-D-CD40L plus pSP-D-OX40L) plus TLR9 agonist (e.g., ISS-ODN)
   CD40 agonist plus 4-1BB agonist (e.g., pSP-D-CD40L plus pSP-D-4-1BBL) plus TLR3 agonist (e.g., Poly(I:C))
   CD40 agonist plus 4-1BB agonist (e.g., pSP-D-CD40L plus pSP-D-4-1BBL) plus TLR4 agonist (e.g., MPL)
   CD40 agonist plus 4-1BB agonist (e.g., pSP-D-CD40L plus pSP-D-4-18BL) plus TLR7 agonist (e.g., isatoribine)
   CD40 agonist plus 4-1BB agonist (e.g., pSP-D-CD40L plus pSP-D-4-1BBL) plus TLR8 agonist (e.g., resiquimod)
   CD40 agonist plus 4-1BB agonist (e.g., pSP-D-CD40L plus pSP-D-4-1BBL) plus TLR9 agonist (e.g., ISS-ODN)
   CD40 agonist plus HVEM agonist (e.g., pSP-D-CD40L plus pSP-D-LIGHT) plus TLR3 agonist (e.g., Poly(I:C))
   CD40 agonist plus HVEM agonist (e.g., pSP-D-CD40L plus pSP-D-LIGHT) plus TLR4 agonist (e.g., MPL)
   CD40 agonist plus HVEM agonist (e.g., pSP-D-CD40L plus pSP-D-LIGHT) plus TLR7 agonist (e.g., isatoribine)
   CD40 agonist plus HVEM agonist (e.g., pSP-D-CD40L plus pSP-D-LIGHT) plus TLR8 agonist (e.g., resiquimod)
   CD40 agonist plus HVEM agonist (e.g., pSP-D-CD40L plus pSP-D-LIGHT) plus TLR9 agonist (e.g., ISS-ODN)
**Condition #6: Two TNFRSF agonists plus two TLR agonists.**
   CD40 agonist plus GITR agonist (e.g., pSP-D-CD40L plus pSP-D-GITRL) plus TLR3 agonist plus TLR7 agonist ((e.g., Poly(I:C) plus isatoribine)
   CD40 agonist plus GITR agonist (e.g., pSP-D-CD40L plus pSP-D-GITRL) plus TLR3 agonist plus TLR8 agonist (e.g., Poly(I:C) plus resiquimod)
   CD40 agonist plus GITR agonist (e.g., pSP-D-CD40L plus pSP-D-GITRL) plus TLR3 agonist plus TLR9 agonist (e.g., Poly(I:C) plus ISS-ODN)
   CD40 agonist plus GITR agonist (e.g., pSP-D-CD40L plus pSP-D-GITRL) plus TLR4 agonist plus TLR7 agonist ((e.g., MPL plus isatoribine)
   CD40 agonist plus GITR agonist (e.g., pSP-D-CD40L plus pSP-D-GITRL) plus TLR4 agonist plus TLR8 agonist (e.g., MPL plus resiquimod)
   CD40 agonist plus GITR agonist (e.g., pSP-D-CD40L plus pSP-D-GITRL) plus TLR4 agonist plus TLR9 agonist (e.g., MPL plus ISS-ODN)
   CD40 agonist plus RANK agonist (e.g., pSP-D-CD40L plus pSP-D-RANKL) plus TLR3 agonist plus TLR7 agonist ((e.g., Poly(I:C) plus isatoribine)
   CD40 agonist plus RANK agonist (e.g., pSP-D-CD40L plus pSP-D-RANKL) plus TLR3 agonist plus TLR8 agonist (e.g., Poly(I:C) plus resiquimod)
   CD40 agonist plus RANK agonist (e.g., pSP-D-CD40L plus pSP-D-RANKL) plus TLR3 agonist plus TLR9 agonist (e.g., Poly(I:C) plus ISS-ODN)
   CD40 agonist plus RANK agonist (e.g., pSP-D-CD40L plus pSP-D-RANKL) plus TLR4 agonist plus TLR7 agonist ((e.g., MPL plus isatoribine)
   CD40 agonist plus RANK agonist (e.g., pSP-D-CD40L plus pSP-D-RANKL) plus TLR4 agonist plus TLR8 agonist (e.g., MPL plus resiquimod)
   CD40 agonist plus RANK agonist (e.g., pSP-D-CD40L plus pSP-D-RANKL) plus TLR4 agonist plus TLR9 agonist (e.g., MPL plus ISS-ODN)
   CD40 agonist plus CD27 agonist (e.g., pSP-D-CD40L plus pSP-D-CD70) plus TLR3 agonist plus TLR7 agonist ((e.g., Poly(I:C) plus isatoribine)
   CD40 agonist plus CD27 agonist (e.g., pSP-D-CD40L plus pSP-D-CD70) plus TLR3 agonist plus TLR8 agonist (e.g., Poly(I:C) plus resiquimod)
   CD40 agonist plus CD27 agonist (e.g., pSP-D-CD40L plus pSP-D-CD70) plus TLR3 agonist plus TLR9 agonist (e.g., Poly(I:C) plus ISS-ODN)
   CD40 agonist plus CD27 agonist (e.g., pSP-D-CD40L plus pSP-D-CD70) plus TLR4 agonist plus TLR7 agonist ((e.g., MPL plus isatoribine)
   CD40 agonist plus CD27 agonist (e.g., pSP-D-CD40L plus pSP-D-CD70) plus TLR4 agonist plus TLR8 agonist (e.g., MPL plus resiquimod)
   CD40 agonist plus CD27 agonist (e.g., pSP-D-CD40L plus pSP-D-CD70) plus TLR4 agonist plus TLR9 agonist (e.g., MPL plus ISS-ODN)
   CD40 agonist plus OX40 agonist (e.g., pSP-D-CD40L plus pSP-D-OX40L) plus TLR3 agonist plus TLR7 agonist ((e.g., Poly(I:C) plus isatoribine)
   CD40 agonist plus OX40 agonist (e.g., pSP-D-CD40L plus pSP-D-OX40L) plus TLR3 agonist plus TLR8 agonist (e.g., Poly(I:C) plus resiquimod)
   CD40 agonist plus OX40 agonist (e.g., pSP-D-CD40L plus pSP-D-OX40L) plus TLR3 agonist plus TLR9 agonist (e.g., Poly(I:C) plus ISS-ODN)
   CD40 agonist plus OX40 agonist (e,g., pSP-D-CD40L plus pSP-D-OX40L) plus TLR4 agonist plus TLR7 agonist ((e.g., MPL plus isatoribine)
   CD40 agonist plus OX40 agonist (e.g., pSP-D-CD40L plus pSP-D-OX40L) plus TLR4 agonist plus TLR8 agonist (e.g., MPL plus resiquimod)
   CD40 agonist plus OX40 agonist (e.g., pSP-D-CD40L plus pSP-D-OX40L) plus TLR4 agonist plus TLR9 agonist (e.g., MPL plus ISS-ODN)
   CD40 agonist plus 4-1BB agonist (e.g., pSP-D-CD40L plus pSP-D-4-1BBL) plus TLR3 agonist plus TLR7 agonist ((e.g., Poly(I:C) plus isatoribine)
   CD40 agonist plus 4-1BB agonist (e.g., pSP-D-CD40L plus pSP-D-4-1BBL) plus TLR3 agonist plus TLR8 agonist (e.g., Poly(I:C) plus resiquimod)
   CD40 agonist plus 4-1BB agonist (e.g., pSP-D-CD40L plus pSP-D-4-1BBL) plus TLR3 agonist plus TLR9 agonist (e.g., Poly(I:C) plus ISS-ODN)
   CD40 agonist plus 4-1BB agonist (e.g., pSP-D-CD40L plus pSP-D-4-1BBL) plus TLR4 agonist plus TLR7 agonist ((e.g., MPL plus isatoribine)
   CD40 agonist plus 4-1BB agonist (e.g., pSP-D-CD40L plus pSP-D-4-1BBL) plus TLR4 agonist plus TLR8 agonist (e.g., MPL plus resiquimod)
   CD40 agonist plus 4-1BB agonist (e.g., pSP-D-CD40L plus pSP-D-4-1BBL) plus TLR4 agonist plus TLR9 agonist (e.g., MPL plus ISS-ODN)
   CD40 agonist plus HVEM agonist (e.g., pSP-D-CD40L plus pSP-D-LIGHT) plus TLR3 agonist plus TLR7 agonist ((e.g., Poly(I:C) plus isatoribine)
   CD40 agonist plus HVEM agonist (e.g., pSP-D-CD40L plus pSP-D-LIGHT) plus TLR3 agonist plus TLR8 agonist (e.g., Poly(I:C) plus resiquimod)
   CD40 agonist plus HVEM agonist (e.g., pSP-D-CD40L plus pSP-D-LIGHT) plus TLR3 agonist plus TLR9 agonist (e.g., Poly(I:C) plus ISS-ODN)
   CD40 agonist plus HVEM agonist (e.g., pSP-D-CD40L plus pSP-D-LIGHT) plus TLR4 agonist plus TLR7 agonist ((e.g., MPL plus isatoribine)
   CD40 agonist plus HVEM agonist (e.g., pSP-D-CD40L plus pSP-D-LIGHT) plus TLR4 agonist plus TLR8 agonist (e.g., MPL plus resiquimod)
   CD40 agonist plus HVEM agonist (e.g., pSP-D-CD40L plus pSP-D-LIGHT) plus TLR4 agonist plus TLR9 agonist (e.g., MPL plus ISS-ODN)
**Condition #7: One TNFRSF agonist plus one NLR or RLH agonist.**
   CD40 agonist (e.g., pSP-D-CD40L) plusNOD1 agonist (e.g. M-TriDAP) CD40
   agonist (e.g., pSP-D-CD40L) plus NOD2 agonist (e.g., M-TriLys) CD40 agonist
   (e.g., pSP-D-CD40L) plus MDA5 agonist (e.g., dsRNA) GITR agonist (e.g., pSP-D-
   GITRL) plus NOD1 agonist (e.g. TriDAP) GITR agonist (e.g., pSP-D-GITRL) plus
   NOD2 agonist (e.g., M-TriLys) GITR agonist (e.g., pSP-D-GITRL) plus MDA5
   agonist (e.g., dsRNA) RANK agonist (e.g., pSP-D-RANKL) plus NOD1 agonist
   (e.g. TriDAP) RANK agonist (e.g., pSP-D-RANKL) plus NOD2 agonist (e.g., M-
   TriLys) RANK agonist (e.g., pSP-D-RANKL) plus MDA5 agonist (e.g., dsRNA)
   OX40 agonist (e.g., pSP-D-OX40L) plus NOD1 agonist (e.g. TriDAP) OX40 agonist
   (e.g., pSP-D-OX40L) plus NOD2 agonist (e.g., M-TriLys) OX40 agonist (e.g., pSP-
   D-OX40L) plus MDA5 agonist (e.g., dsRNA)
   4-1BB agonist (e.g., pSP-D-4-1BBL) plus NOD1 agonist (e.g. TriDAP)
   4-1BB agonist (e.g., pSP-D-4-1BBL) plus NOD2 agonist (e.g., M-TriLys) 4-1BB
   agonist (e.g., pSP-D-4-1BBL) plus MDA5 agonist (e.g., dsRNA) CD27 agonist (e.g.,
   pSP-D-CD70) plus NOD1 agonist (e.g. TriDAP) CD27 agonist (e.g., pSP-D-CD70)
   plus NOD2 agonist (e.g., M-TriLys) CD27 agonist (e.g., pSP-D-CD70) plus MDA5
   agonist (e.g., dsRNA) HVEM agonist (e.g., pSP-D-LIGHT) plus NOD1 agonist (e.g.
   TriDAP) HVEM agonist (e.g., pSP-D-LIGHT) plus NOD2 agonist (e.g., M-TriLys)
   HVEM agonist (e.g., pSP-D-LIGHT) plus MDA5 agonist (e.g., dsRNA)
**Condition #8: One TNFRSF agonist plus two NLR or RLH agonists.**
   CD40 agonist (e.g., pSP-D-CD40L) plus NOD1 + NOD2 agonist (e.g. M-TriDAP + M-TriLys) CD40 agonist (e.g., pSP-D-CD40L) plus NOD1 + MDA5 agonist (e.g., M-TriDAP + dsRNA) CD40 agonist (e.g., pSP-D-CD40L) plus NOD2 + MDA5 agonist (e.g., M-TriLys + dsRNA) GITR agonist (e.g., pSP-D-GITRL) plus NOD1 + NOD2 agonist (e.g. M-TriDAP + M-TriLys) GITR agonist (e.g., pSP-D-GITRL) plus NOD1 + MDA5 agonist (e.g., M-TriDAP + dsRNA) GITR agonist (e.g., pSP-D-GITRL) plus NOD2 + MDA5 agonist (e.g., M-TriLys + dsRNA) RANK agonist (e.g., pSP-D-RANKL) plus NOD1 + NOD2 agonist (e.g. M-TriDAP + M-TriLys)
   RANK agonist (e.g., pSP-D-RANKL) plus NOD1 + MDA5 agonist (e.g., M-TriDAP + dsRNA) RANK agonist (e.g., pSP-D-RANKL) plus NOD2 + MDA5 agonist (e.g., M-TriLys + dsRNA) OX40 agonist (e.g., pSP-D-OX40L) plus NOD1 + NOD2 agonist (e.g. M-TriDAP + M-TriLys) OX40 agonist (e.g., pSP-D-OX40L) plus NOD1 + MDA5 agonist (e.g., M-TriDAP + dsRNA)
   OX40 agonist (e.g., pSP-D-OX40L) plus NOD2 + MDA5 agonist (e.g., M-TriLys + dsRNA)
   4-1BB agonist (e.g., pSP-D-4-1BBL) plus NOD1 +NOD2 agonist (e.g. M-TriDAP + M-TriLys) 4-1BB agonist (e.g., pSP-D-4-1BBL) plus NOD1 + MDA5 agonist (e.g., M-TriDAP+ dsRNA)
   4-1BB agonist (e.g., pSP-D-4-1BBL) plus NOD2 + MDA5 agonist (e.g., M-TriLys + dsRNA) CD27 agonist (e.g., pSP-D-CD70) plus NOD1 + NOD2 agonist (e.g. M-TriDAP + M-TriLys) CD27 agonist (e.g., pSP-D-CD70) plus NOD1 + MDA5 agonist (e.g., M-TriDAP + dsRNA) CD27 agonist (e.g., pSP-D-CD70) plus NOD2 + MDA5 agonist (e.g., M-TriLys + dsRNA) HVEM agonist (e.g., pSP-D-LIGHT) plus NOD 1 + NOD2 agonist (e.g. M-TriDAP + M-TriLys) HVEM agonist (e.g., pSP-D-LIGHT) plus NOD1 + MDA5 agonist (e.g., M-TriDAP + dsRNA) HVEM agonist (e.g., pSP-D-LIGHT) plus NOD2 + MDA5 agonist (e.g., M-TriLys + dsRNA)
**Condition #9: Two TNFRSF agonists plus one NLR or RLH agonist.**
   CD40 agonist plus GITR agonist (e.g., pSP-D-CD40L plus pSP-D-GITRL) plus NOD1 agonist (e.g., M-TriDAP)
   CD40 agonist plus GITR agonist (e.g., pSP-D-CD40L plus pSP-D-GITRL) plus NOD2 agonist (e.g., M-TriLys)
   CD40 agonist plus GITR agonist (e.g., pSP-D-CD40L plus pSP-D-GITRL) plus MDA5 agonist (e.g., dsRNA)
   CD40 agonist plus RANKL (e.g., pSP-D-CD40L plus pSP-D-RANKL) plus NOD1 agonist (e.g., M-TriDAP)
   CD40 agonist plus RANKL (e.g., pSP-D-CD40L plus pSP-D-RANKL) plus NOD2 agonist (e.g., M-TriLys)
   CD40 agonist plus RANKL (e.g., pSP-D-CD40L plus pSP-D-RANKL) plus MDA5 agonist (e.g., dsRNA)
   CD40 agonist plus CD27 agonist (e.g., pSP-D-CD40L plus pSP-D-CD70) plus NOD1 agonist (e.g., M-TriDAP)
   CD40 agonist plus CD27 agonist (e.g., pSP-D-CD40L plus pSP-D-CD70) plus NOD2 agonist (e.g., M-TriLys)
   CD40 agonist plus CD27 agonist (e.g., pSP-D-CD40L plus pSP-D-CD70) plus MDA5 agonist (e.g., dsRNA)
   CD40 agonist plus OX40 agonist (e.g., pSP-D-CD40L plus pSP-D-OX40L) plus NOD1 agonist (e.g., M-TriDAP)
   CD40 agonist plus OX40 agonist (e.g., pSP-D-CD40L plus pSP-D-OX40L) plus NOD2 agonist (e.g., M-TriLys)
   CD40 agonist plus OX40 agonist (e.g., pSP-D-CD40L plus pSP-D-OX40L) plus MDA5 agonist (e.g., dsRNA)
   CD40 agonist plus 4-1BB agonist (e.g., pSP-D-CD40L plus pSP-D-4-1BBL) plus NOD1 agonist (e.g., M-TriDAP)
   CD40 agonist plus 4-1BB agonist (e.g., pSP-D-CD40L plus pSP-D-4-1BBL) plus NOD2 agonist (e.g., M-TriLys)
   CD40 agonist plus 4-1BB agonist (e.g., pSP-D-CD40L plus pSP-D-4-1BBL) plus MDA5agonist (e.g., dsRNA)
   CD40 agonist plus HVEM agonist (e.g., pSP-D-CD40L plus pSP-D-LIGHT) plus NOD1 agonist (e.g., M-TriDAP)
   CD40 agonist plus HVEM agonist (e.g., pSP-D-CD40L plus pSP-D-LIGHT) plus NOD2 agonist (e.g., M-TriLys)
   CD40 agonist plus HVEM agonist (e.g., pSP-D-CD40L plus pSP-D-LIGHT) plus MDA5 agonist (e.g., dsRNA)
**Condition #10: Two TNFRSF agonists plus two NLR or RLH agonists.**
   CD40 agonist plus GITR agonist (e.g., pSP-D-CD40L plus pSP-D-GITRL) plus NOD1 + NOD2 agonist (e.g. M-TriDAP + M-TriLys)
   CD40 agonist plus GITR agonist (e.g., pSP-D-CD40L plus pSP-D-GITRL) plus NOD1 + MDA5 agonist (e.g., M-TriDAP + dsRNA)
   CD40 agonist plus GITR agonist (e.g., pSP-D-CD40L plus pSP-D-GITRL) plus NOD2 + MDA5 agonist (e.g., M-TriLys + dsRNA)
   CD40 agonist plus RANKL (e.g., pSP-D-CD40L plus pSP-D-RANKL) plus NOD1 + NOD2 agonist (e.g. M-TriDAP + M-TriLys)
   CD40 agonist plus RANKL (e.g., pSP-D-CD40L plus pSP-D-RANKL) plus NOD1 + MDA5 agonist (e.g., M-TriDAP + dsRNA)
   CD40 agonist plus RANKL (e.g., pSP-D-CD40L plus pSP-D-RANKL) plus NOD2 + MDA5 agonist (e.g., M-TriLys + dsRNA)
   CD40 agonist plus CD27 agonist (e.g., pSP-D-CD40L plus pSP-D-CD70) plus NOD1 + NOD2 agonist (e.g. M-TriDAP + M-TriLys)
   CD40 agonist plus CD27 agonist (e.g., pSP-D-CD40L plus pSP-D-CD70) plus NOD1 + MDA5 agonist (e.g., M-TriDAP + dsRNA)
   CD40 agonist plus CD27 agonist (e.g., pSP-D-CD40L plus pSP-D-CD70) plus NOD2 + MDA5 agonist (e.g., M-TriLys + dsRNA)
   CD40 agonist plus OX40 agonist (e.g., pSP-D-CD40L plus pSP-D-OX40L) plus NOD 1 + NOD2 agonist (e.g. M-TriDAP + M-TriLys)
   CD40 agonist plus OX40 agonist (e.g., pSP-D-CD40L plus pSP-D-OX40L) plus NOD1 + MDA5 agonist (e.g., M-TriDAP + dsRNA)
   CD40 agonist plus OX40 agonist (e.g., pSP-D-CD40L plus pSP-D-OX40L) plus NOD2 + MDA5 agonist (e.g., M-TriLys + dsRNA)
   CD40 agonist plus 4-1BB agonist (e.g., pSP-D-CD40L plus pSP-D-4-1BBL) plus NOD1 + NOD2 agonist (e.g. M-TriDAP + M-TriLys)
   CD40 agonist plus 4-1BB agonist (e.g., pSP-D-CD40L plus pSP-D-4-1BBL) plus NOD1 + MDA5 agonist (e.g., M-TriDAP + dsRNA)
   CD40 agonist plus 4-1BB agonist (e.g., pSP-D-CD40L plus pSP-D-4-1BBL) plus NOD2 + MDA5 agonist (e.g., M-TriLys + dsRNA)
   CD40 agonist plus HVEM agonist (e.g., pSP-D-CD40L plus pSP-D-LIGHT) plus NOD1 + NOD2 agonist (e.g. M-TriDAP + M-TriLys)
   CD40 agonist plus HVEM agonist (e.g., pSP-D-CD40L plus pSP-D-LIGHT) plus NOD1 + MDA5 agonist (e.g., M-TriDAP + dsRNA)
   CD40 agonist plus HVEM agonist (e.g., pSP-D-CD40L plus pSP-D-LIGHT) plus NOD2 + MDA5 agonist (e.g., M-TriLys + dsRNA)
**Condition #11: One TNFRSF agonist plus one cytokine/chemokine receptor agonist.**
   The combinations of Condition #1 plus a cytokine/chemokine receptor agonist (e.g., IFN-gamma)
   The combinations of Condition #1 plus a cytokine/chemokine receptor agonist (e.g., CCL20)
**Condition #12: Two TNFRSF agonists plus one cytokine/chemokine receptor agonist.**
   The combinations of Condition #2 plus a cytokine/chemokine receptor agonist (e.g., IFN-gamma)
   The combinations of Condition #2 plus a cytokine/chemokine receptor agonist (e.g., CCL20)
**Condition #13: One TNFRSF agonist plus one TLR agonist plus one cytokine/chemokine receptor agonist.**
   The combinations of Condition #3 plus a cytokine/chemokine receptor agonist (e.g., IFN-gamma)
   The combinations of Condition #3 plus a cytokine/chemokine receptor agonist (e.g., CCL20)
**Condition #14: One TNFRSF agonist plus two TLR agonists plus one cytokine/chemokine receptor agonist.**
   The combinations of Condition #4 plus a cytokine/chemokine receptor agonist (e.g., IFN-gamma)
   The combinations of Condition #4 plus a cytokine/chemokine receptor agonist (e.g., CCL20)
**Condition #15: Two TNFRSF agonists plus one TLR agonist plus one cytokine/chemokine receptor agonist.**
   The combinations of Condition #5 plus a cytokine/chemokine receptor agonist (e.g., IFN-gamma)
   The combinations of Condition #5 plus a cytokine/chemokine receptor agonist (e.g., CCL20)
**Condition #16: Two TNFRSF agonists plus two TLR agonists plus one cytokine/chemokine receptor agonist.**
   The combinations of Condition #6 plus a cytokine/chemokine receptor agonist (e.g., IFN-gamma)
   The combinations of Condition #6 plus a cytokine/chemokine receptor agonist (e.g., CCL20)
**Condition #17: One TNFRSF agonist plus one NLR or RLH agonist plus one cytokine/chemokine receptor agonist.**
   The combinations of Condition #7 plus a cytokine/chemokine receptor agonist (e.g., IFN-gamma)
   The combinations of Condition #7 plus a cytokine/chemokine receptor agonist (e.g., CCL20)
**Condition #18: One TNFRSF agonist plus two TLR agonists plus one cytokine/chemokine receptor agonist.**
   The combinations of Condition #8 plus a cytokine/chemokine receptor agonist (e.g., IFN-gamma)
   The combinations of Condition #8 plus a cytokine/chemokine receptor agonist (e.g., CCL20)
**Condition #19: Two TNFRSF agonists plus one NLR or RLH agonist plus one cytokine/chemokine receptor agonist.**
   The combinations of Condition #9 plus a cytokine/chemokine receptor agonist (e.g., IFN-gamma)
   The combinations of Condition #9 plus a cytokine/chemokine receptor agonist (e.g., CCL20)
**Condition #20: Two TNFRSF agonists plus one NLR or RLH agonist plus one cytokine/chemokine receptor agonist.**
   The combinations of Condition #10 plus a cytokine/chemokine receptor agonist (e.g., IFN-gamma)
   The combinations of Condition #10 plus a cytokine/chemokine receptor agonist (e.g., CCL20)
**Condition #21: One TNFRSF agonist plus one TLR agonist plus one NLR or RLH agonist.**
   The combinations of Condition #3 plus a NOD1 agonist (e.g., M-TriDAP)
   The combinations of Condition #3 plus a NOD2 agonist (e.g., M-TriLys)
   The combinations of Condition #3 plus a MDA5 agonist (e.g., dsRNA)
**Condition #22: One TNFRSF agonist plus one TLR agonist plus two NLR or RLH agonists.**
   The combinations of Condition #3 plus NOD1 + NOD2 agonist (e.g. M-TriDAP + M-TriLys) The combinations of Condition #3 plus NOD1 + MDA5 agonist (e.g., M-TriDAP + dsRNA) The combinations of Condition #3 plus NOD2 + MDA5 agonist (e.g., M-TriLys + dsRNA)
**Condition #23: One TNFRSF agonist plus two TLR agonists plus one NLR or RLH agonist.**
   The combinations of Condition #4 plus a NOD1 agonist (e.g., M-TriDAP)
   The combinations of Condition #4 plus a NOD2 agonist (e.g., M-TriLys)
   The combinations of Condition #4 plus a MDA5 agonist (e.g., dsRNA)
**Condition #24: One TNFRSF agonist plus two TLR agonists plus two NLR or RLH agonists.**
   The combinations of Condition #4 plus NOD1 + NOD2 agonist (e.g. M-TriDAP + M-TriLys) The combinations of Condition #4 plus NOD1 + MDA5 agonist (e.g., M-TriDAP + dsRNA) The combinations of Condition #4 plus NOD2 + MDA5 agonist (e.g., M-TriLys + dsRNA)
**Condition #25: Two TNFRSF agonists plus one TLR agonist plus one NLR or RLH agonist.**
   The combinations of Condition #5 plus a NOD1 agonist (e.g., M-TriDAP)
   The combinations of Condition #5 plus a NOD2 agonist (e.g., M-TriLys)
   The combinations of Condition #5 plus a MDA5 agonist (e.g., dsRNA)
**Condition #26: Two TNFRSF agonists plus one TLR agonist plus two NLR or RLH agonists.**
   The combinations of Condition #5 plus NOD1 + NOD2 agonist (e.g. M-TriDAP + M-TriLys) The combinations of Condition #5 plus NOD1 + MDA5 agonist (e.g., M-TriDAP + dsRNA) The combinations of Condition #5 plus NOD2 + MDA5 agonist (e.g., M-TriLys + dsRNA)
**Condition #27: Two TNFRSF agonists plus two TLR agonists plus one NLR agonist.**
   The combinations of Condition #6 plus a NOD1 agonist (e.g., M-TriDAP)
   The combinations of Condition #6 plus a NOD2 agonist (e.g., M-TriLys)
   The combinations of Condition #6 plus a RIG-1 agonist (e.g., dsRNA)
**Condition #28: Two TNFRSF agonists plus two TLR agonists plus two NLR or RLH agonists.**
   The combinations of Condition #6 plus NOD1 + NOD2 agonist (e.g. M-TriDAP + M-TriLys) The combinations of Condition #6 plus NOD1 + MDA5 agonist (e.g., M-TriDAP + dsRNA) The combinations of Condition #6 plus NOD2 + MDA5 agonist (e.g., M-TriLys + dsRNA)
**Condition #29: One TNFRSF agonist plus one TLR agonist plus one NLR or RLH agonist plus one cytokine/chemokine receptor agonist.**
   The combinations of Condition #21 plus a cytokine/chemokine receptor agonist (e.g., IFN-gamma)
   The combinations of Condition #21 plus a cytokine/chemokine receptor agonist (e.g., CCL20)
**Condition #30: One TNFRSF agonist plus one TLR agonist plus two NLR or RLH agonists plus one cytokine/chemokine receptor agonist.**
   The combinations of Condition #22 plus a cytokine/chemokine receptor agonist (e.g., IFN-gamma)
   The combinations of Condition #22 plus a cytokine/chemokine receptor agonist (e.g., CCL20)
**Condition #31: One TNFRSF agonist plus two TLR agonists plus one NLR or RLH agonist plus one cytokine/chemokine receptor agonist.**
   The combinations of Condition #23 plus a cytokine/chemokine receptor agonist (e.g., IFN-gamma)
   The combinations of Condition #23 plus a cytokine/chemokine receptor agonist (e.g., CCL20)
**Condition #32: One TNFRSF agonist plus two TLR agonists plus two NLRor RLH agonists plus one cytokine/chemokine receptor agonist.**
   The combinations of Condition #24 plus a cytokine/chemokine receptor agonist (e.g., IFN-gamma)
   The combinations of Condition #24 plus a cytokine/chemokine receptor agonist (e.g., CCL20)
**Condition #33: Two TNFRSF agonists plus one TLR agonist plus one NLR or RLH agonist plus one cytokine/chemokine receptor agonist.**
   The combinations of Condition #25 plus a cytokine/chemokine receptor agonist (e.g., IFN-gamma)
   The combinations of Condition #25 plus a cytokine/chemokine receptor agonist (e.g., CCL20)
**Condition #34: Two TNFRSF agonists plus one TLR agonist plus two NLR or RLH agonists plus one cytokine/chemokine receptor agonist.**
   The combinations of Condition #26 plus a cytokine/chemokine receptor agonist (e.g., IFN-gamma)
   The combinations of Condition #26 plus a cytokine/chemokine receptor agonist (e.g., CCL20)
**Condition #35: Two TNFRSF agonists plus two TLR agonists plus one NLR or RLH agonist plus one cytokine/chemokine receptor agonist.**
   The combinations of Condition #27 plus a cytokine/chemokine receptor agonist (e.g., IFN-gamma)
   The combinations of Condition #27 plus a cytokine/chemokine receptor agonist (e.g., CCL20)
**Condition #36: Two TNFRSF agonists plus two TLR agonists plus two NLR or RLH agonists plus one cytokine/chemokine receptor agonist.**
   The combinations of Condition #28 plus a cytokine/chemokine receptor agonist (e.g., IFN-gamma)
   The combinations of Condition #28 plus a cytokine/chemokine receptor agonist (e.g., CCL20)
**Condition #37: On TNFRSF agonist plus on purigenic agonist.**
   **The combination of Condition #1 plus a purinergic agonist (e.g., ATPgammaS).**
**Condition # 38: Two TNFRSF agonists plus one purinergic agonist.**
   **The combination of Condition #2 plus a purinergic agonist (e.g., ATPgammaS).**
**Condition # 39: One TNFRSF agonist plus one TLR agonist plus one purinergic agonist.**
   **The combination of Condition #3 plus a purinergic agonist (e.g., ATPgammaS).**
**Condition # 40: ]One TNFRSF agonist plus two TLR agonists plus one purinergic agonists.**
   **The combination of Condition #4 plus a purinergic agonist (e.g., ATPgammaS).**
**Condition # 41: Two TNFRSF agonists plus one TLR agonist plus one purinergic agonist.**
   **The combination of Condition #5 plus a purinergic agonist (e.g., ATPgammaS).**
**Condition # 42: Two TNFRSF agonists plus two TLR agonist plus one purinergic agonist.**
   **The combination of Condition #6 plus a purinergic agonist (e.g., ATPgammaS).**
**Similarly, Conditions # 43 to # 72 are Conditions # 7 to # 36 plus one purinergic agonist, (e.g., ATPgammaS).**
**Furthermore, Conditions # 73 to # 102 are Conditions # 43 to # 72 (i.e., those with an added purinergic agonist) where an inhibitor(s) of ectonucleotidase(s) is added to prolong the effectiveness of the purinergic agonist.**

Although the invention has been described with reference to the above examples, it will be understood that modifications and variations are encompassed within the spirit and scope of the invention. Accordingly, the invention is limited only by the following claims.

## Claims

1. A composition comprising:
(a) one or more Tumor Necrosis Factor Receptor Superfamily (TNFRSF) agonists; and
(b) one or more purinergic receptor agonists.

2. The composition of claim 1, wherein (a) comprises one or more nucleic acids encoding the TNFRSF agonists.

3. The composition of claim 1 or claim 2, wherein the TNFRSF agonist(s) is selected from the group consisting of an LTA (lymphotoxin A) receptor agonist, an LTB (lymphotoxin B) receptor agonist, a TNFSF4 (OX-40L) receptor agonist, a TNFSF5 (CD40L) receptor agonist, a TNFSF6 (FasL) receptor agonist, a TNFSF7 (CD27L or CD70 or CD27L/CD70) receptor agonist, TNFSF8 (CD30L), a TNFSF9 (4-1BBL) receptor agonist, a TNFSF10 (TRAIL) receptor agonist, a TNFSF11 (RANKL) receptor agonist, a TNFSF12 (TWEAK) receptor agonist, a TNFSF13A (APRIL) receptor agonist, a TNFSF13B (BAFF) receptor agonist, a TNFSF14 (LIGHT) receptor agonist, a TNFRSF15 (VEGI) receptor agonist, a TNFRSF18 (GITRL) receptor agonist, and a combination thereof.

4. The composition of any one of claims 1 to 3, wherein the purinergic receptor agonist is extracellular ATP (ATPe).

5. The composition of any one of claims 1, 2 and 4, wherein the TNFRSF agonist is a CD40 receptor agonist.

6. A composition comprising:
(a) the composition of any one of claims 1 to 5;
(b) one or more agonists selected from the group consisting of TNFRSF agonists, TLR agonists, RLH agonists, purinergic receptor agonists, and cytokine/chemokine receptor agonists; and/or
(c) one or more NLR agonists, wherein the NLR agonist is selected from the group consisting of double stranded RNA, gamma-D-Glu-meso-diaminopimelic acid (DAP) or derivatives thereof, and muramyl dipeptide (MDP) or derivatives thereof.

7. The composition of claim 6, further comprising a polymer selected from the group consisting of polyethylenimine, cationic lipids, cationic polymers, dendrimeric polymers, poloxamines, poly-lactide-co-glycolide (PLGA) microparticles, poly(beta-amino ester) (PBAE) polymers, PLGA/PBAE ester microparticles, poly[alpha-(4-aminobutyl)-1-glycolic acid], poly(propylenimine) dendrimers, polylactic acid, polyethylene glycol (PEG)-ylated poly(lactic acid), poly(lactic-co-glycolic acid), poly(ortho esters), PEGylated poly(orthoesters), poly(caprolactone), PEGylated poly(caprolactone), polylysine, PEGylated polylysine, poly(ethylene imine), PEGylated poly(ethylene imine), poly(acrylic acid), PEGylated poly(acrylic acid), poly(urethane), PEGylated poly(urethane), polymeric lipid-protein-sugar microparticles, polymers that are hydrolyzable inside of cellular endosomes, self-assembling particles, and derivatives thereof.

8. The composition of any one of claims 1 to 7 or the composition of claim 6 or claim 7 for use in eliciting an immunotherapeutic response to an infection or neoplastic disease, whereby administration of the combination of agonists to a subject elicits a humoral immune response and/or a cell-mediated response, against the infection or neoplastic disease.

9. The composition for the use of claim 8, wherein administering the composition comprises electroporation, particle bombardment, injection, or a combination thereof.

10. The composition for the use of claim 8, further comprising administering an antigen associated with the infection or neoplastic disease.

11. The composition for the use of claim 10, wherein the antigen is selected from the group consisting of a viral antigen, a bacterial antigen, a parasitic antigen, a protozoal antigen, an abnormal host protein, and a tumor antigen.

12. The composition for the uses according to any one of claims 9 to 11, wherein the composition is administered directly into or around a tumor.


SEQUENCE LISTING

<110> THE REGENTS OF THE UNIVERSITY OF CALIFORNIA
KORNBLUTH, Richard S.
STONE, Geoffrey W.

<120> IMMUNOSTIMULATORY COMBINATIONS OF TNFRSF, TLR, NLR, RHR,
PURINERGIC RECEPTOR, AND CYTOKINE RECEPTOR AGONISTS FOR VACCINES
AND TUMOR IMMUNOTHERAPY

<130> UCSD1890-1EP

<150> PCT/US2007/000616
<151> 2007-01-09

<150> US 06/757,314
<151> 2006-01-09

<160> 8

<170> PatentIn version 3.3

<210> 1
<211> 4399
<212> DNA
<213> Mus musculus

<220>
<221> promoter
<222> (137)..(696)
<223> CMV promoter from pVAX1

<220>
<221> sig_peptide
<222> (727)..(784)
<223> Signal peptide

<220>
<221> CDS
<222> (784)..(1494)

<220>
<221> CDS
<222> (1495)..(2043)

<220>
<221> 3'UTR
<222> (2047)..(2186)
<223> 3' UTR from Mus musculus BAFF NM_009160, nucleotides 1147 to 1285

<220>
<221> misc_feature
<222> (2626)..(3420)
<223> Kanamycin resistance

<400> 1
gactcttcgc gatgtacggg ccagatatac gcgttgacat tgattattga ctagttatta 60

atagtaatca attacggggt cattagttca tagcccatat atggagttcc gcgttacata 120

acttacggta aatggcccgc ctggctgacc gcccaacgac ccccgcccat tgacgtcaat 180

aatgacgtat gttcccatag taacgccaat agggactttc cattgacgtc aatgggtgga 240

ctatttacgg taaactgccc acttggcagt acatcaagtg tatcatatgc caagtacgcc 300

ccctattgac gtcaatgacg gtaaatggcc cgcctggcat tatgcccagt acatgacctt 360

atgggacttt cctacttggc agtacatcta cgtattagtc atcgctatta ccatggtgat 420

gcggttttgg cagtacatca atgggcgtgg atagcggttt gactcacggg gatttccaag 480

tctccacccc attgacgtca atgggagttt gttttggcac caaaatcaac gggactttcc 540

aaaatgtcgt aacaactccg ccccattgac gcaaatgggc ggtaggcgtg tacggtggga 600

ggtctatata agcagagctc tctggctaac tagagaaccc actgcttact ggcttatcga 660

aattaatacg actcactata gggagaccca agctggctag cgaattccac caggaagcaa 720

tctgacatgc tgccctttct ctccatgctt gtcttgcttg tacagcccct gggaaatctg 780

gga gca gaa atg aag agc ctc tcg cag aga tca gta ccc aac acc tgc 828
    Ala Glu Met Lys Ser Leu Ser Gln Arg Ser Val Pro Asn Thr Cys
    1               5                   10                  15

acc cta gtc atg tgt agc cca aca gag aat ggc ctg cct ggt cgt gat 876
Thr Leu Val Met Cys Ser Pro Thr Glu Asn Gly Leu Pro Gly Arg Asp
                20                  25                  30

gga cgg gat ggg aga gaa ggt cca cgg ggt gag aag ggt gat cca ggt 924
Gly Arg Asp Gly Arg Glu Gly Pro Arg Gly Glu Lys Gly Asp Pro Gly
            35                  40                  45

ttg cca gga cct atg ggg ctc tca ggg ttg cag ggc cct aca ggt cca 972
Leu Pro Gly Pro Met Gly Leu Ser Gly Leu Gln Gly Pro Thr Gly Pro
        50                  55                  60

gtt gga ccc aaa gga gag aat ggc tct gct ggc gaa cct gga cca aag 1020
Val Gly Pro Lys Gly Glu Asn Gly Ser Ala Gly Glu Pro Gly Pro Lys
    65                  70                  75

gga gaa cgt gga cta agt gga cct cca gga ctt cca ggt att cct ggt 1068
Gly Glu Arg Gly Leu Ser Gly Pro Pro Gly Leu Pro Gly Ile Pro Gly
80                  85                  90                  95

cca gct ggg aaa gaa ggt ccc tct ggg aag cag ggg aac ata gga cct 1116
Pro Ala Gly Lys Glu Gly Pro Ser Gly Lys Gln Gly Asn Ile Gly Pro
                100                 105                 110

caa ggc aaa cca ggt cct aaa gga gag gct ggg ccc aaa gga gaa gta 1164
Gln Gly Lys Pro Gly Pro Lys Gly Glu Ala Gly Pro Lys Gly Glu Val
            115                 120                 125

ggt gct cct ggc atg caa gga tct aca ggg gca aaa ggc tcc aca ggc 1212
Gly Ala Pro Gly Met Gln Gly Ser Thr Gly Ala Lys Gly Ser Thr Gly
        130                 135                 140

ccc aag gga gaa aga ggt gcc cct ggt gtg caa gga gcc cca ggg aat 1260
Pro Lys Gly Glu Arg Gly Ala Pro Gly Val Gln Gly Ala Pro Gly Asn
    145                 150                 155

gct gga gca gca gga cct gcc gga cct gcc ggt cca cag gga gct cca 1308
Ala Gly Ala Ala Gly Pro Ala Gly Pro Ala Gly Pro Gln Gly Ala Pro
160                 165                 170                 175

ggt tcc agg ggg ccc cca gga ctc aag ggg gac aga ggt gtt cct gga 1356

Gly Ser Arg Gly Pro Pro Gly Leu Lys Gly Asp Arg Gly Val Pro Gly
                180                 185                 190

gac aga gga atc aaa ggt gaa agc ggg ctt cca gac agt gct gct ctg      1404
Asp Arg Gly Ile Lys Gly Glu Ser Gly Leu Pro Asp Ser Ala Ala Leu
            195                 200                 205

agg cag cag atg gag gcc tta aaa gga aaa cta cag cgt cta gag gtt      1452
Arg Gln Gln Met Glu Ala Leu Lys Gly Lys Leu Gln Arg Leu Glu Val
        210                 215                 220

gcc ttc tcc cac tat cag aaa gct gca ttg ttc cct gat ggc gct ttc      1500
Ala Phe Ser His Tyr Gln Lys Ala Ala Leu Phe Pro Asp Gly Ala Phe
    225                 230                 235

cag gga cca gag gaa aca gaa caa gat gta gac ctc tca gct cct cct      1548
Gln Gly Pro Glu Glu Thr Glu Gln Asp Val Asp Leu Ser Ala Pro Pro
240                 245                 250                 255

gca cca tgc ctg cct gga tgc cgc cat tct caa cat gat gat aat gga      1596
Ala Pro Cys Leu Pro Gly Cys Arg His Ser Gln His Asp Asp Asn Gly
                260                 265                 270

atg aac ctc aga aac atc att caa gac tgt ctg cag ctg att gca gac      1644
Met Asn Leu Arg Asn Ile Ile Gln Asp Cys Leu Gln Leu Ile Ala Asp
            275                 280                 285

agc gac acg ccg act ata cga aaa gga act tac aca ttt gtt cca tgg      1692
Ser Asp Thr Pro Thr Ile Arg Lys Gly Thr Tyr Thr Phe Val Pro Trp
        290                 295                 300

ctt ctc agc ttt aaa aga gga aat gcc ttg gag gag aaa gag aac aaa      1740
Leu Leu Ser Phe Lys Arg Gly Asn Ala Leu Glu Glu Lys Glu Asn Lys
    305                 310                 315

ata gtg gtg agg caa aca ggc tat ttc ttc atc tac agc cag gtt cta      1788
Ile Val Val Arg Gln Thr Gly Tyr Phe Phe Ile Tyr Ser Gln Val Leu
320                 325                 330                 335

tac acg gac ccc atc ttt gct atg ggt cat gtc atc cag agg aag aaa      1836
Tyr Thr Asp Pro Ile Phe Ala Met Gly His Val Ile Gln Arg Lys Lys
                340                 345                 350

gta cac gtc ttt ggg gac gag ctg agc ctg gtg acc ctg ttc cga tgt      1884
Val His Val Phe Gly Asp Glu Leu Ser Leu Val Thr Leu Phe Arg Cys
            355                 360                 365

att cag aat atg ccc aaa aca ctg ccc aac aat tcc tgc tac tcg gct      1932
Ile Gln Asn Met Pro Lys Thr Leu Pro Asn Asn Ser Cys Tyr Ser Ala
        370                 375                 380

ggc atc gcg agg ctg gaa gaa gga gat gag att cag ctt gca att cct      1980
Gly Ile Ala Arg Leu Glu Glu Gly Asp Glu Ile Gln Leu Ala Ile Pro
    385                 390                 395

cgg gag aat gca cag att tca cgc aac gga gac gac acc ttc ttt ggt      2028
Arg Glu Asn Ala Gln Ile Ser Arg Asn Gly Asp Asp Thr Phe Phe Gly
400                 405                 410                 415

gcc cta aaa ctg ctg taactcactt gctggagtgc gtgatcccct tccctcgtct      2083
Ala Leu Lys Leu Leu
                420

tctctgtacc tccgagggag aaacagacga ctggaaaaac taaaagatgg ggaaagccgt      2143

cagcgaaagt tttctcgtga cccgttgaat ctgatccaaa ccctcgagtc tagagggccc 2203

gtttaaaccc gctgatcagc ctcgactgtg ccttctagtt gccagccatc tgttgtttgc 2263

ccctcccccg tgccttcctt gaccctggaa ggtgccactc ccactgtcct ttcctaataa 2323

aatgaggaaa ttgcatcgca ttgtctgagt aggtgtcatt ctattctggg gggtggggtg 2383

gggcaggaca gcaaggggga ggattgggaa gacaatagca ggcatgctgg ggatgcggtg 2443

ggctctatgg cttctactgg gcggttttat ggacagcaag cgaaccggaa ttgccagctg 2503

gggcgccctc tggtaaggtt gggaagccct gcaaagtaaa ctggatggct ttctcgccgc 2563

caaggatctg atggcgcagg ggatcaagct ctgatcaaga gacaggatga ggatcgtttc 2623

gcatgattga acaagatgga ttgcacgcag gttctccggc cgcttgggtg gagaggctat 2683

tcggctatga ctgggcacaa cagacaatcg gctgctctga tgccgccgtg ttccggctgt 2743

cagcgcaggg gcgcccggtt ctttttgtca agaccgacct gtccggtgcc ctgaatgaac 2803

tgcaagacga ggcagcgcgg ctatcgtggc tggccacgac gggcgttcct tgcgcagctg 2863

tgctcgacgt tgtcactgaa gcgggaaggg actggctgct attgggcgaa gtgccggggc 2923

aggatctcct gtcatctcac cttgctcctg ccgagaaagt atccatcatg gctgatgcaa 2983

tgcggcggct gcatacgctt gatccggcta cctgcccatt cgaccaccaa gcgaaacatc 3043

gcatcgagcg agcacgtact cggatggaag ccggtcttgt cgatcaggat gatctggacg 3103

aagagcatca ggggctcgcg ccagccgaac tgttcgccag gctcaaggcg agcatgcccg 3163

acggcgagga tctcgtcgtg acccatggcg atgcctgctt gccgaatatc atggtggaaa 3223

atggccgctt ttctggattc atcgactgtg gccggctggg tgtggcggac cgctatcagg 3283

acatagcgtt ggctacccgt gatattgctg aagagcttgg cggcgaatgg gctgaccgct 3343

tcctcgtgct ttacggtatc gccgctcccg attcgcagcg catcgccttc tatcgccttc 3403

ttgacgagtt cttctgaatt attaacgctt acaatttcct gatgcggtat tttctcctta 3463

cgcatctgtg cggtatttca caccgcatac aggtggcact tttcggggaa atgtgcgcgg 3523

aacccctatt tgtttatttt tctaaataca ttcaaatatg tatccgctca tgagacaata 3583

accctgataa atgcttcaat aatagcacgt gctaaaactt catttttaat ttaaaaggat 3643

ctaggtgaag atcctttttg ataatctcat gaccaaaatc ccttaacgtg agttttcgtt 3703

ccactgagcg tcagaccccg tagaaaagat caaaggatct tcttgagatc ctttttttct 3763

gcgcgtaatc tgctgcttgc aaacaaaaaa accaccgcta ccagcggtgg tttgtttgcc 3823

ggatcaagag ctaccaactc tttttccgaa ggtaactggc ttcagcagag cgcagatacc 3883

aaatactgtc cttctagtgt agccgtagtt aggccaccac ttcaagaact ctgtagcacc 3943

gcctacatac ctcgctctgc taatcctgtt accagtggct gctgccagtg gcgataagtc 4003

gtgtcttacc gggttggact caagacgata gttaccggat aaggcgcagc ggtcgggctg 4063

aacggggggt tcgtgcacac agcccagctt ggagcgaacg acctacaccg aactgagata 4123

cctacagcgt gagctatgag aaagcgccac gcttcccgaa gggagaaagg cggacaggta 4183

tccggtaagc ggcagggtcg gaacaggaga gcgcacgagg gagcttccag ggggaaacgc 4243

ctggtatctt tatagtcctg tcgggtttcg ccacctctga cttgagcgtc gatttttgtg 4303

atgctcgtca ggggggcgga gcctatggaa aaacgccagc aacgcggcct ttttacggtt 4363

cctggccttt tgctggcctt ttgctcacat gttctt 4399

<210> 2
<211> 420
<212> PRT
<213> Mus musculus

<400> 2

Ala Glu Met Lys Ser Leu Ser Gln Arg Ser Val Pro Asn Thr Cys Thr
1               5                   10                  15

Leu Val Met Cys Ser Pro Thr Glu Asn Gly Leu Pro Gly Arg Asp Gly
            20                  25                  30

Arg Asp Gly Arg Glu Gly Pro Arg Gly Glu Lys Gly Asp Pro Gly Leu
        35                  40                  45

Pro Gly Pro Met Gly Leu Ser Gly Leu Gln Gly Pro Thr Gly Pro Val
    50                  55                  60

Gly Pro Lys Gly Glu Asn Gly Ser Ala Gly Glu Pro Gly Pro Lys Gly
65                  70                  75                  80

Glu Arg Gly Leu Ser Gly Pro Pro Gly Leu Pro Gly Ile Pro Gly Pro
                85                  90                  95

Ala Gly Lys Glu Gly Pro Ser Gly Lys Gln Gly Asn Ile Gly Pro Gln
            100                 105                 110

Gly Lys Pro Gly Pro Lys Gly Glu Ala Gly Pro Lys Gly Glu Val Gly
        115                 120                 125

Ala Pro Gly Met Gln Gly Ser Thr Gly Ala Lys Gly Ser Thr Gly Pro
    130                 135                 140

Lys Gly Glu Arg Gly Ala Pro Gly Val Gln Gly Ala Pro Gly Asn Ala
145                 150                 155                 160

Gly Ala Ala Gly Pro Ala Gly Pro Ala Gly Pro Gln Gly Ala Pro Gly
                165                 170                 175

Ser Arg Gly Pro Pro Gly Leu Lys Gly Asp Arg Gly Val Pro Gly Asp

180                 185                 190

Arg Gly Ile Lys Gly Glu Ser Gly Leu Pro Asp Ser Ala Ala Leu Arg
        195                 200                 205

Gln Gln Met Glu Ala Leu Lys Gly Lys Leu Gln Arg Leu Glu Val Ala
    210                 215                 220

Phe Ser His Tyr Gln Lys Ala Ala Leu Phe Pro Asp Gly Ala Phe Gln
225                 230                 235                 240

Gly Pro Glu Glu Thr Glu Gln Asp Val Asp Leu Ser Ala Pro Pro Ala
                245                 250                 255

Pro Cys Leu Pro Gly Cys Arg His Ser Gln His Asp Asp Asn Gly Met
            260                 265                 270

Asn Leu Arg Asn Ile Ile Gln Asp Cys Leu Gln Leu Ile Ala Asp Ser
        275                 280                 285

Asp Thr Pro Thr Ile Arg Lys Gly Thr Tyr Thr Phe Val Pro Trp Leu
    290                 295                 300

Leu Ser Phe Lys Arg Gly Asn Ala Leu Glu Glu Lys Glu Asn Lys Ile
305                 310                 315                 320

Val Val Arg Gln Thr Gly Tyr Phe Phe Ile Tyr Ser Gln Val Leu Tyr
                325                 330                 335

Thr Asp Pro Ile Phe Ala Met Gly His Val Ile Gln Arg Lys Lys Val
            340                 345                 350

His Val Phe Gly Asp Glu Leu Ser Leu Val Thr Leu Phe Arg Cys Ile
        355                 360                 365

Gln Asn Met Pro Lys Thr Leu Pro Asn Asn Ser Cys Tyr Ser Ala Gly
    370                 375                 380

Ile Ala Arg Leu Glu Glu Gly Asp Glu Ile Gln Leu Ala Ile Pro Arg
385                 390                 395                 400

Glu Asn Ala Gln Ile Ser Arg Asn Gly Asp Asp Thr Phe Phe Gly Ala
                405                 410                 415

Leu Lys Leu Leu
            420

<210>  3

<211>  6061
<212>  DNA
<213>  Mus musculus

<220>
<221>  rep_origin
<222>  (745)..(1081)
<223>  SV40 ori

<220>
<221>  rep_origin
<222>  (1292)..(1926)
<223>  ColE1 replication origin

<220>
<221>  misc_feature
<222>  (2083)..(2943)
<223>  Ampicillin resistance gene

<220>
<221>  promoter
<222>  (3091)..(4791)
<223>  CAG promoter from pCAGGS (Niwa et al. Gene 108, 193-199 (1991).

<220>
<221>  promoter
<222>  (3091)..(4791)
<223>  CAG promoter from pCAGGS (Niwa et al. Gene 108, 193-199 (1991).

<220>
<221>  sig_peptide
<222>  (4807)..(4875)
<223>  Signal sequence from human tissue plasminogen activator
       precursor, GenBank NM_000930 nucleotides 209 to 277

<220>
<221>  CDS
<222>  (4876)..(5586)
<223>  Mus musculus pulmonary surfactant protein D, GenBank NM_009160,
       nucleotides 100 to 810, deleting the carbohydrate recognition
       domain (CRD)

<220>
<221>  CDS
<222>  (5587)..(6024)
<223>  CD40L from Mus musculus, GenBank NM_011616, nucleotides 357 to
       792, deleting most of the extracellular "stalk"

<400>  3
tcgaggatat cgcggccgca ctcctcaggt gcaggctgcc tatcagaagg tggtggctgg      60

tgtggccaat gccctggctc acaaatacca ctgagatctt tttccctctg ccaaaaatta     120

tggggacatc atgaagcccc ttgagcatct gacttctggc taataaagga aatttatttt     180

cattgcaata gtgtgttgga attttttgtg tctctcactc ggaaggacat atgggagggc     240

aaatcattta aaacatcaga atgagtattt ggtttagagt ttggcaacat atgccatatg     300

ctggctgcca tgaacaaagg tggctataaa gaggtcatca gtatatgaaa cagccccctg     360

ctgtccattc cttattccat agaaaagcct tgacttgagg ttagattttt tttatatttt     420

gttttgtgtt atttttttct ttaacatccc taaaattttc cttacatgtt ttactagcca     480

gatttttcct cctctcctga ctactcccag tcatagctgt ccctcttctc ttatgaagat 540

ccctcgacct gcagcccaag cttggcgtaa tcatggtcat agctgtttcc tgtgtgaaat 600

tgttatccgc tcacaattcc acacaacata cgagccggaa gcataaagtg taaagcctgg 660

ggtgcctaat gagtgagcta actcacatta attgcgttgc gctcactgcc cgctttccag 720

tcgggaaacc tgtcgtgcca gcggatccgc atctcaatta gtcagcaacc atagtcccgc 780

ccctaactcc gcccatcccg cccctaactc cgcccagttc cgcccattct ccgccccatg 840

gctgactaat tttttttatt tatgcagagg ccgaggccgc ctcggcctct gagctattcc 900

agaagtagtg aggaggcttt tttggaggcc taggcttttg caaaaagcta acttgtttat 960

tgcagcttat aatggttaca aataaagcaa tagcatcaca aatttcacaa ataaagcatt 1020

tttttcactg cattctagtt gtggtttgtc caaactcatc aatgtatctt atcatgtctg 1080

gatccgctgc attaatgaat cggccaacgc gcggggagag gcggtttgcg tattgggcgc 1140

tcttccgctt cctcgctcac tgactcgctg cgctcggtcg ttcggctgcg gcgagcggta 1200

tcagctcact caaaggcggt aatacggtta tccacagaat caggggataa cgcaggaaag 1260

aacatgtgag caaaaggcca gcaaaaggcc aggaaccgta aaaaggccgc gttgctggcg 1320

tttttccata ggctccgccc ccctgacgag catcacaaaa atcgacgctc aagtcagagg 1380

tggcgaaacc cgacaggact ataaagatac caggcgtttc cccctggaag ctccctcgtg 1440

cgctctcctg ttccgaccct gccgcttacc ggatacctgt ccgcctttct cccttcggga 1500

agcgtggcgc tttctcaatg ctcacgctgt aggtatctca gttcggtgta ggtcgttcgc 1560

tccaagctgg gctgtgtgca cgaacccccc gttcagcccg accgctgcgc cttatccggt 1620

aactatcgtc ttgagtccaa cccggtaaga cacgacttat cgccactggc agcagccact 1680

ggtaacagga ttagcagagc gaggtatgta ggcggtgcta cagagttctt gaagtggtgg 1740

cctaactacg gctacactag aaggacagta tttggtatct gcgctctgct gaagccagtt 1800

accttcggaa aaagagttgg tagctcttga tccggcaaac aaaccaccgc tggtagcggt 1860

ggtttttttg tttgcaagca gcagattacg cgcagaaaaa aaggatctca agaagatcct 1920

ttgatctttt ctacggggtc tgacgctcag tggaacgaaa actcacgtta agggattttg 1980

gtcatgagat tatcaaaaag gatcttcacc tagatccttt taaattaaaa atgaagtttt 2040

aaatcaatct aaagtatata tgagtaaact tggtctgaca gttaccaatg cttaatcagt 2100

gaggcaccta tctcagcgat ctgtctattt cgttcatcca tagttgcctg actccccgtc 2160

gtgtagataa ctacgatacg ggagggctta ccatctggcc ccagtgctgc aatgataccg 2220

cgagacccac gctcaccggc tccagattta tcagcaataa accagccagc cggaagggcc 2280

gagcgcagaa gtggtcctgc aactttatcc gcctccatcc agtctattaa ttgttgccgg 2340

gaagctagag taagtagttc gccagttaat agtttgcgca acgttgttgc cattgctaca 2400

ggcatcgtgg tgtcacgctc gtcgtttggt atggcttcat tcagctccgg ttcccaacga 2460

tcaaggcgag ttacatgatc ccccatgttg tgcaaaaaag cggttagctc cttcggtcct 2520

ccgatcgttg tcagaagtaa gttggccgca gtgttatcac tcatggttat ggcagcactg 2580

cataattctc ttactgtcat gccatccgta agatgctttt ctgtgactgg tgagtactca 2640

accaagtcat tctgagaata gtgtatgcgg cgaccgagtt gctcttgccc ggcgtcaata 2700

cgggataata ccgcgccaca tagcagaact ttaaaagtgc tcatcattgg aaaacgttct 2760

tcggggcgaa aactctcaag gatcttaccg ctgttgagat ccagttcgat gtaacccact 2820

cgtgcaccca actgatcttc agcatctttt actttcacca gcgtttctgg gtgagcaaaa 2880

acaggaaggc aaaatgccgc aaaaaaggga ataagggcga cacggaaatg ttgaatactc 2940

atactcttcc tttttcaata ttattgaagc atttatcagg gttattgtct catgagcgga 3000

tacatatttg aatgtattta gaaaaataaa caaatagggg ttccgcgcac atttccccga 3060

aaagtgccac ctggtcgaca ttgattattg actagttatt aatagtaatc aattacgggg 3120

tcattagttc atagcccata tatggagttc cgcgttacat aacttacggt aaatggcccg 3180

cctggctgac cgcccaacga cccccgccca ttgacgtcaa taatgacgta tgttcccata 3240

gtaacgccaa tagggacttt ccattgacgt caatgggtgg actatttacg gtaaactgcc 3300

cacttggcag tacatcaagt gtatcatatg ccaagtacgc cccctattga cgtcaatgac 3360

ggtaaatggc ccgcctggca ttatgcccag tacatgacct tatgggactt tcctacttgg 3420

cagtacatct acgtattagt catcgctatt accatgggtc gaggtgagcc ccacgttctg 3480

cttcactctc cccatctccc ccccctcccc acccccaatt ttgtatttat ttatttttta 3540

attattttgt gcagcgatgg gggcgggggg gggggggcgg cgcgccaggc ggggcggggc 3600

ggggcgaggg gcggggcggg gcgaggcgga gaggtgcggc ggcagccaat cagagcggcg 3660

cgctccgaaa gtttcctttt atggcgaggc ggcggcggcg gcggccctat aaaaagcgaa 3720

gcgcgcggcg ggcgggagtc gctgcgttgc cttcgccccg tgccccgctc cgcgccgcct 3780

cgcgccgccc gccccggctc tgactgaccg cgttactccc acaggtgagc gggcgggacg 3840

gcccttctcc tccgggctgt aattagcgct tggtttaatg acggctcgtt tcttttctgt 3900

ggctgcgtga aagccttaaa gggctccggg agggcccttt gtgcgggggg gagcggctcg 3960

gggggtgcgt gcgtgtgtgt gtgcgtgggg agcgccgcgt gcggcccgcg ctgcccggcg 4020

gctgtgagcg ctgcgggcgc ggcgcggggc tttgtgcgct ccgcgtgtgc gcgaggggag 4080

cgcggccggg ggcggtgccc cgcggtgcgg gggggctgcg aggggaacaa aggctgcgtg 4140

cggggtgtgt gcgtgggggg gtgagcaggg ggtgtgggcg cggcggtcgg gctgtaaccc 4200

ccccctgcac ccccctcccc gagttgctga gcacggcccg gcttcgggtg cggggctccg 4260

tgcggggcgt ggcgcggggc tcgccgtgcc gggcgggggg tggcggcagg tgggggtgcc 4320

gggcggggcg gggccgcctc gggccgggga gggctcgggg gaggggcgcg gcggccccgg 4380

agcgccggcg gctgtcgagg cgcggcgagc cgcagccatt gccttttatg gtaatcgtgc 4440

gagagggcgc agggacttcc tttgtcccaa atctggcgga gccgaaatct gggaggcgcc 4500

gccgcacccc ctctagcggg cgcgggcgaa gcggtgcggc gccggcagga aggaaatggg 4560

cggggagggc cttcgtgcgt cgccgcgccg ccgtcccctt ctccatctcc agcctcgggg 4620

ctgccgcagg gggacggctg ccttcggggg ggacggggca gggcggggtt cggcttctgg 4680

cgtgtgaccg gcggctctag agcctctgct aaccatgttc atgccttctt ctttttccta 4740

cagctcctgg gcaacgtgct ggttattgtg ctgtctcatc attttggcaa agaattcgcc 4800

gccaccatgg atgcaatgaa gagagggctc tgctgtgtgc tgctgctgtg tggagcagtc 4860

ttcgtttcgc ccagc gca gaa atg aag agc ctc tcg cag aga tca gta ccc 4911
Ala Glu Met Lys Ser Leu Ser Gln Arg Ser Val Pro
1               5                   10

aac acc tgc acc cta gtc atg tgt agc cca aca gag aat ggc ctg cct 4959
Asn Thr Cys Thr Leu Val Met Cys Ser Pro Thr Glu Asn Gly Leu Pro
        15                  20                  25

ggt cgt gat gga cgg gat ggg aga gaa ggt cca cgg ggt gag aag ggt 5007
Gly Arg Asp Gly Arg Asp Gly Arg Glu Gly Pro Arg Gly Glu Lys Gly
    30                  35                  40

gat cca ggt ttg cca gga cct atg ggg ctc tca ggg ttg cag ggc cct 5055
Asp Pro Gly Leu Pro Gly Pro Met Gly Leu Ser Gly Leu Gln Gly Pro
45                  50                  55                  60

aca ggt cca gtt gga ccc aaa gga gag aat ggc tct gct ggc gaa cct 5103
Thr Gly Pro Val Gly Pro Lys Gly Glu Asn Gly Ser Ala Gly Glu Pro
                65                  70                  75

gga cca aag gga gaa cgt gga cta agt gga cct cca gga ctt cca ggt 5151
Gly Pro Lys Gly Glu Arg Gly Leu Ser Gly Pro Pro Gly Leu Pro Gly
            80                  85                  90

att cct ggt cca gct ggg aaa gaa ggt ccc tct ggg aag cag ggg aac 5199
Ile Pro Gly Pro Ala Gly Lys Glu Gly Pro Ser Gly Lys Gln Gly Asn
        95                  100                 105

ata gga cct caa ggc aaa cca ggt cct aaa gga gag gct ggg ccc aaa 5247
Ile Gly Pro Gln Gly Lys Pro Gly Pro Lys Gly Glu Ala Gly Pro Lys
    110                 115                 120

gga gaa gta ggt gct cct ggc atg caa gga tct aca ggg gca aaa ggc 5295
Gly Glu Val Gly Ala Pro Gly Met Gln Gly Ser Thr Gly Ala Lys Gly
125                 130                 135                 140

tcc aca ggc ccc aag gga gaa aga ggt gcc cct ggt gtg caa gga gcc 5343
Ser Thr Gly Pro Lys Gly Glu Arg Gly Ala Pro Gly Val Gln Gly Ala
                145                 150                 155

cca ggg aat gct gga gca gca gga cct gcc gga cct gcc ggt cca cag 5391
Pro Gly Asn Ala Gly Ala Ala Gly Pro Ala Gly Pro Ala Gly Pro Gln
            160                 165                 170

gga gct cca ggt tcc agg ggg ccc cca gga ctc aag ggg gac aga ggt 5439

Gly Ala Pro Gly Ser Arg Gly Pro Pro Gly Leu Lys Gly Asp Arg Gly
        175                 180                 185

gtt cct gga gac aga gga atc aaa ggt gaa agc ggg ctt cca gac agt      5487
Val Pro Gly Asp Arg Gly Ile Lys Gly Glu Ser Gly Leu Pro Asp Ser
    190                 195                 200

gct gct ctg agg cag cag atg gag gcc tta aaa gga aaa cta cag cgt      5535
Ala Ala Leu Arg Gln Gln Met Glu Ala Leu Lys Gly Lys Leu Gln Arg
205                 210                 215                 220

cta gag gtt gcc ttc tcc cac tat cag aaa gct gca ttg ttc cct gat      5583
Leu Glu Val Ala Phe Ser His Tyr Gln Lys Ala Ala Leu Phe Pro Asp
                225                 230                 235

ggc ggt gat gag gat cct caa att gca gca cac gtt gta agc gaa gcc      5631
Gly Gly Asp Glu Asp Pro Gln Ile Ala Ala His Val Val Ser Glu Ala
            240                 245                 250

aac agt aat gca gca tcc gtt cta cag tgg gcc aag aaa gga tat tat      5679
Asn Ser Asn Ala Ala Ser Val Leu Gln Trp Ala Lys Lys Gly Tyr Tyr
        255                 260                 265

acc atg aaa agc aac ttg gta atg ctt gaa aat ggg aaa cag ctg acg      5727
Thr Met Lys Ser Asn Leu Val Met Leu Glu Asn Gly Lys Gln Leu Thr
    270                 275                 280

gtt aaa aga gaa gga ctc tat tat gtc tac act caa gtc acc ttc tgc      5775
Val Lys Arg Glu Gly Leu Tyr Tyr Val Tyr Thr Gln Val Thr Phe Cys
285                 290                 295                 300

tct aat cgg gag cct tcg agt caa cgc cca ttc atc gtc ggc ctc tgg      5823
Ser Asn Arg Glu Pro Ser Ser Gln Arg Pro Phe Ile Val Gly Leu Trp
                305                 310                 315

ctg aag ccc agc att gga tct gag aga atc tta ctc aag gcg gca aat      5871
Leu Lys Pro Ser Ile Gly Ser Glu Arg Ile Leu Leu Lys Ala Ala Asn
            320                 325                 330

acc cac agt tcc tcc cag ctt tgc gag cag cag tct gtt cac ttg ggc      5919
Thr His Ser Ser Ser Gln Leu Cys Glu Gln Gln Ser Val His Leu Gly
        335                 340                 345

gga gtg ttt gaa tta caa gct ggt gct tct gtg ttt gtc aac gtg act      5967
Gly Val Phe Glu Leu Gln Ala Gly Ala Ser Val Phe Val Asn Val Thr
    350                 355                 360

gaa gca agc caa gtg atc cac aga gtt ggc ttc tca tct ttt ggc tta      6015
Glu Ala Ser Gln Val Ile His Arg Val Gly Phe Ser Ser Phe Gly Leu
365                 370                 375                 380

ctc aaa ctc tgaacagtgc gctgtcctag gctgcagcag ggtaccc                 6061
Leu Lys Leu

<210>  4
<211>  383
<212>  PRT
<213>  Mus musculus

<400>  4

Ala Glu Met Lys Ser Leu Ser Gln Arg Ser Val Pro Asn Thr Cys Thr

1               5                   10                  15

Leu Val Met Cys Ser Pro Thr Glu Asn Gly Leu Pro Gly Arg Asp Gly
                20                  25                  30

Arg Asp Gly Arg Glu Gly Pro Arg Gly Glu Lys Gly Asp Pro Gly Leu
        35                  40                  45

Pro Gly Pro Met Gly Leu Ser Gly Leu Gln Gly Pro Thr Gly Pro Val
    50                  55                  60

Gly Pro Lys Gly Glu Asn Gly Ser Ala Gly Glu Pro Gly Pro Lys Gly
65                  70                  75                  80

Glu Arg Gly Leu Ser Gly Pro Pro Gly Leu Pro Gly Ile Pro Gly Pro
                85                  90                  95

Ala Gly Lys Glu Gly Pro Ser Gly Lys Gln Gly Asn Ile Gly Pro Gln
            100                 105                 110

Gly Lys Pro Gly Pro Lys Gly Glu Ala Gly Pro Lys Gly Glu Val Gly
        115                 120                 125

Ala Pro Gly Met Gln Gly Ser Thr Gly Ala Lys Gly Ser Thr Gly Pro
    130                 135                 140

Lys Gly Glu Arg Gly Ala Pro Gly Val Gln Gly Ala Pro Gly Asn Ala
145                 150                 155                 160

Gly Ala Ala Gly Pro Ala Gly Pro Ala Gly Pro Gln Gly Ala Pro Gly
                165                 170                 175

Ser Arg Gly Pro Pro Gly Leu Lys Gly Asp Arg Gly Val Pro Gly Asp
            180                 185                 190

Arg Gly Ile Lys Gly Glu Ser Gly Leu Pro Asp Ser Ala Ala Leu Arg
        195                 200                 205

Gln Gln Met Glu Ala Leu Lys Gly Lys Leu Gln Arg Leu Glu Val Ala
    210                 215                 220

Phe Ser His Tyr Gln Lys Ala Ala Leu Phe Pro Asp Gly Gly Asp Glu
225                 230                 235                 240

Asp Pro Gln Ile Ala Ala His Val Val Ser Glu Ala Asn Ser Asn Ala
                245                 250                 255

Ala Ser Val Leu Gln Trp Ala Lys Lys Gly Tyr Tyr Thr Met Lys Ser

260                 265                 270

Asn Leu Val Met Leu Glu Asn Gly Lys Gln Leu Thr Val Lys Arg Glu
        275                 280                 285

Gly Leu Tyr Tyr Val Tyr Thr Gln Val Thr Phe Cys Ser Asn Arg Glu
    290                 295                 300

Pro Ser Ser Gln Arg Pro Phe Ile Val Gly Leu Trp Leu Lys Pro Ser
305                 310                 315                 320

Ile Gly Ser Glu Arg Ile Leu Leu Lys Ala Ala Asn Thr His Ser Ser
                325                 330                 335

Ser Gln Leu Cys Glu Gln Gln Ser Val His Leu Gly Gly Val Phe Glu
            340                 345                 350

Leu Gln Ala Gly Ala Ser Val Phe Val Asn Val Thr Glu Ala Ser Gln
        355                 360                 365

Val Ile His Arg Val Gly Phe Ser Ser Phe Gly Leu Leu Lys Leu
    370                 375                 380

<210>  5
<211>  22
<212>  DNA
<213>  Artificial sequence

<220>
<223>  Unmethylated oligonucleotide sequence that is a ligand for
       Toll-Like Receptor 9 (TLR9)

<220>
<221>  misc_feature
<222>  (1)..(22)
<223>  All linkages are phosphorothioate, used as an agonist for TLR9

<400>  5
tgactgtgaa cgttcgagat ga                                              22

<210>  6
<211>  9
<212>  PRT
<213>  Artificial sequence

<220>
<223>  Synthetic construct

<400>  6

Ala Met Gln Met Leu Lys Glu Thr Ile
1               5

<210>  7

<211>  24
<212>  DNA
<213>  Artificial sequence

<220>
<223>  Synthetic construct

<400>  7
tgaactgtga acgttcgaga ttga                                              24


<210>  8
<211>  4
<212>  PRT
<213>  Artificial sequence

<220>
<223>  Synthetic construct

<400>  8

Lys Gly Tyr Tyr
1